# EUROPEAN PATENT SPECIFICATION

(11) **EP 4 069 438 B1**
(45) Date of publication and mention of the grant of the patent: **01.10.2025**
(21) Application number: 20895471.9
(22) Date of filing: 03.12.2020
(51) Int. Cl.: B06B 1/06, A61N 7/02, B06B 3/00, A61N 7/00

(54) **SKIN TREATMENTS SYSTEM**
HAUTBEHANDLUNGSSYSTEM
SYSTÈME DE TRAITEMENT DE LA PEAU

(30) Priority: 04.12.2019 US 201962943309 P
(43) Date of publication of application: 12.10.2022
(73) Proprietor: Sofwave Medical Ltd., 2069200 Yokneam Illit (IL)
(72) Inventor: SVERDLIK, Ariel, 6971504 Tel-Aviv (IL); ECKHOUSE, Shimon, 3491274 Haifa (IL)
(74) Representative: Puchberger & Partner Patentanwälte
(86) International application number: PCT/IL2020/051252
(87) International publication number: WO 2021/111450

(56) References cited:
- WO-A2-2015/075471
- WO-A2-2017/212489
- US-A1- 2019 105 520
- US-A1- 2019 224 501

## Description

### RELATED APPLICATION

This application claims the benefit of priority under 35 USC §119(e) ofU.S. from Provisional Patent Application No. 62/943,309 filed 4 December 2019, and from international application publication number WO2020/194312 filed 26 March 2020.

### FIELD AND BACKGROUND OF THE INVENTION

The present invention, in some embodiments thereof, relates to system for skin treatments and, more particularly, but not exclusively, to an ultrasound system for skin treatments.

International Patent Application Publication No. WO2015075471A2 describes, "an ultrasound transducer array comprising at least one composite material layer. The layer comprises a polymer base in which polymer particles are embedded. The polymer particles are coated with a material that has a thermal conductivity that is higher than the thermal conductivity of the polymer base and the polymer particles" (Abstract).

International Patent Application Publication No. WO2017212489A2 describes, "some embodiments of the invention relate to an applicator for applying ultrasound energy to a tissue volume, comprising: an array comprising a plurality of ultrasound transducers, the transducers arranged side by side, the transducers configured to emit unfocused ultrasound energy suitable to thermally damage at least a portion of the tissue volume, each of the transducers comprising a coating thin enough so as not to substantially affect heat transfer via the coating to the tissue; and a cooling module configured to apply cooling via the transducers to prevent overheating of a surface of the tissue volume being contacted by the transducers" (Abstract).

### SUMMARY OF THE INVENTION

The invention is defined by the appended claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present invention may be embodied as a system, method or computer program product. Accordingly, some embodiments of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, some embodiments of the present invention may take the form of a computer program product embodied in one or more computer readable medium(s) having computer readable program code embodied thereon. Implementation of the method and/or system of some embodiments of the invention can involve performing and/or completing selected tasks manually, automatically, or a combination thereof. Moreover, according to actual instrumentation and equipment of some embodiments of the method and/or system of the invention, several selected tasks could be implemented by hardware, by software or by firmware and/or by a combination thereof, e.g., using an operating system.

For example, hardware for performing selected tasks according to some embodiments of the invention could be implemented as a chip or a circuit. As software, selected tasks according to some embodiments of the invention could be implemented as a plurality of software instructions being executed by a computer using any suitable operating system. In an exemplary embodiment of the invention, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the invention. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present invention may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as measuring skin temperature, measuring transducers temperature, and/or measuring at least one parameter related to the RF power signal, might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWINGS

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1A is a flow chart of a general process for delivery of ultrasound waves, according to some embodiments of the invention;
Fig. 1B is a schematic illustration showing connections between features of an ultrasound applicator and the applicator mean time between failures (MTBF), according to some exemplary embodiments of the invention;
Fig. 1C is a flow chart of a process for activating and monitoring the activation of an ultrasound applicator, according to some exemplary embodiments of the invention;
Fig. 2A is a schematic illustration showing penetration of ultrasonic energy into deep layers of the skin while cooling the skin surface, according to some embodiments of the invention;
Fig. 2B is a schematic illustration showing a penetration depth of the ultrasonic waves, according to some embodiments of the invention;
Figs. 2C and 2D are schematic illustrations showing heating of large tissue volumes located deep inside the skin, according to some embodiments of the invention;
Figs. 2E-2H are schematic illustrations of different types of ultrasound transducers, and the tissue volumes affected by the ultrasound transducers, according to some exemplary embodiments of the invention;
Fig. 2I is a schematic illustration showing heating of large spaced-apart tissue volumes deep inside the skin by a plurality of ultrasonic transducers of a single applicator, according to some embodiments of the invention;
Fig. 3A is a schematic block diagram of a system for delivery of ultrasonic waves for skin treatments, according to some embodiments of the invention;
Fig. 3B is a schematic illustration of a system and system components for delivery of ultrasonic waves for skin treatments, according to some embodiments of the invention;
Fig. 3C is a flow chart of a skin treatment process, according to some embodiments of the invention;
Fig. 4A is a flow chart of a detailed process including user actions when delivering ultrasonic energy for skin treatments, according to some embodiments of the invention;
Fig. 4B is a flow chart of a detailed process including system actions when delivering ultrasonic energy for skin treatments, according to some embodiments of the invention;
Figs. 5A-5C are graphs showing activation modes of a system for delivery of ultrasonic energy for skin treatments, according to some embodiments of the invention;
Figs. 6A-6B are graphs showing skin contact monitoring, according to some embodiments of the invention;
Figs. 6C-6D are graphs showing impedance monitoring, according to some embodiments of the invention;
Fig. 6E is a flow chart of a process for identifying degradation in potential efficiency of ultrasound energy generation and/or delivery, according to some exemplary embodiments of the invention;
Fig. 6F is a flow chart of a process for modifying activation of one or more ultrasound transducers and/or at least one parameter of an ultrasound treatment according to impedance changes, according to some exemplary embodiments of the invention;
Figs. 7A-7C are schematic illustrations of a system including a control console and an applicator for delivery of skin treatments, according to some embodiments of the invention;
Figs. 8A-8C are schematic illustrations of an applicator, according to some embodiments of the invention;
Figs. 9A-9B are schematic illustrations of a user interface of an applicator, according to some embodiments of the invention;
Figs. 10A-10D are schematic illustrations showing elements of an energizer unit of an applicator, according to some embodiments of the invention;
Fig. 11A and 11B are schematic cross-section illustrations showing interactions between layers of an energizer unit, according to some embodiments of the invention;
Figs. 12A-12B are schematic illustrations showing welding of a planar strip to upper PZT electrode, according to some embodiments of the invention;
Figs. 13A-13D are schematic illustrations of a flex PCB, according to some embodiments of the invention;
Figs. 14A-14B are schematic illustrations of a holder of an energizer unit, according to some embodiments of the invention;
Figs. 15A-15D are schematic illustrations of a transducers PCB of an energizing unit, according to some embodiments of the invention;
Fig. 15E is a schematic diagram showing collection and transmission of measurements from the energizer to the console, according to some exemplary embodiments of the invention;
Fig. 16A is an image showing sealing of the applicator, according to some embodiments of the invention;
Figs. 16B-16D are schematic illustrations of an applicator assembly and components, according to some exemplary embodiments of the invention;
Fig. 17 is a schematic illustration of an applicator and umbilical connecting channel, showing connectors of the umbilical connecting channel, according to some embodiments of the invention;
Figs. 18A-18C are schematic illustrations of a console of a system for delivery of ultrasonic waves, according to some embodiments of the invention;
Fig. 19A is a schematic block diagram showing electric power measurements is ultrasound channels, according to some embodiments of the invention;
Figs. 19B is a schematic illustrations of an ultrasound power card, according to some embodiments of the invention;
Fig. 19C-19E is a schematic illustrations of a connection between two or more ultrasound cards to a main board in the console, according to some embodiments of the invention;
Fig. 20 is a block diagram of a cooling system of a system for delivery of ultrasonic waves, according to some embodiments of the invention; and
Figs. 21A-21B are screen shots of a visual interface, according to some embodiments of the invention

### DESCRIPTION OF SPECIFIC EMBODIMENTS OF THE INVENTION

The present invention, in some embodiments thereof, relates to an ultrasound system, and, more particularly, but not exclusively, to an ultrasound system for skin treatment.

According to some embodiments, ultrasound energy is delivered in the form of ultrasound waves into tissue layers located at different depths underneath the skin surface. In some embodiments, the ultrasound energy is optionally delivered in the way of converging (focused) or non-converging ultrasound waves. In some embodiments, the interaction of ultrasound waves with the tissue generates a thermal effect used to heat different tissue layers. In some embodiments, the heated tissue layers optionally change their shape, volume, and texture. Optionally the changes of the tissue layers affect the shape of the skin surface, for example, the skin surface above the heated layers and could result in skin tightening and additional cosmetic effects. In some embodiments, ultrasound transducers placed in contact with the skin optionally deliver the ultrasound energy through the skin surface to the deep tissue layers. In some embodiments, skin surface cooling prevents damage or injury of the skin surface by the heat generated by the interaction of tissue with ultrasound energy.

According to some embodiments, devices termed applicators deliver the ultrasound energy to the skin. In some embodiments, an applicator contains an ultrasound transducer connected to a source of ultrasound energy. In some embodiments, an ultrasound transducer comprises a PZT element. Alternatively, an ultrasound transducer comprises other elements configured to vibrate when electrified. In some embodiments, coupling of the ultrasound energy to the skin or tissue optionally heats a single skin or tissue volume. In some embodiments, the applicators are configured to support easy translation over the skin surface and apply the ultrasound to larger skin areas. In some embodiments, the applicators or systems optionally provide ultrasound energy and skin temperature monitoring. In some embodiments, the monitoring of the treatment parameters provides the operator with instant feedback related to the ultrasound treatment regimen.

A broad aspect of some embodiments relates to delivery of ultrasonic energy into deep layers of the skin in a reliable, robust and safe way. In some embodiments, the ultrasonic energy is delivered as part of a therapeutic treatment of the skin, for example Acne, Scars, Hypohidrosis, Malanoma, Basal Cell Carcinoma and other. Alternatively, the ultrasonic energy is delivered as part of a cosmetic non-therapeutic treatment of the skin. In some embodiments, in order to deliver ultrasound energy in a reliable, robust and/or safe way, the system, for example the ultrasound applicator is assembled and built to generate and deliver ultrasound energy for a time period of at least two weeks, for example at least 3 weeks, at least one month, when treating 2 or more subjects. Additionally, the system continuously or intermittently monitors the activity of the ultrasound applicator, for example the activity of each ultrasound transducer during the activation, to identify potential degradation in an efficiency of ultrasound energy generation and/or delivery. For example, the system monitors one or more electrical parameters of the ultrasound transducers. In some embodiments, changes in values of the monitored electrical parameters over time and/or between ultrasound transducers of the same applicator, optionally indicate malfunction of one or more ultrasound transducers, for example degradation of the one or more ultrasound transducers. Alternatively or additionally changes in values of the monitored electrical parameters over time and/or between ultrasound transducers optionally indicate usage of the ultrasound applicator not according to authorized activation guidelines.

According to some embodiments, degradation of an ultrasound transducer, for example degradation in at least one of, activity of the ultrasound transducer, an efficiency of the ultrasound transducer to convert electricity into vibrations, degradation in an efficiency of the ultrasound transducer to generate ultrasound energy, degradation in an efficiency of the ultrasound transducer to deliver ultrasound energy to the tissue, is detected by monitoring impedance or changes in impedance. In some embodiments, the degradation of the ultrasound transducer is detected by separately monitoring impedance or changes in impedance for each ultrasound transducer in an ultrasound applicator, for example relative to a reference or a baseline value.

Additionally or alternatively, degradation of an ultrasound transducer is detected by monitoring at least one other electrical parameter or changes in the electrical parameter, for example the reflected power from the ultrasound transducer, a ratio value between the power delivered to the ultrasound transducer and the power reflected from the ultrasound transducer, capacitance of the transducer, capacitance between the transducer and the skin, and skin bioimpedance change before and after the delivery of an ultrasound energy pulse. It is expected that changes in some of the electrical parameters listed above will have high correlation with changes in impedance, for example changes in impedance of the ultrasound transducer. Optional changes in the electrical parameters due to transducer degradation are listed in the table below:

| # | Parameter | Change due to transducer degradation |
|---|---|---|
| 1. | P_rev (the reflected power) | Increase |
| 2. | Power Ratio P_fwd/P_rev | Decrease |
| 3. | Transducer capacitance | Decrease |
| 4. | Capacitance between the transducer and the skin | Decrease |
| 5. | Skin bioimpedance change before/after pulse | Decrease |

Additionally or alternatively, in order to deliver ultrasound energy in a reliable, robust and/or safe way, temperature of the ultrasound transducers and/or the skin contacting the ultrasound transducers is monitored continuously or intermittently during the activation of the ultrasound transducers. In some embodiments, changes in temperature values optionally indicate malfunction of one or more transducers and/or usage of the ultrasound applicator not according to authorized activation guidelines.

According to some embodiments, the ultrasonic energy delivery system is configured to deliver ultrasonic energy to the skin layer without damaging the skin surface. Alternatively or additionally, the system is designed to deliver ultrasonic energy in an accurate and a repeatable way, for example to allow a measurable and/or a safe effect. In some embodiments, the system is configured to accurately measure the effect of the delivered energy on the tissue, and/or to deliver feedback to a user regarding the effect and/or the system operation in a clear way.

According to some embodiments, the system is configured to accurately measure parameters related to the ultrasound waves, and power signals generating the ultrasound waves. In some embodiments, the system is configured to monitor a thermal effect on the skin, for example by directly recording temperature of the skin and/or by recording temperature of applicator elements, for example transducers temperature and/or transducers holder temperature.

According to some embodiments, the system is configured to provide feedback, for example on-line feedback during the delivery of ultrasound waves, to an operator of the system regarding the activity of the system components and the effect on the subject.

According to some embodiments, the ultrasound applicator is configured to optionally have a mean time between failures (MTBF) of at least 21 days, for example at least 28 days, at least 2 months, at least 3 months, or any intermediate, shorter or longer time period, when delivering an average of at least 500 ultrasound waves pulses per day, for example, at least 800 pulses per day, at least 1000 pulses per day, at least 2000 pulses per day or any intermediate, smaller or larger number of pulses per day. In some embodiments, a failure of the connector means at least one of inability to deliver ultrasound waves.

An aspect of some embodiments relates to reducing a distance between an ultrasound transducer and an emitting surface of an ultrasound applicator contacting the skin. In some embodiments, the distance is reduced, for example to allow efficient ultrasound energy delivery between the ultrasound transducer and the skin contacting the emitting surface of the ultrasound applicator. Alternatively or additionally, the distance is reduced, for example to allow accurate temperature measurements of the skin by temperature sensors located near the emitting surface of the applicator.

According to some exemplary embodiments, the distance is reduced, for example by attaching non-passivated electrical conductors or wiring to each of the ultrasound transducers between the ultrasound transducer and the emitting surface of the applicator. Alternatively, the distance is reduced by electrifying each of the ultrasound transducers through a base, for example a base holder, on which a bottom surface of the ultrasound transducer is positioned. In some embodiments, the distance is reduced by coating the non-passivated electrical wiring and the ultrasound transducer with a thin coating of an electrical insulation polymer, for example Parylene.

An aspect of some embodiments relates to detecting changes in impedance values of an ultrasound transducer relative to a reference value, during the activation of the ultrasound transducers. In some embodiments,the detected changes in impedance values optionally indicate malfunction in an activity of the ultrasound transducer. In some embodiments, the impedance values and changes thereof are monitored continuously or intermittently during the activation of the ultrasound transducer. In some embodiments, the impedance values and changes thereof are monitored separately for each ultrasound transducer of the applicator.

An aspect of some embodiments relates to preventing physical damage to the skin surface by attaching a planar strip having a planar surface, to a surface of a PZT element of an applicator. In some embodiments, the planar surface of the wide strip faces and/or is shaped and sized for contacting skin surface. In some embodiments, the planar surface of the strip, is, for example, a surface for contacting skin surface that does not contain any bulges and/or protrusions, is positioned between a PZT element and the skin surface.

According to some embodiments, the wide strip is an electrical conductor configured to conduct electricity to the PZT element. In some embodiments, the wide strip electrical conductor is coated or is made from an electrical conducting material, for example silver. In some embodiments, the wide strip is attached to an upper surface of the PZT element, for example a surface of the PZT element facing the skin, by welding, for example spark welding. In some embodiments, the wide strip is not attached to the surface of the PZT element by soldering.

According to some embodiments, the wide strip comprises a silver ribbon, for example a wide silver ribbon. In some embodiments, a width of the wide strip is in a range of 0.5mm to 5mm, for example 0.5mm-1.5mm, 2mm-4mm, 3mm-5mm or any intermediate smaller or larger width. In some embodiments, the width of the wide strip is adjusted to a width of a surface of the PZT element facing the skin, for example an upper surface of the PZT element.

According to some embodiments, the wide strip has a bigger cross section relative to a wire, for example to conduct higher electrical currents. In some embodiments, attachment of the strip, for example a planar wide strip to the PZT by welding and not soldering allows, for example to prevent solder usage, and therefore to allow repeatable transmission-block electrode area coverage.

An aspect of some embodiments related to placing a PZT element on a surface of a base layer made from a material with controlled stiffness. In some embodiments, the base layer comprises conductive particles, for example electrical conductive particles, embedded in a non-conductive filler, for example a material that does not conduct electrical power or that conduct electrical power with low efficiency of less than 30%, for example less than 20%, less than 10% or any intermediate, smaller or larger percentage value, relative to the electrical conductivity efficiency of the conductive particles. In some embodiments, the non-conductive material is a non-conductive solidified material, for example, a non-conductive solidified adhesive, configured to solidify over time or in response to force, a chemical or a physical reaction.

According to some embodiments, the base layer is positioned between at least one electrode of the PZT element and an electrical conductor, for example, an electrically conducting wire, and/or an electrically conducting foil. In some embodiments, the conductive particles mechanically contact the electrical conductor and the at least one PZT electrode, and are configured to deliver electrical power between the electrical conductor and the at least one PZT electrode.

According to the invention, the conductive particles are aligned as a monolayer, for example a single, closely packed layer of conductive particles, within non-conductive material between the at least one PZT electrode and the electrical conductor. According to the invention, the conductive particles are fixed as a monolayer within the non-conductive adhesive while contacting the electrical conductor and the at least one PZT electrode, for example at different contacting points of the particles. In some embodiments, the conductive particles have a similar size, and/or a similar diameter. Alternatively, the size and/or diameter of the particles vary in less than 20%, for example less than 10%, less than 5%, less than 2%, less than 1% or any intermediate, smaller or larger value.

According to some embodiments, at least 25% of the conductive particles, for example at least 50%, at least 80%, at least 90%, at least 95% or any intermediate, smaller or larger percentage of the particles is formed from a rigid core surrounds at least partly with a soft layer, which is softer relative to the rigid core. In some embodiments, at least one or both of the at least one PZT electrode and the electrical conductor contact the soft layer of each of the conducting particles at different contacting points.

According to some embodiments, at least 30% of the conductive particles, for example at least 50%, at least 80%, at least 90%, at least 95%, 100% or any intermediate or smaller percentage of the particles have the same shape, for example have a spherical shape or a round shape. In some embodiments, at least 30% of the conductive particles, for example at least 50%, at least 80%, at least 90%, at least 95%, 100% or any intermediate or smaller percentage of the particles, are thermal conductive, for example allow to conduct heat away from the PZT element.

According to some embodiments, at least 25% of the conductive particles, for example at least 50%, at least 80%, at least 90%, at least 95% or any intermediate, smaller or larger percentage of the particles is formed from a non-conductive rigid core, surrounded at least partly by an electrical conductive layer. In some embodiments, the non-conductive rigid core is made from a non-conductive polymer or a combination of non-conductive polymers, for example polyurethane, polystyrene, polyimide, PEEK, Nylon. In some embodiments, the conductive surrounding layer comprises one or more of Silver, copper and Tungsten or any electrical conducting metal material or combination of metal materials.

A potential advantage of having a non-conductive material that includes a plurality of round conductive particles in a very similar or identical diameter, may be to allow a very even mechanical support of the PZT element, if mechanical forces are applied at the PZT towards the non-conductive layer. An additional potential advantage of the plurality of conductive particles may be to allow high electric conductivity between at least one PZT electrode and an electric conductor, through the plurality of conductive particles.

A potential advantage of having conductive particles with a rigid polymer core and a softer surrounding layer placed in contact with the PZT may be to allow less dampening of the PZT vibration compared to particles formed entirely from a rigid material, for example a rigid polymer. An additional potential advantage of round conductive particles with a soft surrounding layer may be to reduce dampening of the PZT vibration by allowing a contact point between the round conductive particles and the PZT that has a minimal contact area due to the curved outer surface of the particle.

An additional potential advantage of aligning the conductive particles as a monolayer between the PZT and an electrical conductor may be to increase the heat conductivity from the PZT through the conductive particles, which is important for optimized cooling of the skin through the transducer surface.

In some embodiments, a surface of the PZT lower electrode is positioned on a conductive film, for example a conductive glue film. In some embodiments, the conductive glue film comprises a double-sided conductive glue film. In some embodiments, the conductive glue film comprises particles, for example particles with an even diameter.

According to some embodiments, the distribution of the conductive particles inside the glue film determines a stiffness of the glue film. Optionally, pressing the PZT into the glue film, distributes the particles to generate a single-layer of conductive particles between the PZT and a lower, surface, for example a stiffer surface underneath the glue film. In some embodiments, the single layer of the conductive particles having a similar diameter, is a layer with even thickness.

Some potential advantages of placing the PZT on a single layer of electrical conducting particles may include continuous and even mechanical support of PZT to prevent bending of the PZT when pressing the PZT, for example against the skin surface, better resistance to external mechanical stresses and pressing the PZT as part of the assembly process. An additional potential advantage may be using standard commercial PCB automated flat assembly technologies, for example for mass production.

An aspect of some embodiments relates to using a PZT element made from a stiff material. In some embodiments, the PZT is made from a material having a mechanical quality factor (Qm) larger than 600, for example 800, 1000, 1200 or any intermediate, smaller or larger value. In some embodiments, the PZT element is made from a material having a Qm of at least 950, for example 1000, 1050, 1100 or any intermediate, smaller or larger Qm value. In some embodiments, using a PZT element with a high Qm value allows, for example, high energy conversion efficiency.

As used herein, mechanical quality factor is a non-dimensional factor indicating the mechanical loss of a component under dynamic operating conditions.

Additionally or alternatively, the PZT is made from a material with a high Curie temperature, for example a Curie temperature of at least 250°C, for example, 300°C, 320°C, 350°C, 380°C or any intermediate, smaller or larger Curie temperature.

Some potential advantages of a PZT element made from a stiff material, and/or a material with a high Curie temperature may include a possibility to apply relatively high pressing stresses of up to 500 MPa, UP TO 400 MPa, up to 300MPa or any intermediate, smaller or larger value, on the PZT without breakage, for example to allow use of PCB automated flat assembly technologies for mass production, for example pick-and-place technologies and/or to allow easy manipulation for manual assembly.

Additional potential advantages of using a PZT element made from a stiff material, and/or a material with a high Curie temperature may include a possibility to heat an energizer assembly of an applicator during a manufacturing process up to 50% of the Curie temp = ~160°C without losing the piezoelectric properties, using a standard bi-component glue curing temperatures (100-120°C) and other standard commercial PCB automated flat assembly technologies for mass production, and applying manufacturing processes of wave soldering in a range of 180°C - 240°C for short periods (up to 30 min).

An aspect of some embodiments relates to having PZT electrodes with a thin electrode layer having a thickness of up to 15µm, for example up to 12 µm, up to 10 µm, up to 8 µm, up to 5 µm or any intermediate, smaller or larger range of values.

Some potential advantages of using thin PZT electrodes of up to 10µm may include allowing easy soldering processes for electric connection and/or a very good heat conduction on the PZT surface, for example to prevent heat differences (heat points) which optionally can cause local thermal damage to the skin or to the adhesion glue.

An aspect of some embodiments relates to a polyimide PCB, for example a Kapton PCB positioned on top at least one PZT element. In some embodiments, the polyimide PCB is a flex polyimide PCB, for example a flex Kapton PCB. In some embodiments, the flex Kapton PCB comprises a thermistor circuitry. In some embodiments, the flex Kapton PCB is thinner above the PZT elements, for example to allow better energy transmission from the PZT elements to the tissue through the PCB, compared to other regions.

According to some embodiments, a thickness of the flex Kapton PCB above the PZT elements is in a range of 10µm-35µm, for example 10µm-20µm, 18µm-28µm, 20µm-35µm or any intermediate, smaller or larger range of values. In some embodiments, a thickness of the flex Kapton PCB in regions configured to be placed away from the PZT elements is in a range of 70µm-120µm, for example 70µm -90µm, 80µm-110µm, 90µm-120µm or any intermediate, smaller or larger range of values. In some embodiments, no passivation layer is formed in the PZT region and on the thermistor circuitry, for example thermistor copper circuitry, in the PZT region of the flex Kapton PCB.

According to some embodiments, electrical wiring or conductors located on top or near ultrasound transducers are not passivated, for example to reduce a distance between the ultrasound transducers and the skin and/or to prevent loss of ultrasound energy due to the passivation layer. In some embodiments, electrical wiring and/or electrical conductors connected to each ultrasound transducer are optionally not passivated. Additionally or alternatively, electrical wiring and/or electrical conductors comprising temperature sensors, for example thermistors positioned near ultrasound transducers at the emitting surface of the ultrasound applicator are not passivated.

According to some embodiments, the non-passivated electrical wiring and/or electrical conductors are isolated, for example electrically isolated by an isolation layer. In some embodiments, the isolation layer isolates the electrical wiring and/or electrical conductors against interaction with fluids and/or air. Additionally, the isolation layer prevents shorting between non-passivated electrical wiring and/or electrical conductors electrifying the ultrasound transducers and non-passivated electrical wiring and/or electrical conductors comprising the thermistors.

According to some exemplary embodiments, the isolation layer comprises a strong adhesive, for example a strong Epoxy glue, configured to attach non-passivated electrical wiring and/or electrical conductors to the ultrasound transducers. Additionally, the isolation layer comprises a thin coating of Kapton insulation having a thickness in a range of 12-50 µm, for example 12-20µm, 10-25µm or any intermediate, smaller or larger range of values, covering the electrical wiring and/or electrical conductors and the ultrasound transducers. In some embodiments, the thin coating of Kapton is applied under vacuum conditions as a gas. In some embodiments, the isolation layer includes a layer of Parylene which is a trade name for a variety of chemical vapor deposited poly(p-xylylene) polymers used as moisture and dielectric barriers, where Parylene C is the most frequently used polymer, covering the Kapton and positioned between the ultrasound transducers and a skin surface.

Some potential advantage of the flex Kapton PCB may be sealing of the energizer face from water/humidity/Ultrasound gel etc. In addition, the relatively small thickness may allow the ultrasonic energy to pass through the flex PCB material with negligible energy loss. The relative stiffness of Polyimide may allow resistance to physical impacts from the environment, thus preserving the sealing. In addition, the flex Kapton PCB allows (1) manufacturing in standard mass production methods to allow low production costs, and (2) electric isolation from the skin/surrounding with an electric breakage voltage of 300V-1500V, for example 300V-800V, 600V-1200V, 1000V-1500V or any intermediate, smaller or larger value.

According to some embodiments, the flex PCB is flipped so the electric circuit is not facing the skin. In some embodiments, the flex PCB is glued on the PZT and the frame, for example to allow the thermistors be positioned in place between the PZT elements, and the circuit connector reaches the connector in the applicator main PCB in the correct length.

Some potential advantages of the interaction between a flex PCB, for example a thin flex PCB and the thermistors may be fast heat transfer from the skin to the thermistors, for example to allow continually updated temperature measurement of the skin. In addition, the thermistors arrangement may allow correct positioning of the flex in place on the frame with the PZT, for example by positioning the thermistors in specific grooves in the frame. The flex PCB may also allow simple electric connection of the flex circuit to the applicator main PCB, which optionally simplifies the assembly process.

An aspect of some embodiments relates to gluing the flex PCB on the PZT by applying a glue, pressing and curing in an oven. In some embodiments, the glue, for example an Epoxy glue is pressed during the curing process. In some embodiments, the glue comprises the EPO-TEK^{®} 353ND Epoxy glue. In some embodiments, the glue is applied between the PZT, the frame and the flex prior to pressing, for example horizontal or lateral pressing. In some embodiments, the oven is heated to a temperature in a range of 100°C-150°C, for example 100°C-130°C, 110°C-140°C, 120°C-150°C, or any intermediate, smaller or larger range of temperatures, during the curing process.

A potential advantage of pressing the glue during the curing may be to allow gluing of the flex to the PZT upper silver electrode with a very thin (~20um) glue layer, and therefore minimal ultrasonic energy losses in the glue layer. A potential advantage of using the EpoTEK ND353 glue is that it may allow good adhesion of the flex PCB to the PZT silver electrode. An additional potential advantage of using the EpoTEK ND353 glue is that it may allow mechanically fixing and support of the entire structure of silver strips and PZT elements, which are weakly supported on the soft conductive glue and particles film.

An aspect of some embodiments relates to a frame configured to cover at least partly the holder, having smooth rounded edges. In some embodiments, an external surface of the frame has smooth rounded edges, for example to allow tight gluing of the flex PCB on the frame, in big enough bending radiuses of the flex copper lead circuits, for example to prevent damage to the copper leads. In addition, the frame with the smooth rounded edges allows, for example good water sealing by the gasket, between the frame and the external cover, and utilizing injection molding manufacturing for mass production.

An aspect of some embodiments relates to having a holder of PZT elements with a width larger than a width of a PZT element surface contacting the holder. In some embodiments, a width of the surfaces of the holder on which the PZT is positioned, is at least 10% wider than a width of a surface of the PZT element contacting the holder. In some embodiments, a surface of the holder shaped and sized to be attached to a PZT element has a width in a range of 1.3mm-2mm, for example 1.3mm-1.5mm, 1.4mm-1.7mm, 1.6mm- 1.8mm, 1.7mm-2mm or any intermediate, smaller or larger width. In some embodiments, a width of a surface of the PZT element shaped and sized to contact the holder is in a range of 0.8mm-1.2mm, for example 0.8mm-1mm, 0.9mm-1.1mm 1mm-1.2mm or any intermediate, smaller or larger range of values. 1.6mm wide, and not 1mm wide like the PZT element.

A potential advantage of having a wider holder surface compared to a PZT element width may be to allow for the ND353 glue to accumulate on the sides of the PZT elements for better mechanical support and adhesion of the PZT to the holder surface from the PZT sides, for sealing of the PZT from Parylene penetration between the PZT electrode and the glue, and for allowing small errors in alignment in the positioning of the PZT on the holder surface.

An aspect of some embodiments relates to electrically isolating a PZT element and electrical wiring thereof, from other PZT elements and/or from the holder. In some embodiments, a copper strip that is electrically connected to a lower PZT electrode is electrically isolated from the holder, for example from the aluminum holder. Optionally, the copper strip is isolated from the holder by a non-conductive glue film, and/or by a passivation of the Aluminum surface.

A potential advantage of electrically isolating a PZT element may be to allow capacitance and/or impedance measurements of each PZT separately, with no errors/noises from other PZT elements, and optionally no capacitive effects of water electrolytes on the flex external surface. An additional potential advantage may be to allow operation of different transducers in different phase and/or different frequencies.

An aspect of some embodiments relates to a main applicator having a PCB with a rigid-flex-rigid-flex-rigid PCB, with optionally 50 Ohm matching, in all parts, for example to allow bending of the PCB structure to fit on the holder structure, to allow an easy assembly process and to allow transfer of the RF signals in the flex.

An aspect of some embodiments relates to a flex part of a PCB having RF power channels that are to the ground on both sides of the flex part, for example to allow low EMC, low disturbance or changes in channels capacitance and impedances , and no need for connectors and/or coax wires soldering.

An aspect of some embodiments relates to positioning soldering footpads of the applicator main PCB on "bars" in each side of the PZT, for example to allow shorter distance of bottom copper strip and upper silver strip from the PZT to the pad, easier assembly, less EMC, and less parasitic capacitance and/or impedance variations.

An aspect of some embodiments relates to a flex PCB with electrical wiring for measuring temperature of the holder separately from temperatures of the PZT elements. In some embodiments, a "RF phase bar" of the applicator PCB includes a short extension flex on which the holder temperature thermistor is positioned. In some embodiments, the flex tip+ thermistor is positioned in a hole in the holder side and glued with a heat conductive glue.

Potential advantages of a flex PCB with a separate flex wiring for measuring holder temperature may include simple assembly of the energizer for temperature measurement of the holder temperature, fast planar assembly of the applicator PCB + holder thermistor with optionally no need for manual soldering, and transfer the thermistor reading to the PCB electrical connectors through the flex part.

An aspect of some embodiments relates to a rigid board of the flex PCB which includes connectors to external wiring. In some embodiments, the rigid board of the flex PCB includes one or more connectors, for example push connectors, comprising RF, communication and/or a switch. Optionally the rigid board includes at least one indicator configured to deliver a human detectable indication, for example an LED. Additionally or alternatively, the rigid board comprises at least one flat connector.

Potential advantages of a rigid board of a flex-PCB with multiple connectors may be to allow simple assembly of connection to the applicator umbilical connecting channel, and to the switch and/or to the applicator cover. An additional advantage may be to allow simple connection of the flex electric circuitry for thermistor reading.

An aspect of some embodiments relates to a rigid board of the flex PCB comprising one or more of a measurement circuit, analog to digital (A2D) sampling and/or SPI communication to allow, for example, transmission of the temperature and button signal readings from the applicator to a control console.

Potential advantages of a rigid board having one or more of a measurement circuit, analog to digital (A2D) sampling and/or SPI communication, may be to reduce or eliminate effects of EMC noises or capacitance errors on the transmitted readings accuracy to the console, to cancel a need for multiple wires for analog readings, thus decreasing EMC effects, and to allow integration of multiple additional signals in the future, if needed.

An aspect of some embodiments relates to coating the energizer assembly with Parylene, for example Parylene-C. In some embodiments, spacing between the frame and the holder allows for example, penetration of the coating into the internal cavity between the frame and the holder, and to cover, optionally evenly, all surfaces.

Potential advantages of a Parylene coating may include bio-compatibility of the applicator face that is touching the patient skin, electric isolation of all of the exposed electrically conductive surfaces and corrosion prevention inside the frame cavity, mechanical stiffening and stabilization of the thin silver strips and the PZT positioning, increased electric isolation of the applicator face to the skin/surrounding, and/or increased sealing against water and humidity penetration through the applicator face.

An aspect of some embodiments relates to using standard electric connectors between a connecting channel connecting a control console and an applicator, for example an umbilical connecting channel, for RF transmission and impedance measurements.

Potential advantages of using standard connectors may be lower cost, simple assembly, no need for soldering, easy connection between the applicator and the umbilical connecting channel, for example by clicking the connectors into sockets in the applicator PCB.

An aspect of some embodiments relates to sealing both ends of an umbilical connecting channel connecting an applicator and a console, to penetration of air. In some embodiments, the umbilical connecting channel includes coolant liquid tubes, for example water tubes. In some embodiments, the umbilical connecting channel is sealed with a non-acidic sealer, for example a non-acidic RTV silicone, to prevent penetration of humid air into an inner lumen of the umbilical connecting channel.

Potential advantages of sealing an umbilical connecting channel including coolant liquid tubes to air, may be minimizing condensation of water on the water tubes, from the air inside the umbilical, minimizing water drip into the applicator space, from the umbilical air cavity, in case of condensation, and Mechanical positioning of the cables for prevention of twisting.

An aspect of some embodiments relates to separately electrically shielding electric cables inside the umbilical connecting channel. In some embodiments, an additional electric shielding covers all the cables together, and is optionally electrically connected to their shields. In some embodiments, the external shielding is electrically connected to the console chassis, and not to the RF or signal electric ground.

A potential advantage of separately electrically shielding the electric cables may be to achieve a sufficient EMC shielding according to regulatory requirements.

An aspect of some embodiments relates to an electrically conductive elongated pin in a connection between the umbilical connecting channel and the control console. In some embodiments, the pin is electrically connected to at least one electrical shielding of the umbilical connecting channel, for example to at least one electrical shielding of electric wires inside the umbilical connecting channel, and/or to an electrical shield surrounding two or more electric wires inside the umbilical connecting channel. In some embodiments, the elongated pin extends from the umbilical connecting channel towards the control console, for example towards a connecting port of the control console.

An aspect of some embodiments relates to a multiple pins connector, for example a connector having 20-70 pins, for example 20-50 pins, 40-70 pins or any intermediate, smaller or larger number of pins, of the umbilical connecting channel configured to be connected to a connector of the control console, for example, a 50 pin D-type connector. In some embodiments, the multiple pins connector is used for power RF transfer with stable and repeatable impedance measurements. In some embodiments, using a multiple pins connector, for example a standard multiple pins connector, allows, for example, to reduce costs due to the usage of standard parts, simple assembly without soldering, simple applicator assembly by clicking the connectors into the sockets in the applicator PCB.

An aspect of some embodiments relates to molding an umbilical connecting connector to the control console with soft silicon, around the multiple pins connector. For example to cover an uninsulated part of the connector and wires. In some embodiments, the molding with the soft silicon allows, for example to have an electric isolation in case of water penetration into the umbilical connecting channel or condensation, and/or to prevent corrosion.

An aspect of some embodiments relates to sealing an energizer assembly from water and air. In some embodiments, the energizer, for example after Parylene-C coating, is covered with an external applicator cover and a gasket. In some embodiments, non-acidic silicone, for example non-acidic RTV silicone is positioned around an air gap or any other gap between the external cover and the heat exchanger, to seal the gap.

Potential advantages of sealing the energized assembly from air and water may be prevention of water condensation and/or ice formation on the energizer holder, from air humidity or condensate water on the water tubes, preventing water or other liquids penetrating into the frame space and near the PZT elements, which can generate one or more of changes in the PZT mechanical support or stresses, electric impedance changes and thermistor measurement sensitivity changes.

An aspect of some embodiments relates to securing a rigid board of the flex PCB to at least one applicator cover, for example to an internal surface of the at least one applicator cover. In some embodiments, the rigid board is fixed with screws to the applicator cover, for example to the internal surface of the applicator cover. In some embodiments, pins of the cover are connected to the heat exchanger, while fixing the energizer assembly position between the cover and the heat exchanger.

Potential advantages of securing the rigid board of the flex PCB to an internal surface of the cover may be to mechanically support the flex-PCB, to prevent mechanical stresses and damage to the flex portion of the flex PCB and the thermistors flex, to provide mechanical support against pulling forces applied by the umbilical connecting channel on the flex-PCB.

An aspect of some embodiments relates to fixing the internal part of the applicator in silicone. In some embodiments, once the energizer assembly and wiring are positioned in a desired orientation within the applicator, they are covered with silicone. In some embodiments, the electric connector of the top cover switch and LED is connected to the PCB rigid board; the umbilical connecting channel insert is positioned in a desired orientation and the left applicator cover is closed and glued with a glue. In some embodiments, the applicator is positioned at an angle in a range of 30 degrees and 60 degrees or any intermediate, smaller or larger range of values, and then soft silicone molding material is added into the applicator head to fill it up to the opening for the upper cover containing a user interface. In some embodiments, silicone is added only to a limited volume inside the applicator, for example to reduce weight.

Potential advantages of filling a lumen of the applicator casing containing the energizer and electrical wiring may be prevention of water condensation on the water tubes due to penetration of humid air, preventing water dripping and penetrating into the electric connectors within the applicator, for example electric connectors on the applicator PCB and the TEC connector, electric isolation of the inner components of the applicator, preventing water dripping from the applicator head in case of condensation formation, and mechanical stabilization and stiffening of the applicator structure.

An aspect of some embodiments relates to a user interface of the applicator that is water sealed, for example to seal the user interface and/or inner lumen from external liquid, for example sweat fluids and ultrasonic gel.

An aspect of some embodiments relates to closing a casing of the applicator using adhesive and not by screws, for example to prevent opening and closing of the applicator housing without indication.

An aspect of some embodiments relates to maintaining a nearly sinusoidal power signal in an output from a low-pass filter of an ultrasound PCB by positioning air coils on the high pass filter. In some embodiments, the air coils allow, for example, correct power measurement, high efficiency energy conversion to US power. In some embodiments, the air coils allow, for example, electric power measurement according to a simple capacitor charging, which can be easily measured in a low sampling rate.

An aspect of some embodiments relates to performing forward and/or reverse power measurements on an ultrasound power card. Additionally, impedance measurements are performed on the ultrasound power card.

An aspect of some embodiments relates to an electrical shielding on the air coils of the ultrasound (US) low-pass filter. In some embodiments, the electric shielding on the air coils is connected to ground.

An aspect of some embodiments relates to an external electric shielding on the entire US power card, for example on at least 50%, for example at least 70%, at least 85% or any intermediate, smaller or larger percentage of the US power card. In some embodiments, the external electric shielding is connected to the chassis of the console and not to the ground.

According to some embodiments, the PCB RF ground is not connected to the chassis of the control console.

An aspect of some embodiments relates to connecting US power cards directly to a backplane PCB board of the control console, for example, without a harness, thereby decreasing EMC emissions and/or decreasing the complexity to building the system on the manufacturing plant.

An aspect of some embodiments relates to a chiller, of an ultrasound system cooling system positioned at a lower part of a control console. In some embodiments, the chiller comprises a pump, a fan, and a coolant liquid storage. In some embodiments, the chiller frame is not electrically connected to a frame, for example a chassis of the control console. In some embodiments, the coolant liquid storage comprises anti-freeze cooling liquid.

An aspect of some embodiments relates to EMC shielding of control console components. In some embodiments, the control console components comprise at least one surface of the chiller, US power cards, and/or at least one rotating fan included in the control console. In some embodiments, the EMC shielding is made from ferrites.

An aspect of some embodiments relates to a leaf spring of a console socket for connecting an applicator. In some embodiments, the leaf spring allows, for example, good electric contact between a pin of an umbilical connecting channel connected to shielding of the applicator, and a control console frame, for example chassis.

An aspect of some embodiments relates to a standard multiple pin socket (30-50 pins) of a console socket for connecting an applicator, for example a 50 pin D-type socket. In some embodiments, the multiple pin socket is used to deliver radiofrequency (RF) signals to the applicator. Using a multiple pin socket allows, for example, low costs due to usage of standard parts, simple assembly without soldering, and an easy assembly of the applicator.

An aspect of some embodiments relates to a display of a control console, positioned at angle of about 45 degrees, relative to a longitudinal axis of the control console. In some embodiments, positioning the display at an angle of 45 degrees allows, for example easy visualization of the display by a user of the ultrasonic energy delivery system.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary general process

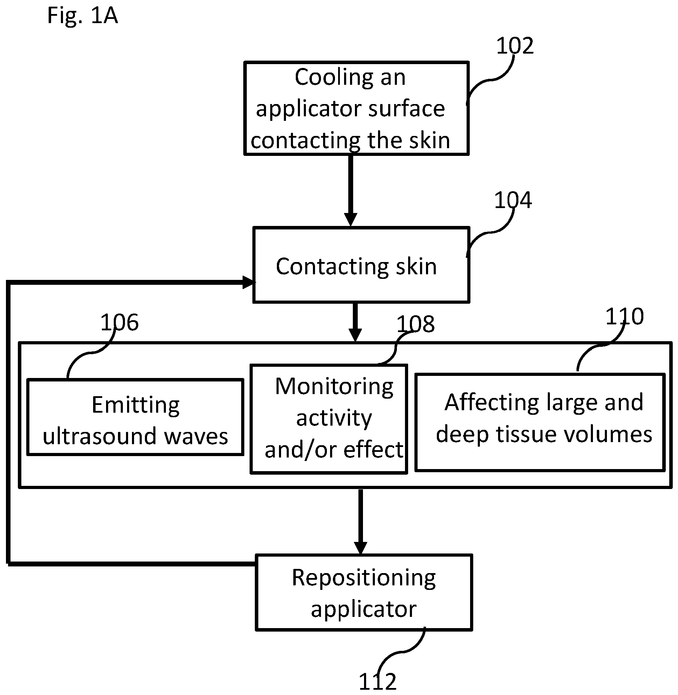

According to some exemplary embodiments, ultrasonic energy is delivered in the form of ultrasonic waves into deep tissue layers of the skin, underneath the skin surface. In some embodiments, the ultrasonic waves, for example non-converging ultrasonic waves are used to heat deep tissue layers of the skin. In some embodiments, healing of the heated deep tissue layers affects the shape of the skin surface, for example the skin surface above the heated layers. In some embodiments, healing, for example recovery of the deep tissue layers causes skin tightening. In some embodiments, heating of the deep tissue layers is performed while the skin surface is cooled down, for example to prevent injury of the skin surface from the heat generated by the transmitted ultrasonic waves. In some embodiments, the delivery of ultrasonic energy is used during cosmetic treatments in humans, and in non-human mammals. Reference is now made to fig. 1A, depicting a general process for using ultrasonic energy for skin treatments.

According to some exemplary embodiments, a surface of an ultrasound applicator shaped and sized to be placed in contact with the skin surface, is cooled at block 102. In some embodiments, the applicator surface is cooled to a temperature lower than 10°C, for example lower than 8°C, lower than 5°C, lower than 2°C or any intermediate, smaller or larger value.

According to some exemplary embodiments, the temperature of the ultrasound applicator surface is monitored during cooling, for example to prevent formation of ice fragments on the surface. In some embodiments, when ice fragments are detected, a de-freezing program is activated, for example to remove the ice fragments. Optionally, the de-freezing program is automatically activated based on the surface temperature, changes in impedance values and/or conductance values.

According to some exemplary embodiments, once the applicator surface reaches a desired temperature level, the applicator surface is placed in contact with the skin at block 104. In some embodiments, the skin temperature is monitored when the applicator surface, for example a cold applicator surface contacts the skin surface. In some embodiments, the skin temperature is monitored while the applicator surface contacts the skin surface at block 104, for example to prevent the formation of cold burns or any damage to the skin caused by the low temperature.

According to some exemplary embodiments, the applicator comprises one or more ultrasound transducers, arranged for example in an array of ultrasound transducers located near and optionally underneath the surface of the applicator configured to be placed in contact with the skin. In some embodiments, the ultrasound transducers array is a planar array. In some embodiments, the surface of the applicator comprises one or more isolating coatings, for example one or more isolating layers configured to isolate the one or more ultrasound transducers from fluid, for example sweat fluids and/or air. Alternatively or additionally, the one or more isolating layers are configured to isolate the ultrasound transducers from the skin surface, when the surface of the applicator is placed in contact with the skin. In some embodiments, the skin is cooled down by delivering cold from the applicator, through the one or more isolating layers to the skin.

According to some exemplary embodiments, ultrasound waves are emitted at block 106. In some embodiments, ultrasound waves are emitted while the surface of the applicator contacts the skin surface. In some embodiments, the ultrasound waves are emitted while the applicator cools down the skin surface. In some embodiments, the ultrasound waves are emitted from the ultrasound transducers of the applicator, through the one or more isolating layers into the skin.

According to some exemplary embodiments, the emitted ultrasound waves generate a thermal effect in deep tissue layers of the skin, at block 110. In some embodiments, the ultrasound waves affect large and deep tissue volumes in the skin. In some embodiments, the size and shape of the large tissue volumes are determined based on the size and shape of an emitting surface of each ultrasound transducer, for example a length and/or a width an emitting surface of the ultrasound transducer facing the skin. Alternatively or additionally, the size and shape of the large tissue volumes are determined based on at least one parameter of the transmitted waves, for example amplitude, frequency, and/or duration of the transmitted waves.

According to some exemplary embodiments, an arrangement pattern of the ultrasound transducers, for example a distance between adjacent transducers, a distance between a first and last ultrasound transducers in an array of ultrasound transducers, that emit ultrasound waves, determines the size and shape of treated skin surface, for example cosmetically treated skin surface.

According to some exemplary embodiments, during the delivery of ultrasonic energy into the skin by the emitted ultrasound waves, an activity of the ultrasound treatment system is monitored at block 108. Alternatively or additionally, the effect of the delivered ultrasonic energy and/or the cooling of the skin is monitored at block 108. In some embodiments, the activity of the ultrasound treatment system comprises amount of delivered energy, at least one parameter of the delivery method, one or more electrical parameters related to the US signal delivered to the ultrasound transducers, for example impedance and signal shape, and/or temperature of at least one component of the cooling system, for example holder temperature, transducers temperature, contact and/or pressure between the applicator and the skin surface.

According to some exemplary embodiments, the effect of the delivered ultrasonic energy comprises temperature of the skin surface, estimated temperature in deep tissue layers, estimated temperature distribution in the deep tissue layers, and estimated penetration depth of the ultrasound waves, estimated shape and size of the affected tissue volumes in the deep tissue layers.

According to some exemplary embodiments, the applicator is repositioned on the skin surface at block 112. In some embodiments, the applicator is positioned at a different region of the skin, for example once a predetermined amount of energy is delivered to a first skin region and/or once a desired effect is reached at the first region. In some embodiments, the applicator is repositioned as part of a previously determined treatment plan, for example a plan to treat several areas of the skin in an anatomical region, for example face, neck, submental region, or in two or more anatomical regions.

### Exemplary applicator mean time between failures

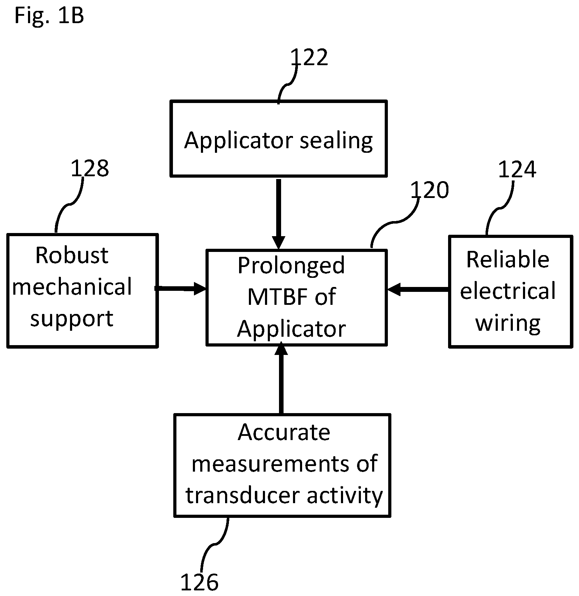

According to some exemplary embodiments, the Applicator of the ultrasound system is designed to be robust and reliable, for example, to allow long MTBF. In some embodiments, the Applicator is used for a time period of at least 21 days for example at least 28 days, at least 2 months, at least 3 months, or any intermediate, shorter or longer time period, when delivering an average of at least 500 ultrasound waves pulses per day, before a failure occurs. Reference is now made to fig. 1B, depicting features of the ultrasound applicator that contribute to a prolonged MTBF, according to some exemplary embodiments.

According to some exemplary embodiments, the Applicator is used to deliver therapeutic ultrasound waves, for example non-converging ultrasound waves into layers of the tissue. In some embodiments, the energy delivered by the ultrasound applicator heats the skin tissue layers and the surface of the skin that is placed in contact with the applicator, for example with ultrasound transducers that generate and deliver the ultrasound waves.

According to some exemplary embodiments, in order to reduce pain sensation by the patient during treatment, the skin of the patient at a treatment region contacted by the applicator is covered with a pain-reducing substance, for example a pain-reducing lotion.

One of the problems when using the applicator in a humid or wet surrounding, for example due to sweat or the pain-reducing lotion, may be penetration of liquid and/or humidity into the applicator housing that may interfere with proper functioning of the applicator. In some embodiments, in order to prevent damage to internal components of the applicator from fluids and humidity in the air and on the skin, the applicator housing is sealed to fluids from the outside.

According to some exemplary embodiments, applicator sealing 122 comprises sealing of an energizer assembly comprising the ultrasound transducers and electrical wiring, from liquid and/or air, for example by a cover that prevents direct contact with the energizer assembly. Additionally, the ultrasound transducers, for example the PZT elements are sealed, for example by at least one sealing layer that prevents direct contact between the ultrasound transducers and the skin surface. Additionally, openings and/or gaps within the applicator housing are sealed with a sealer, for example a silicone sealer. In some embodiments, the silicone comprises a roomtemperature-vulcanizing (RTV) silicone.

According to some exemplary embodiments, in order to prevent damage to the skin surface, cold is transferred through the ultrasound transducers to the skin surface contacting the transducers. In some embodiments, the applicator comprises an internal cooling system that comprises delivering of coolant fluid through the inner lumen of the applicator, in relative proximity to electrical wiring. One of the problems when using an ultrasound applicator with an internal flow of coolant fluids may be internal condensation that can affect electrical wiring within the applicator, and interfere with the proper functioning of the applicator.

According to some exemplary embodiments, in order to have reliable electrical wiring 124, the internal electrical wiring is isolated from tubes of the coolant liquid. In some embodiments, gaps within the applicator are filled with a filler that does not conduct electricity, for example a silicone filler. In some embodiments, the silicone filler comprises RTV silicone. In some embodiments, the filler contacting the coolant liquid tubes within the applicator prevents, for example condensation on the tubes, and/or contact between electrical wiring and the tubes.

Additionally, in some embodiments, electrical wires to the ultrasound transducers of the energizer are grouped in a flexible PCB, and are not physically separated from each other, for example to increase the reliability of the applicator electrical wiring.

According to some exemplary embodiments, one of the problems when heating the skin as part of a cosmetic and/or a therapeutic process may be how to generate a desired effect without damaging the skin surface. In some embodiments, the skin surface may be damaged when one or more of the transducers is not working properly and/or when the applicator, for example at least one transducer over heats the skin surface. In addition, in some embodiments, the generated effect may not be a desired effect, for example if one or more of the transducers is not placed in contact with the skin surface.

According to some exemplary embodiments, in order to generate a desired effect on the skin tissue, accurate measurements of parameters related to the transducers activity 126 in fig. 1B, are performed. In some embodiments, measurements of the transducers activity parameters comprise measuring at least one electrical parameter of the transducers, for example current, voltage and/or impedance. Alternatively or additionally, measurements of parameters related to the transducers activity 126 comprise measurements of the transducers temperature, and/or measurements of the skin surface temperature.

According to some exemplary embodiments, in order to accurately measure transducer activity, electrical wiring to the transducers is separated from electrical wiring of temperature sensors, for example thermistors that are configured to measure the temperature of one or more ultrasound transducers and/or the temperature of the skin surface. In addition, in some embodiments, thermistors that measure skin surface temperature are positioned near, and optionally, between ultrasound transducers on an ultrasound emitting surface of the applicator that is placed in contact with skin. In some embodiments, the thermistors are covered by a thin layer of isolating material that is thin enough to allow, for example, accurate temperature sensing of the skin surface.

According to some exemplary embodiments, in order to maintain proper functioning of the applicator, the structure of the applicator, for example the mechanical support of the ultrasound transducers, is robust. In some embodiments, having robust mechanical support 128 is important, for example to maintain electrical connection to and from the ultrasound transducers over prolonged time periods.

According to some exemplary embodiments, in order to have robust mechanical support, the ultrasound transducers are fixed to electrical contacts, for example electrodes, in a firm and stable way that prevents relative movement of the transducers, when pressure is applied by the skin on the applicator surface. In some embodiments, in order to maintain robust mechanical support over time, the transducers are pressed into an adhesive layer that contains a layer of electrical conducting particles. In some embodiments, pressing the transducers into the adhesive layer fixes the position of the transducers while maintaining proper electrical connection between the transducers and electrical wiring of the ultrasound applicator.

According to some exemplary embodiments, additional robustness is achieved, for example by the filler of the applicator internal lumen. In some embodiments, the filler prevents the movement of electrical and non-electrical elements within the applicator.

### Exemplary activation and monitoring of an ultrasound applicator

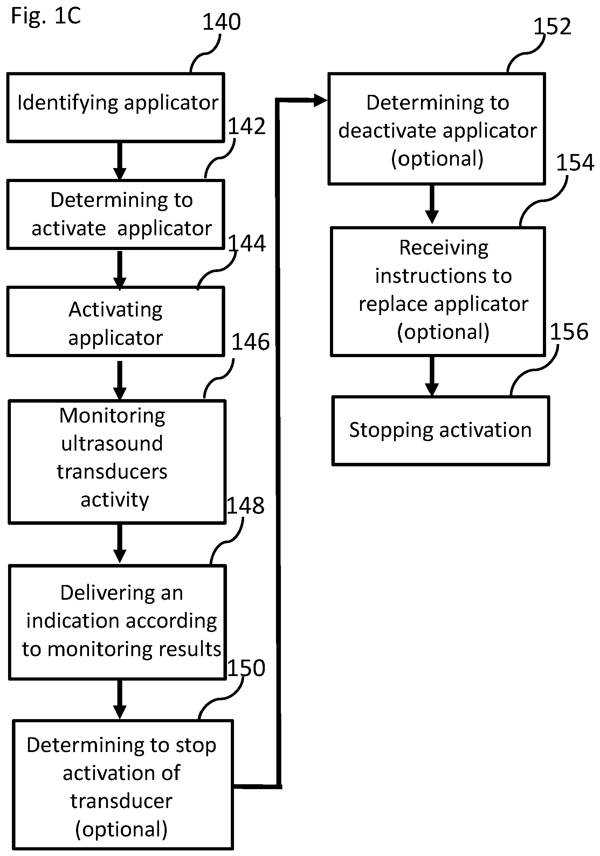

According to some exemplary embodiments, in order to provide a reliable treatment, for example a cosmetic non-therapeutic treatment, the activation of an ultrasound transducer is monitored. In some embodiments, the activation of ultrasound transducers of the ultrasound applicator is monitored, for example to identify if and when one or more ultrasound transducers is not functioning properly, for example according to predetermined parameter values and/or standards. Reference is now made to fig. 1C, depicting activation and monitoring of an ultrasound applicator, according to some exemplary embodiments of the invention.

According to some exemplary embodiments, an ultrasound applicator is identified at block 140. In some embodiments, an identification (ID) tag, for example an ID code, of the ultrasound applicator is read and inserted into a memory of a control unit, for example a control console. In some embodiments, the ID tag comprises ID information of a specific ultrasound applicator. In some embodiments, the ID tag is read by a reader of the console. Optionally, the ID tag comprises a radio-frequency identification (RFID) tag, and the console comprises a RFID reader configured to read the RFID tag of the ultrasound applicator. Optionally, the ultrasound applicator comprises an electronic circuit which includes the ID information, and a control circuitry of the console is configured to read the ID information, for example when the ultrasound applicator is coupled to the console.

According to some exemplary embodiments, the ID information comprises information associating the ultrasound applicator with a specific manufacturer, no. of ultrasound pulses generated by the ultrasound applicator, manufacturing date, manufacturing information, type of ultrasound applicator, and/or setup or calibration information of the ultrasound applicator.

According to some exemplary embodiments, a control circuitry of the control console determines if the ultrasound applicator is an authorized applicator based on the ID code or ID information associated with the applicator. In some embodiments, the control circuitry determines if the ultrasound applicator is an authorized applicator by determining a correlation between the ID code or information associated with the ultrasound applicator and at least one indication stored in a memory of the console and/or in a memory of a remote device, for example a remote computer, a remote server or a cloud storage. In some embodiments, if the ultrasound applicator is not authorized, the control circuitry of the console prevents the activation of the ultrasound applicator, for example by locking a software interface with the user. Alternatively, the control circuitry of the console prevents the activation of the ultrasound applicator, for example by mechanically locking at least one activation button in the console.

According to some exemplary embodiments, the control console, for example the control circuitry, determines to activate the ultrasound applicator at block 142. In some embodiments, the control console determines to activate the ultrasound applicator if the ultrasound applicator is authorized, for example based on the ID information associate with the ultrasound applicator.

According to some exemplary embodiments, the control console activates the ultrasound applicator, for example ultrasound transducers of the ultrasound applicator at block 144. In some embodiments, the ultrasound applicator is activated to generate at least 30,000 pulses of ultrasound energy over a time period of at least 1 week, for example at least 2 weeks, at least 1 month or any intermediate, shorter or longer time period. In some embodiments, the ultrasound applicator generates at least 30,000 pulses optionally when treating two or more subjects.

According to some exemplary embodiments, at least one activation parameter of the ultrasound applicator, is monitored at block 146. In some embodiments, at least one activation parameter is monitored during the activation of the ultrasound transducers, for example as described at block 144. In some embodiments, at least one activation parameter of two or more ultrasound transducers of the ultrasound applicator is monitored at block 146. In some embodiments, the at least one activation parameter is monitored by a control console, for example by a control circuitry. In some embodiments, at least one activation parameter comprises at least one of impedance, voltage, current, and number of pulses generated by the ultrasound applicator in total and/or by each of the ultrasound transducers. In some embodiments, at least one activation parameter is monitored separately for each ultrasound transducer of the two or more ultrasound transducers. Optionally, at least one activation parameter is monitored by measuring values of the activation parameter for each ultrasound transducer and determining a correlation between the measured values and at least one reference value.

According to some exemplary embodiments, at least one indication is generated and delivered based on the results of the monitoring, at block 148. In some embodiments, at least one indication is a human detectable indication. Optionally, at least one indication comprises an audio and/or a visual indication. In some embodiments, at least one indication is delivered to a user of the ultrasound system. Alternatively or additionally, the indication is transmitted to a remote device, for example to allow monitoring of the ultrasound applicator activity by a manufacturing of the ultrasound applicator and/or the ultrasound system.

According to some exemplary embodiments, at least one indication comprises an alert signal, for example an alert signal that is generated if the monitored at least one activation parameter is not within a desired range of values. In some embodiments, the alert signal is generated and delivered to a user of the ultrasound system. Alternatively, the alert signal is transmitted to a remote device.

According to some exemplary embodiments, the control console optionally determines, for example automatically, to stop the activation of at least one ultrasound transducer, at block 150. In some embodiments, the control console determines to stop the activation of at least one ultrasound transducer based on the results of the monitoring, for example if the monitored at least one activation parameter of the at least one ultrasound transducer is not within a desired range of values. In some embodiments, the control console delivers an indication to a user prior to and/or after stopping the activation of the at least one ultrasound transducers. Alternatively or additionally, the control console delivers an indication to a remote device prior to and/or after stopping the activation of the at least one ultrasound transducers. In some embodiments, the activation of at least one ultrasound transducer is stopped, for example if the number of pulses generated by the at least one ultrasound transducer exceeds a predetermined value.

According to some exemplary embodiments, the control console modifies one or more activation parameters, for example current and/or voltage, of other ultrasound transducers of the ultrasound applicator, after stopping the activation of at least one ultrasound transducer. In some embodiments, the control console modifies the one or more activation parameters of other ultrasound transducers, for example to compensate for the loss of ultrasound energy from the stopped at least one ultrasound applicator.

According to some exemplary embodiments, the control console receives instructions to a user to stop the activation of at least one ultrasound transducer, for example in response to the alert signal transmitted to a remote device.

According to some exemplary embodiments, the ultrasound applicator is optionally deactivated at block 152, for example by a control console. In some embodiments, the ultrasound applicator is deactivated based on the results of the monitoring performed at block 146. In some embodiments, the ultrasound applicator is deactivated, for example automatically, if activation of two or more transducers of the ultrasound applicator is stopped. In some embodiments, the ultrasound applicator is deactivated if the stopping of the two or more ultrasound transducers affects the safety and/or efficacy of the treatment. In some embodiments, the ultrasound applicator is optionally deactivated at block 152, for example if the number of pulses generated by transducers of the ultrasound applicator exceeds a predetermined value.

According to some exemplary embodiments, instructions to replace the ultrasound applicator are optionally received at block 154. In some embodiments, the instructions are received during the activation at block 144. In some embodiments, the instructions to replace the ultrasound applicator are received from a remote device. Alternatively, the instructions to replace the ultrasound applicator are generated, optionally automatically, by the control console.

According to some exemplary embodiments, the activation of the ultrasound applicator is stopped at 146, for example when a treatment session ends. In some embodiments, the activation of the ultrasound applicator is stopped automatically by the control console or according to input from a user of the ultrasound system.

### Exemplary ultrasound energy effect

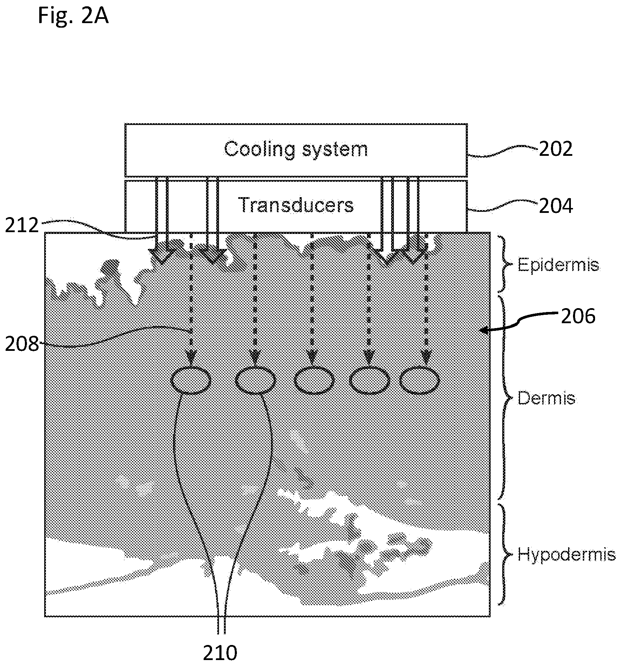

According to some exemplary embodiments, ultrasonic waves emitted from at least one ultrasound transducer, for example non-converging ultrasonic waves, penetrate into deep tissue layers. In some embodiments, while deep tissue layers are affected by the heat generated by the ultrasonic waves, superficial layers, for example layers that are placed in contact with the applicator or with ultrasound transducers of the applicator are not thermally affected by the generated heat. Reference is now made to Fig. 2A depicting a differential effect of ultrasound energy on tissue layers of the skin, according to some exemplary embodiments of the invention.

According to some exemplary embodiments, ultrasonic waves 208 are emitted into the skin 206. The skin 206 includes 3 main tissue layers: (1) Epidermis, (2) Dermis, and (3) Hypodermis. In some embodiments, transducers, for example ultrasound transducers 204 emit non-converging ultrasound energy or waves 208 with parameter values, for example frequency and/or amplitude values, that allow penetration of the ultrasonic waves 208 into the skin tissue. In some embodiments, the waves 208 penetrated into the skin deliver non-converging ultrasound energy that heat target tissue regions 210 located at a distance of at least 0.5mm, for example at least 1mm, at least 1.5mm, at least 2 mm or any intermediate, smaller or larger distance from the transducers 204. In some embodiments, the emitted ultrasound waves heat a specific tissue volume, for example a large tissue volume in each of the target regions 210.

According to some exemplary embodiments, concurrently with the emitting of the ultrasound waves, a cooling system 202 cools the epidermis layer that is in proximity to the emitting surface of the ultrasound transducers 204 or contact the transducers 204. In some embodiments, the cooling system keeps the external surface of the transducer which contacts the epidermis cool enough to prevent thermal damage to the epidermis. In some embodiments, the cooling system 202 cools the external surface of the ultrasound transducer to a temperature in a range of 5°C-35°C, for example 5°C-20°C, 10°C-30°C, 15°C-35°C or any intermediate, smaller or larger range of values. Optionally, the temperature of the external surface of the ultrasound transducer depends on the amplitude, frequency and/or duration of the ultrasound emission. In some embodiments, the cooling system 202 generates cold waves of a flow of cold 212 conducted from the cooling system 202, through the external surface of the ultrasound transducer 204 into the skin tissue 206.

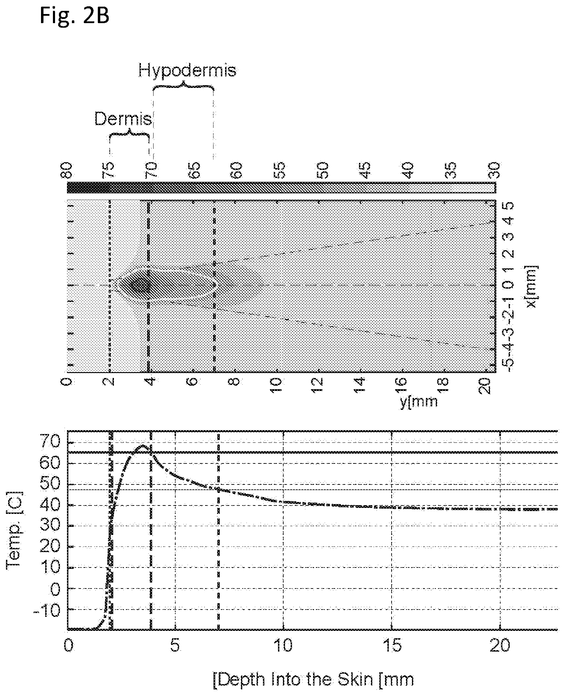

According to some exemplary embodiments, for example as described in fig. 2B, the ultrasound energy heats a tissue volume located at a depth of at least 0.5 mm, for example at least 2mm, at least 2.5mm or any intermediate, smaller or larger value, to a temperature level in a range of 50°C-70°C, for example in a range of 50°C-60°C, in a range of 55°C-65°C, in a range of 60°C-70°C or any intermediate, smaller or larger range of values. In some embodiments, the temperature gradually decreases in tissue layers of the skin deeper than 2.5mm, for example deeper than 3mm, deeper than 3.5mm or any intermediate, smaller or larger range of values. In some embodiments, by proper selection of parameters of the ultrasound power emitted into the tissue, and/or parameters of the flow of cold 212, the most heated volume of tissue could be shifted along the direction of the ultrasound wave propagation.

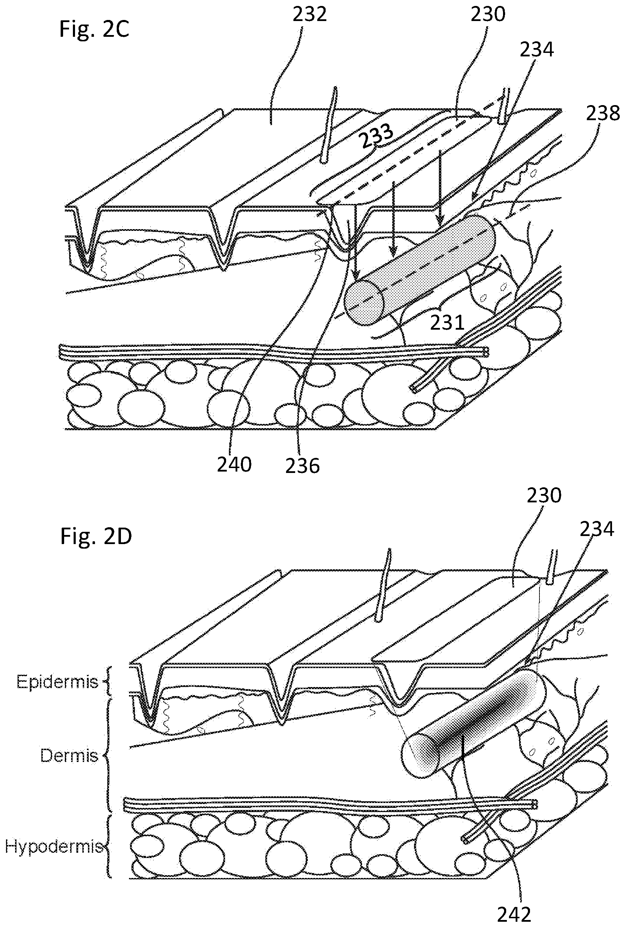

Reference is now made to figs. 2C and 2D, illustrating the heating of large deep tissue volumes, according to some exemplary embodiments of the invention.

According to some exemplary embodiments, for example as shown in figs. 2C and 2D, ultrasound waves 236 emitted from a surface of an ultrasound transducer, for example, elongated surface 230 applied to skin 232 heats an elongated tissue volume 234. In some embodiments, the elongated tissue volume has a cylindrical shape or wavy shape, having a longitudinal axis 238, which is almost parallel, for example is at an angle smaller than 10 degrees, for example smaller than 5 degrees, smaller than 3 degree, smaller than 1 degree or any intermediate, smaller or larger angle, relative to a longitudinal axis 240 of the ultrasound transducer elongated surface 230. In some embodiments, tissue composition beneath the skin surface and the heated tissue volume affects the direction of longitudinal axis 238.

According to some exemplary embodiments, a length 231 of the elongated cylindrical tissue volume 234 heated by the ultrasound waves 236 is equal or shorter in up to 10% or any intermediate, smaller or larger value, from a length 233 of the elongated surface 230 of the ultrasound transducer emitting the ultrasound waves. In some embodiments, a length 231 of the elongated cylindrical tissue volume 234 heated by the ultrasound waves 236 is 1%--15% shorter, for example 1%-5% shorter, 3% to 10% shorter, 5%-8% shorter or any intermediate, smaller or larger range of values, from a length 233 of elongated surface 230 of the ultrasound transducer emitting the ultrasound waves. In some embodiments, tissue composition beneath the skin surface could also affects the cross-section of the elongated cylindrical tissue volume 234. Optionally, the cross-section becomes uneven along longitudinal axis 238.

According to some exemplary embodiments, an array of ultrasound transducers is configured to generate and emit ultrasound waves to heat spaced apart elongated tissue volumes. Optionally, the heated elongated tissue volumes are on a plane in a depth of at least 3 mm from a surface of a skin contacting the ultrasound applicator. In some embodiments, the plane of the heated elongated tissue volumes is substantially parallel to the array of transducers.

According to some exemplary embodiments, for example as shown in fig. 2D, the heated region of a tissue volume is a tissue volume core. In some embodiments, the temperature level of an elongated core 242 of the elongated tissue volume 234 is higher than a temperature level of tissue layers surrounding the elongated core 242 within the elongated tissue volume 234. In some embodiments, the temperature of the elongated core 242 of the elongated tissue volume 234 is in a range of 50°C to 70°C, for example, in a range of 50°C-60°C, in a range of 55°C-65°C, in a range of 60°C-70°C or any intermediate, smaller or larger range of values. In some embodiments, the temperature of the elongated core 242 of the elongated tissue volume 234 is at least 50°C, for example at least 55°C, at least 65°C or any intermediate, smaller or larger value. In some embodiments, the cylindrical tissue volume heated by the ultrasound waves is located in the dermis tissue layer.

According to some exemplary embodiments, a shape and/or size of the elongated tissue volume is based on an amplitude, frequency of the ultrasound waves, distance of the ultrasound transducer from the skin surface, and/or content of the tissue layers, tissue type and tissue composition between the skin surface and the heated tissue volume. In some embodiments, the dependence of the affected tissue volume shapes on a plurality of ultrasound transducers operating parameters and tissue composition optionally indicates that other than cylindrical shapes of the heated tissue volumes could exist. In some embodiments, the shapes are optionally ellipsoidal, trapezoidal, and other types of shapes. In some embodiments, the heated tissue shapes optionally change their profile along the ultrasound applicator and in the depth of the tissue.

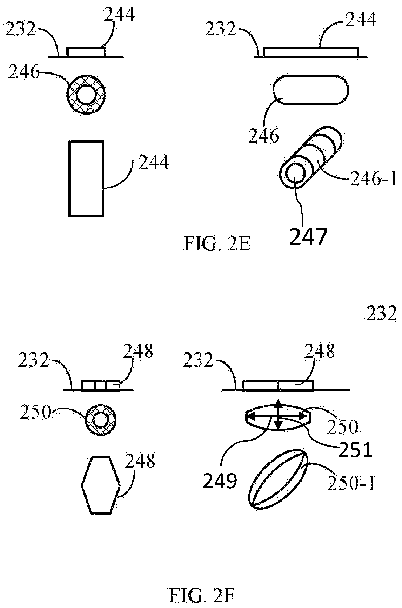

Reference is now made to figs. 2E - 2H depicting examples of different ultrasound transducers shapes and heated tissue volumes that the different transducer shapes generate, according to some exemplary embodiments of the invention.

Fig. 2E illustrates an elevation and side view of a rectangular piezo ultrasound transducer (PZT element) 244, similar to the ultrasound transducers described above and according to some exemplary embodiments of the invention. Reference numeral 246 illustrates an elongated cylindrical tissue volume heated by ultrasound transducer 244, and reference numeral 246-1 is a three-dimensional illustration of an elongated cylindrical tissue volume heated by ultrasound transducer 244.

According to some exemplary embodiments, for example as shown in fig. 2E, the ultrasound transducer comprises a rectangular PZT element. In some embodiments, the rectangular PZT element heats an elongated cylindrical tissue volume 246 and 246-1, which has open ends, for example end 247. A potential advantage of having one or two open ends may be to allow penetration of cells and/or biological material into the heated cylindrical tissue, for example to allow faster regeneration.

Fig. 2F illustrates an elevation and side view of a stretched hexagonal ultrasound transducer 248, according to some exemplary embodiments of the invention. Reference numerals 250 and 250-1 indicate *mutatis mutandis* an elongated cylindrical tissue volume 250 heated by a stretched hexagonal ultrasound transducer, and 250-1 is a three-dimensional illustration of an elongated cylindrical tissue volume heated by stretched hexagonal ultrasound transducer 244.

According to some exemplary embodiments, for example as shown in fig. 2F, the ultrasound transducer, for example a PZT element of the ultrasound transducer has an upper view of a hexagon, having a lower planar surface shaped and sized to face skin tissue. In some embodiments, the hexagon-shaped ultrasound transducer heats an elongated cylindrical tissue volume having a width 249 which is larger than a height 251 of the heated cylindrical tissue volume. A potential advantage of a hexagon-shaped ultrasound transducer may be to treat larger areas of deep tissue layers with lower penetration of ultrasound energy.

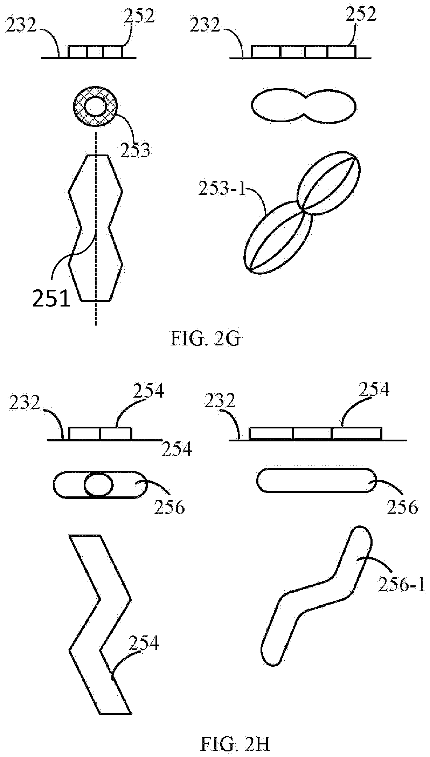

Fig. 2G illustrates an elevation and side view of a stretched hexagonal ultrasound transducer 252 placed in contact with skin surface 232, according to some exemplary embodiments of the invention. Reference numerals 251 and 253-1 indicate *mutatis mutandis* an elongated tissue volume 251 heated by a stretched hexagonal ultrasound transducer, and 253-1 is a three-dimensional illustration of an elongated tissue volume heated by stretched hexagonal ultrasound transducer 252.

According to some exemplary embodiments, for example as shown in fig. 2G, the ultrasound transducer, for example a PZT element of the ultrasound transducer, has an upper view of a polygon with multiple sides, for example a decagon. In some embodiments, the polygon-shaped ultrasound transducer heats a tissue volume having variable widths. Optionally, the tissue volume heated by the polygon-shaped ultrasound transducer has narrow or closed front and rear end openings.

Fig. 2H illustrates an elevation and side view of a wavy ultrasound transducer 254, according to some exemplary embodiments of the invention. Reference numerals 256 and 256-1 indicate *mutatis mutandis* an elongated tissue volume 256 heated by a wavy hexagonal ultrasound transducer, and 256-1 is a three-dimensional illustration of a wavy tissue volume heated by a wavy ultrasound transducer 254.

A potential advantage of having an ultrasound transducer with a skin facing surface that has an irregular shape or an irregular projection on the skin, for example a polygonal shape as shown in figs. 2F-2H, may be to generate a pattern on the skin that is less artificial, and therefore less readily noticeable compared to a rectangular shape, as in fig. 2E.

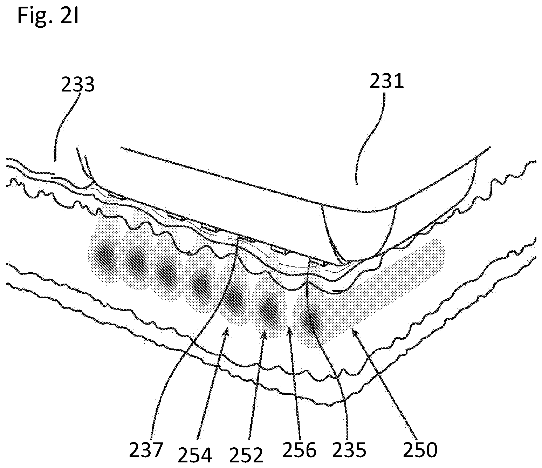

Fig. 2I, is an example of a thermal effect generated in deep tissue layers of the skin by an array of ultrasound transducers, according to some exemplary embodiments of the invention. In some embodiments, an ultrasound applicator, for example, applicator 204 shown in fig. 2A, having an array of ultrasound transducers, is placed in contact with skin 232. In some embodiments, The contact area of the ultrasound transducers 235 with the skin is up to 30mmX10mm, for example up to 20mmX10mm, up to 15mmX10mm, up to 10mmX10mm, or any intermediate, smaller or larger surface area which is sufficient to fit curved or non-planar areas of skin of a treated subject. In some embodiments, curved areas of skin comprise areas on the forehead and/or around the neck. In some examples, the ultrasound transducer, for example transducer 235, emits non-converging ultrasound energy into skin 232. In some embodiments, the ultrasound energy penetrates deep tissue layers of the skin and heats elongated tissue volumes, 234 in the deep tissue layers.

According to some exemplary embodiments, despite the different geometry of the ultrasound transducers illustrated in FIGS. 2F-2H, the cross-section of the heated tissue volumes is similar to the described above cross-sections of heated skin volumes 234. In some embodiments, depending on the tissue composition beneath the skin surface, the cross-section of the heated tissue volumes 246-1, 250-1, 253-1, and 256-1 is optionally uneven along their longitudinal axis.

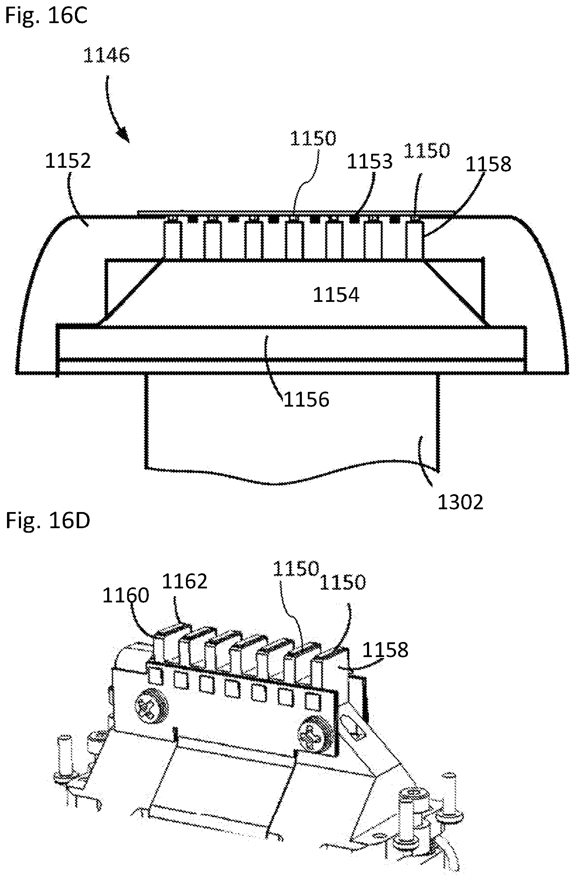

According to some exemplary embodiments, an array of ultrasound transducers, comprise at least two spaced-apart ultrasound transducers, for example transducers 1150 shown in figs. 16C and 16D. In some embodiments, the ultrasound transducers, are positioned at a distance of at least 0.5mm, at least 0.7mm, at least 1mm, at least 5mm, at least 10mm, at least 20mm, or any intermediate, smaller or larger value, between each other. In some embodiments, the maximal distance between two adjacent transducers of the same ultrasound applicator is in a range of 0.5mm-20mm, for example 0.5mm-5mm, 2mm-10mm, 5mm-15mm or any intermediate, smaller or larger range of values. In some embodiments, the distance between adjacent transducers is modified by the user of the ultrasound system, for example according to the type of the treated region, anatomy, and/or tissue composition. Optionally, the user selects an ultrasound applicator with a desired distance between ultrasound transducers according to at least one of a planned treatment protocol, type of the treated region, anatomy and tissue composition.

According to some exemplary embodiments, a user of the ultrasound system selects an ultrasound applicator, for example an ultrasound applicator that has a desired number of ultrasound transducers, having a desired shape, having a desired distance between them and that are arranged in an array with a desired shape, according to at least one of, the size, location and shape of the skin region that is going to be treated, and/or according to the size and shape of the heated region inside the skin.

According to some exemplary embodiments, a distance or spacing between two adjacent ultrasound transducers in the array is fixed. Alternatively the distance or spacing between some adjacent ultrasound transducers varies. In some embodiments, a console of the system is connectable to different ultrasound applicators with different distances between adjacent transducers. In some embodiments, the ultrasound energy or waves emitted from the spaced-apart ultrasound transducers heats spaced apart elongated tissue volumes in deep tissue layers of the skin, for example, tissue volumes 234, shown in fig. 2I. In some embodiments, the distance or spacing between adjacent ultrasound transducers determines a distance or spacing between adjacent heated elongated tissue volumes 234. In some embodiments, the tissue in the deep tissue layers located between adjacent heated elongated tissue volumes, for example, tissue 258, is not affected or is minimally affected by the energy emitted by ultrasound transducers 235.

According to some exemplary embodiments, the distance or spacing between the ultrasound transducers determines the fractionated heating of deep tissue layers and related to the deep heating skin effect, for example skin tightening. Additionally, the effect on the skin is determined based on the size and/or shape of the unaffected tissue volumes 258, located between heated tissue volumes 234.

According to some exemplary embodiments, at least some or each parameter of a fractionated heating pattern, for example heated tissue volumes separated by less affected tissue volume in deep tissue layers of the skin, affects the healing process of the heated tissue volumes. In some embodiments, the at least one parameter comprises the overall size of the treated area, for example, the fractionated area distance between heated tissue volumes, cell types between heated tissue volumes, size and/or shape of the heated tissue volumes, amount of damage to cells in the heated tissue volumes.

According to some exemplary embodiments, at least one parameter of the emitted ultrasound waves, for example, amplitude, frequency and/or duration determines at least one of size and/or shape of the heated tissue volumes, and distance or spacing between adj acent heated tissue volumes in the fractionated heating pattern.

According to some exemplary embodiments, a tissue volume, for example, an elongated tissue volume heated in the deep tissue layers has a volume in a range of 1 cm³ to 20 cm³, for example1 cm³ to 10 cm³, 5 cm³ to 15 cm³, 10 cm³ to 20 cm³, or any intermediate, smaller or larger range of elongated tissue volumes.

According to some exemplary embodiments, the ultrasound power applied and/or the volume of heated elongated skin volumes under skin 232 could be such that it will leave an impression or even a lesion 260 on the surface of skin 232.

According to some exemplary embodiments, the elongated tissue volumes are heated to a temperature in a range of 30-75°C, for example 30-50°C, 45-60°C, 55-75°C or any intermediate, smaller or larger range of temperatures.

According to some exemplary embodiments, an emitting surface of a planar transducers array has an area size in a range of 10mm² - 200 mm², for example. 10 mm²-50 mm², 40 mm²-100 mm², 10 mm²-100 mm², 100 mm²-200 mm² or any intermediate, smaller or larger range of surface areas.

According to some exemplary embodiments for example as shown in FIG. 2I, a minimal distance between two adjacent cores 242 of heated tissue volumes, for example, tissue volumes 234 is in a range of 0.5-10mm, for example, 0.5-5mm, 3-8mm, 4-10mm or any intermediate, smaller or larger range of distances.

### Exemplary system

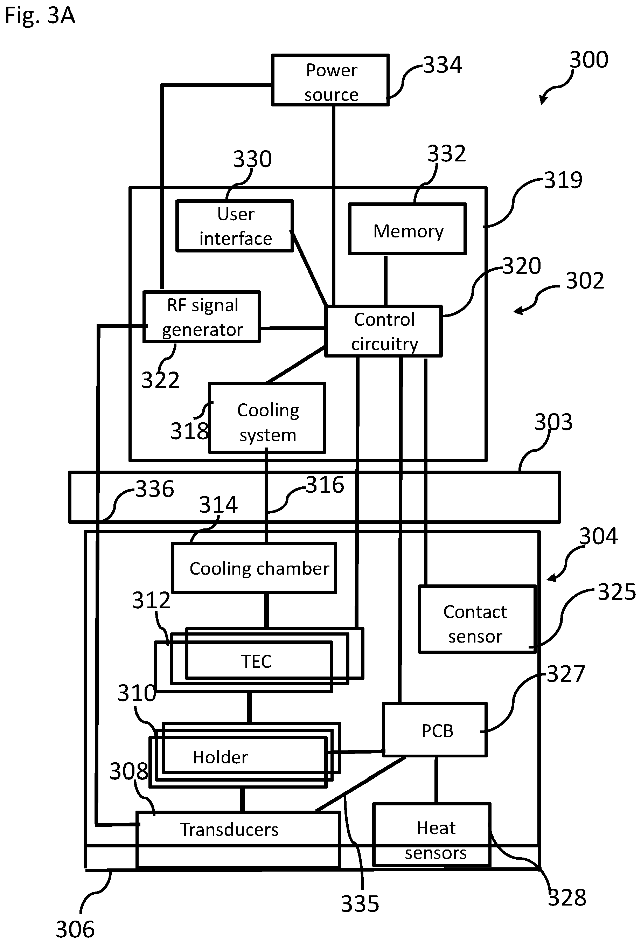

Reference is now made to fig. 3A depicting a system for delivery of skin treatments, for example cosmetic skin treatments, using ultrasound waves, according to some exemplary embodiments of the invention.

According to some exemplary embodiments, a system for delivery of skin treatments using ultrasound waves, for example system 300 comprises a control console, for example console 302, an ultrasound applicator, for example applicator 304, and an umbilical connecting channel, for example channel 303, connecting the applicator 304 to the control console. In some embodiments, the channel 303 is part of the applicator 304. In some embodiments, the applicator 304 is irreversibly assembled with channel 303, for example to prevent separation of the applicator 304 from the channel 303 by a user.

According to some exemplary embodiments, the ultrasound applicator 304 is reusable, for example can be optionally used for a time period of at least 1 week, at least 2 weeks, at least 1 month, or any intermediate, shorter or longer time period. In some embodiments, the ultrasound applicator 304 is optionally used to treat at least two subjects.

According to some exemplary embodiments, the applicator 304 comprises at least one ultrasound transducer. In some embodiments, the applicator 304 comprises two or more ultrasound transducers, for example 2,4,6,8,10,12,14,18,20 or any smaller or larger number of ultrasound transducers, for example transducers 308. In some embodiments, the transducers 308 are arranged in an array of ultrasound transducers. In some embodiments, a distance between adjacent transducers in the array is in a range of 0.1mm-10mm, for example 0.1mm-3mm, 2mm-4mm, 2mm-5mm, 4mm-7mm, 5mm-10mm or any intermediate, smaller or larger range of values. In some embodiments, a distance between adjacent transducers is fixed, for example to generate a uniform effect on the tissue by all transducers. Alternatively, a distance between at least some of adjacent transducers of the array varies, for example to generate a differential effect on a tissue region that has a non-uniform structure, shape and/or tissue composition.

According to some exemplary embodiments, each of the transducers and/or the array of transducers is shaped as a rectangle or a square. In some embodiments, an ultrasound energy emitting area of the applicator 304, positioned to face the skin, has an area size in a range of 1mm²-10mm² for example 2mm²-6 mm², 3mm²-5mm², or any intermediate, smaller or larger area size or range of sizes.

According to some exemplary embodiments, an ultrasound energy emitting surface of the transducers is positioned near a surface of the applicator which is shaped and sized to contact the skin. In some embodiments, the skin contacting surface of the applicator is curved. Alternatively or additionally, the skin contacting surface of the applicator is flat, for example with no bulges or protrusion emanating from the surface towards the skin.

According to some exemplary embodiments, the skin contacting surface is part of the applicator casing. In some embodiments, the applicator casing comprises a window in said skin contacting surface of the applicator. In some embodiments, the transducers 308 are arranged at least partly within the window, and oriented such that the ultrasound energy emitting surface of the transducers are within the window and are directed outward. In some embodiments, the window is covered by at least one isolating layer, for example isolating layer 308, configured to close the window and to isolate the ultrasound transducers from the skin.

According to some exemplary embodiments, the at least one isolating layer is thin enough to allow passage of the ultrasound waves from the ultrasound transducers 308 into the skin. In some embodiments, a thickness of at least one isolating layer is in a range of 10-50 µm, for example 12.5µm-25µm, 10µm-20µm, 15µm-35µm, 30µm-50µm or any intermediate, smaller or larger value or range of values. In some embodiments, at least one isolating layer 306 is configured to isolate the transducers 308 and/or inner component of the applicator from water and air. In some embodiments, at least one isolating layer comprises polyimide and/or glue and/or Parylene and/or Latex.

According to some exemplary embodiments, the transducers are coupled to at least one transducers holder, for example holder 310. In some embodiments, a back surface of the transducers, for example a surface that is opposite to the ultrasound emitting surface of the transducers is coupled to the holder 310. In some embodiments, the holder 310 comprises mounting surfaces, which are shaped and sized to contact the transducers. In some embodiments, a size, for example a width and/or a length, of each mounting surface is larger in at least 0.5%, for example at least 1%, at least 5%, at least 10% or any intermediate, smaller or larger percentage from a size, for example a width and/or a length, of transducers configured to be mounted to the mounting surface.

According to some exemplary embodiments, the holder 310 comprises a heat conducting material, for example aluminum or copper, or stainless steel or any other heat conducting material, for example to conduct heat away from the ultrasound transducers towards a cooler, for example a thermoelectric cooler (TEC) 312. In some embodiments, a surface of the holder 310 is placed in contact with a cold surface of the TEC 312, for example to allow cooling of the transducers 308 by the TEC 312 through the cold surface. In some embodiments, a hot surface of the TEC 312 is placed in contact with a liquid cooling system of the system, that circulates a coolant liquid between the console 302 and the applicator 304 through the umbilical connecting channel 303. Optionally, the hot surface contacts a cooling chamber 314 in the applicator304 containing the circulating coolant liquid.

According to some exemplary embodiments, an energizer comprises the transducers 308 assembled to the holder 310, and the TEC 312 contacting the holder. In some embodiments, the applicator 304 comprises a main printed circuit board (PCB) 327. In some embodiments, the main PCB 327 comprises a flex portion and a rigid board comprising at least one electric connector. In some embodiments, the flex portion is electrically connected to the transducers 308, for example to deliver to the transducers electric power from the rigid board. Optionally, the flex portion connected to the transducers 308 is not passivated. In some embodiments, the rigid board of the main flex-PCB is electrically connected to a power source in the console 302, for example via at least one electric connector.

According to some exemplary embodiments, the applicator 304 comprises at least one heat sensor, for example a thermistor configured to measure a temperature of the skin and/or a temperature of at least one transducer. In some embodiments, at least one heat sensor comprises at least one surface heat sensor, positioned near a surface of the ultrasound applicator that contacts the skin. In some embodiments, at least one surface heat sensor is configured to sense and transmit temperature levels of the skin contacting the applicator. In some embodiments, at least one surface heat sensor comprises a thermistor.

According to some exemplary embodiments, the applicator comprises two or more heat sensors 328, for example thermistors. In some embodiments, at least one thermistor is configured to record, for example sense and transmit, temperature levels of the skin, and at least one other thermistor is configured to record the temperature of at least one transducer and/or the temperature of the holder 310. In some embodiments, the heat sensors are part of a thermistors flex-PCB, configured to bend and contact the transducers 308 and/or the holder 310. Optionally, the heat sensors 328, for example the heat sensors of the thermistors flex-PCB are shaped and sized to be positioned between transducers 308 on the holder 310, for example to record the temperature of the skin when the applicator is placed in contact with the skin surface. In some embodiments, the thermistors flex-PCB is electrically connected to at least one electric connector of the main flex-PCB of the applicator.

According to some exemplary embodiments, the applicator 304 comprises at least one contact sensor, for example contact sensor 325, configure to record signals indicating contact of the applicator 304 with the skin. In some embodiments, the contact sensor 325 records changes in impedance and/or electric conductivity caused by the contact with skin.

According to some exemplary embodiments, the applicator 304 is mechanically connected to the console 302 by an umbilical connecting channel, for example connecting channel 303. In some embodiments, the channel 303 is connected to an umbilical connecting channel socket in the console 302, for example in the console casing 319. In some embodiments, the connecting channel socket comprises at least one electrical connector, configured to connect the electrical wiring of the connecting channel to the console 302. Additionally, the connecting channel socket comprises at least one cooling system connector, configured to connect tubing in the connecting channel 303 and the applicator 304 to tubing in the console 302 leading to the cooling system 318.

According to some exemplary embodiments, the channel 303 is a flexible channel, formed as a tube with an inner lumen. In some embodiments, the connecting channel comprises electrical wiring of the applicator 304 and tubes of the cooling system. In some embodiments, the cooling system tubes are isolated from the electrical wiring. In some embodiments, at least part of the connecting channel 303 is sealed from air and water, for example to prevent condensation of water on the cooling system tubes due to penetration of humid air into the connecting channel. In some embodiments, the electrical wiring is electrically isolated from the cooling system by a sealing fluid, for example a solidified sealing fluid. In some embodiments, the solidified sealing fluid is configured to cover the electrical wiring within the applicator body, when the sealing fluid is in a liquid state, and to be solidified within the applicator body.

According to some exemplary embodiments, the console 302 comprises a cooling system 318, for example a chiller, positioned at a bottom of a casing of the console 302, for example to lower a center of gravity of the console. In some embodiments, the chiller comprises a coolant liquid reservoir and an electric pump configured to circulate the liquid coolant between the reservoir and the applicator.

According to some exemplary embodiments, the console comprises at least one radiofrequency (RF) signal generator 322 configured to generate and deliver an electric power signal to the transducers 308 via electrical wiring between the console 302 and the main flex-PCB 327 of the applicator 304. In some embodiments, the console 302 comprises a control circuitry 320 configured to control the generation of the RF signal by the RF signal generator and the delivery of the signal to the transducers 308.

According to some exemplary embodiments, the control circuitry 320 is electrically connected to at least one heat sensor, for example sensors 328, of the applicator. In some embodiments, the control circuitry 320 controls the activation of the cooling system 318 and/or the activation of the RF signal generator 322 based on signals received from the heat sensors 328 and/or the at least one contact sensor 325 of the applicator 304.

According to some exemplary embodiments, the console 302 comprises a user interface, for example user interface 330, configured to receive input from an operator of the system, and/or to deliver at least one human detectable indication to the operator of the system. In some embodiments, the user interface 330 comprises a display for presenting visual indications to the operator. In some embodiments, the display is a touch display, configured to receive input from the operator. Alternatively or additionally, the user interface comprises a keyboard and/or at least one button for receiving input from the operator.

According to some exemplary embodiments, the console 302, for example the RF signal generator 322 and/or the control circuitry 320 of the console 302 is connected to an external power source 334.

According to some exemplary embodiments, the console 302 comprises a memory 332 electrically connected to the control circuitry 320. In some embodiments, the memory 332 stores at least one treatment protocol or parameters thereof, for example amplitude and/or frequency of ultrasound waves, desired amount of total ultrasonic energy per a selected treatment area, duration of a single pulse of ultrasound waves, number of pulses in a train of pulses, number of trains per a treatment session. In some embodiments, the memory stores treatment history of a specific subject, for example in a database including information of specific subjects. In some embodiments, the memory 332 stores safety parameter values, for example maximal allowed temperature levels of the skin, maximal allowed temperature levels of the holder, maximal allowed intensity, frequency and duration of energy delivery to the tissue.

According to some exemplary embodiments, if the temperature of the skin and/or the transducers 308 is higher than a pre-determined value, then the control circuitry 320 signals the cooling system to increase the cooling of the transducers and/or skin. Alternatively or additionally, the control circuitry 320 signals the RF signal generator to stop generating an RF power signal. Alternatively or additionally, the control circuitry 320 generates a human detectable indication, for example an alert signal via the user interface 330, for example to provide an alert to an operator regarding the temperature increase.

According to some exemplary embodiments, the casing 319 of the console 302, is shaped as a tower, positioned on a surface, for example a floor. In some embodiments, a projection of the casing 319 on the floor has a small footprint, for example a foot print smaller than 2m², for example smaller than 0.8 m², smaller than 0.6 m² or any intermediate, smaller or larger value.

According to some exemplary embodiments, the console 302 comprises two or more wheels, coupled to a surface of the console facing the floor. In some embodiments, the two or more wheels are configured to roll on the floor and move the console to a desired location. In some embodiments, the console 302 comprises at least one brake, for example a hand-operated brake and/or a leg-operated brake, configured to stop the rolling of the wheels on the floor. In some embodiments, the console 302 comprises at least one handle having one or more gripping members, shaped and sized to be held by a hand of a human subject and configured to allow movement of the console 302 to a desired location on the floor.

According to some exemplary embodiments, the console 302 comprises at least one applicator holder coupled to the console casing 319, and shaped and sized to hold the applicator 304 when the applicator is not in use. In some embodiments, the applicator holder comprises a ring or a funnel shaped and sized to surround the body of the applicator 304.

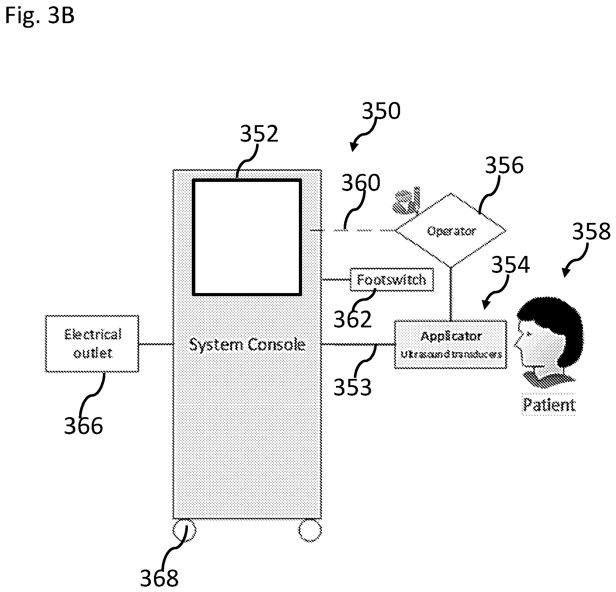

Reference is now made to fig. 3B, depicting an additional example of a system for delivery of ultrasound waves for skin treatments, according to some exemplary embodiments of the invention.

According to some exemplary embodiments, a system for delivery of ultrasound waves for skin treatments, for example system 350 comprises a system console 352 and an applicator 354 comprising ultrasound transducers. In some embodiments, the applicator 354 is connected to the console 352 by electrical wiring. Additionally, the applicator 354 is connected to the console 352 by at least one tube of a cooling system shaped and sized to circulate coolant liquid between the system console 352 and the applicator 354.

According to some exemplary embodiments, the applicator comprises a handle with at least one gripping member shaped and sized to be held by a single hand of an operator of the system 350, for example operator 356. In some embodiments, the operator 356 places in contact the applicator 354 with at least one skin area of a subject, for example patient 358. In some embodiments, the at least one skin area comprises one or more of a facial, a neck and submental skin area.

According to some exemplary embodiments, the system console 350 comprises at least one user interface, configured to receive output and/or deliver input to the system console 352. In some embodiments, the user interface comprises a display 364 configured to deliver a visual indication to the operator 356. Optionally, the display 364 comprises a touch interface, configured to receive input from the operator 356. In some embodiments, the user interface comprises a footswitch 362 configured to receive input from the operator 356, for example when the footswitch 362 is pressed by a leg of the operator. Alternatively or additionally, the applicator comprises a user interface, for example a button for transmitting activation and/or deactivation input signals to the system 350.

According to some exemplary embodiments, the operator 356 activates the system 350 to deliver ultrasound waves to the skin of the patient 358 using a user interface of the console 352 or a user interface of the applicator 354. In some embodiments, the operator 356 activates the system to deliver the ultrasound waves after holding and placing the applicator against the skin of the patient 358, by pressing one or more of the footswitch 362, at least one activation button of the applicator 354, and/or using a user interface of the console 352.

According to some exemplary embodiments, the console 352 is shaped as a tower having a smaller footprint relative to a height of the console. In some embodiments, the console is movable on a surface, for example a floor, by at least two wheels 368 coupled to a surface of the console 352 facing the floor. In some embodiments, the console 352 is electrically connected to an external electrical outlet, for example outlet 366.

According to some exemplary embodiments, the ultrasound applicator 304 comprises at least one ID tag, for example RFID tag, which includes ID information. In some embodiments, the console 302 comprises at least one reader, for example a RFID reader for reading the ID information. Alternatively, the ID information is stored in an electronic circuit of the ultrasound applicator 304. In some embodiments, the ID information includes information associating the ultrasound applicator with a specific manufacturer, number of ultrasound pulses generated by the ultrasound applicator, manufacturing date, manufacturing information, type of ultrasound applicator, and/or setup or calibration information of the ultrasound applicator.

### Exemplary skin treatment

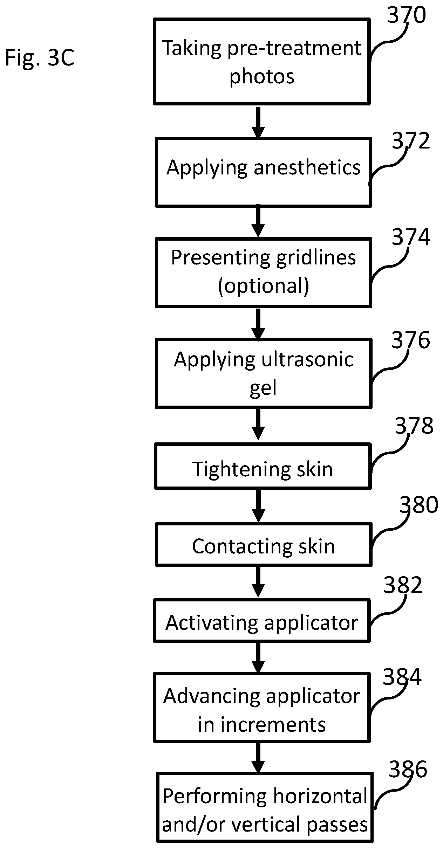

Reference is now made to fig. 3C depicting a skin treatment process, according to some exemplary embodiments of the invention.

According to some exemplary embodiments, pre-treatment photos are taken at block 370. In some embodiments, photos of at least one determined treatment region, for example photos of specific regions of the face or neck are taken at block 370. In some embodiments, the photos are taken at block 370 to plan a treatment procedure, for example to determine at least one of the number of treatment sessions needed to cover the at least one treatment region, duration of each treatment session and the number of passes of the ultrasound transducer need to cover the at least one determined treatment region.

According to some exemplary embodiments, anesthetics is applied at block 372. In some embodiments, the anesthetics is applied, by applying an anesthetic cream or lotion on the skin surface of the at least one determined treatment region.

According to some exemplary embodiments, markings, for example gridlines are optionally presented on the skin at block 374. In some embodiments, the gridlines are presented by drawing the gridlines on the skin, for example to mark boundaries of the at least one determined treatment region. Alternatively, the gridlines are projected on the skin.

According to some exemplary embodiments, ultrasonic gel is applied at block 376.

According to some exemplary embodiments, the skin is tightened at block 378. In some embodiments, the skin is tightened, for example to flatten wrinkles or dents in the skin.

According to some exemplary embodiments, the skin is contacted with an ultrasound emitting surface of the ultrasound applicator, at block 380. In some embodiments, a user of the applicator ensures contact of the applicator with the skin by pressing the emitting surface of the ultrasound applicator against the skin. Optionally, the user receives an indication regarding the contact of ultrasound transducers of the ultrasound applicator with the skin. Optionally, the indication is received prior to activating at least one ultrasound transducer of the ultrasound applicator.

According to some exemplary embodiments, the ultrasound applicator, for example at least one ultrasound transducer of the applicator is activated at block 382. In some embodiments, the ultrasound transducer is activated while contacting the skin, and optionally during the tightening of the skin. In some embodiments, during the activation of the at least one ultrasound transducer, the at least one ultrasound transducer generates ultrasound energy by converting electricity into vibrations. In some embodiments, during activation, ultrasonic energy is delivered to the skin at the determined at least one treatment region. In some embodiments, during activation pulses of ultrasound energy are delivered to the skin.

According to some exemplary embodiments, the ultrasound applicator comprises 2 or more ultrasound transducers, for example 5, 7, or 4 to 8 ultrasound transducers. In some embodiments, the ultrasound applicator is activated even if at least one ultrasound transducer is not operating properly, for example if the conversion efficiency of electricity into vibrations and ultrasound energy by the at least one ultrasound transducer is lower than a predetermined reference or baseline value. In some embodiments, the ultrasound applicator is deactivated if at least two adjacent ultrasound transducers are not operating properly.

According to some exemplary embodiments, the ultrasound applicator is advanced on the skin in increments, at block 384. In some embodiments, the ultrasound applicator is advanced on the skin while activating the at least one ultrasound transducer. Alternatively, the at least one ultrasound transducer is activated when the ultrasound applicator is stationary. In some embodiments, the ultrasound applicator is advanced in increments of at least 10% overlap between ultrasound applicator emitting surface locations, for example at least 30% overlap, at least 40% overlap, at least 50% overlap or any intermediate, smaller or larger percentage of overlap. In some embodiments, the ultrasound applicator is advanced in increments of about 50% overlap, for example to proper treatment even if at least one ultrasound transducer is not operating properly.

According to some exemplary embodiments, the ultrasound applicator is advanced to form horizontal and/or vertical passes, at block 386. In some embodiments, the ultrasound applicator is advanced on the skin to form horizontal and then vertical passes. In some embodiments, the ultrasound applicator is advanced on the skin to form at least 2 horizontal passes and at least 2 vertical passes.

In some embodiments, when treating forehead and/or Peri Orbital skin regions, the at least one ultrasound transducer is activated to deliver 20 to 50 pulses of ultrasound energy, for example 20-40 pulses, 25-40 pulses, 30-45 pulses or any intermediate smaller or larger range of pulses. In some embodiments, when treating left and/or right cheeks, Chin and/or Upper Lip skin regions, the at least one ultrasound transducer is activated to deliver 70-120 pulses of ultrasound energy, for example 70-90 pulses, 80-100 pulses, 90-110 pulses or any intermediate, smaller or larger range of pulses. In some embodiments, when treating Neck and Submental skin region, the at least one ultrasound transducer is activated to deliver 50-90 pulses of ultrasound energy, for example 50-70 pulses, 60-80 pulses, 70-90 pulses or any intermediate, smaller or larger range of pulses.

### Exemplary use of the ultrasound system

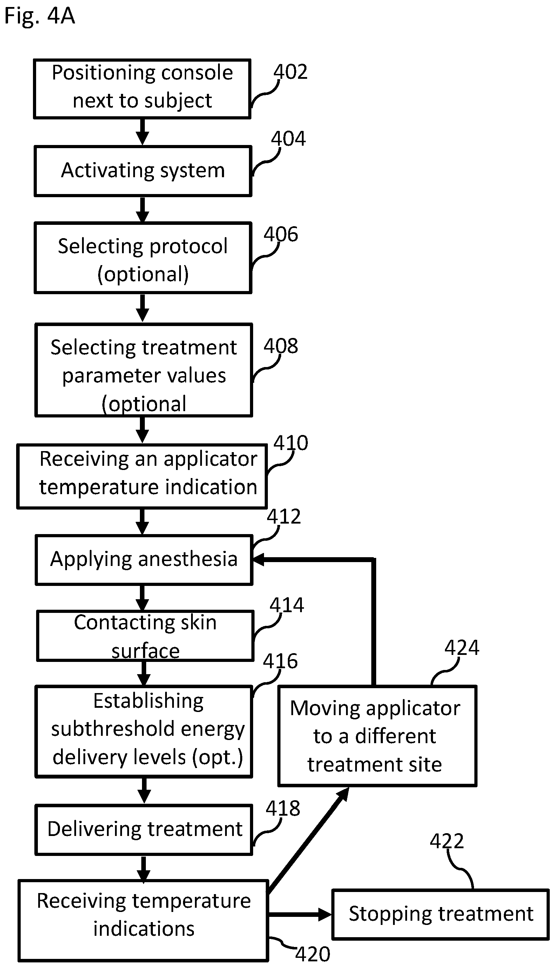

Reference is now made to fig. 4A, depicting a process of using the system by an operator, according to some exemplary embodiments of the invention.

According to some exemplary embodiments, a control console of the system, for example console 302 show in in fig. 3A or console 352 show in in fig. 3B, is positioned in a desired location near a subject, for example a patient. In some embodiments, the location is selected according to the distance between an applicator connected to the console via the umbilical connecting channel, and a treatment target in the subject.

According to some exemplary embodiments, the system is activated at block 404. In some embodiments, the system is activated using the user interface of the console. In some embodiments, activation of the system initiates activation of the cooling system.

According to some exemplary embodiments, a user of the system, for example an operator selects a protocol, for example a cosmetic treatment protocol, at block 406. In some embodiments, the protocol is selected based on at least one parameter of the patient, for example skin type and/or skin condition at the treatment area, location of the treatment area, wrinkles appearance in the treatment area, history of treatment of the patient and/or of other patients having similar characteristics, sensitivity of the patient to pain and/or to drugs used to reduce pain level.

According to some exemplary embodiments, at least one treatment parameter value is selected, for example by an operator, at block 408. In some embodiments, the at least one parameter comprises amplitude and/or frequency of the ultrasound waves. Additionally or alternatively, the at least one parameter comprises time duration for delivery of the ultrasound waves, total energy to be delivered to a selected treatment region in a single treatment session.

According to some exemplary embodiments, an indication related to a temperature of the applicator, for example an applicator component, is received at block 410. In some embodiments, an operator of the system receives the indication from a user interface of the system, for example a user interface of the console. In some embodiments, an indication related to one or more of a temperature of the transducers, a temperature of a holder, a temperature of the coolant liquid, a temperature of a cold surface of a TEC, is received at block 410.

According to some exemplary embodiments, anesthesia, for example local anesthesia is applied at a selected treatment area, at block 412. In some embodiments, local anesthesia is topically applied to the skin at a selected treatment area. In some embodiments, the anesthesia comprises one of or a combination of Lidocaine and prilocaine and/or NO gas for relaxation.

According to some exemplary embodiments, an operator places the applicator in contact with the skin at the treatment area, at block 414. In some embodiments, the operator places the applicator in contact with the skin if a temperature of the transducers and/or a temperature of the coolant liquid is lower than a predetermined value or is within a range of predetermined values of (-15°C) - 20°C, for example (-10°C) - 5°C or (-10°C) - (-6°C) or any intermediate value or range of values. In some embodiments, the operator places an ultrasound emitting surface of the applicator in contact with the skin.

According to some exemplary embodiments, a subthreshold energy delivery level is established at block 416. In some embodiments, a threshold of energy delivery is the level of energy that cause intolerable side effects, for example pain, to a subject. In some embodiments, once an energy threshold is determined, parameter values of ultrasound waves are set to be deliver ultrasonic energy which is lower than the determined threshold, for example to prevent injury to the specific treated subject.

According to some exemplary embodiments, ultrasound waves are delivered to the skin at a target area, at block 418. In some embodiments, an operator initiates the delivery of the treatment using the user interface of the console or the user interface of an applicator, for example an activating button on the applicator. In some embodiments, the parameter values of the delivered treatment are calculated based on one or more of a selected protocol, selected treatment parameter values and determined subthreshold energy delivery levels.

According to some exemplary embodiments, during the delivery of the treatment, temperature related indications are received at block 420. In some embodiments, an operator of the system receives the indications by the user interface of the console, for example a display of the console, and/or by a user interface of the applicator, for example a display or at least one light indicator on the applicator. In some embodiments, the temperature related indications comprise one or more indications related to a temperature of the skin at the treatment area, indications related to a temperature of the transducers and/or a holder of the transducers, indications related to a temperature of a cold and/or hot surfaces of a TEC, and indications related to a temperature of a coolant liquid of the cooling system.

According to some exemplary embodiments, if the temperature indications indicate that a recorded temperature level is within a desired range of values, then the operator continuous to deliver the treatment. In some embodiments, the operator moves the applicator to a different treatment region, at block 424.

According to some exemplary embodiments, if the temperature indications indicate that a recorded temperature level is not within a desired or a predetermined range of temperature levels, then the treatment is stopped at block 422. In some embodiments, the treatment is manually stopped by the operator. Alternatively, the treatment is automatically stopped by the control console.

### Exemplary system actions

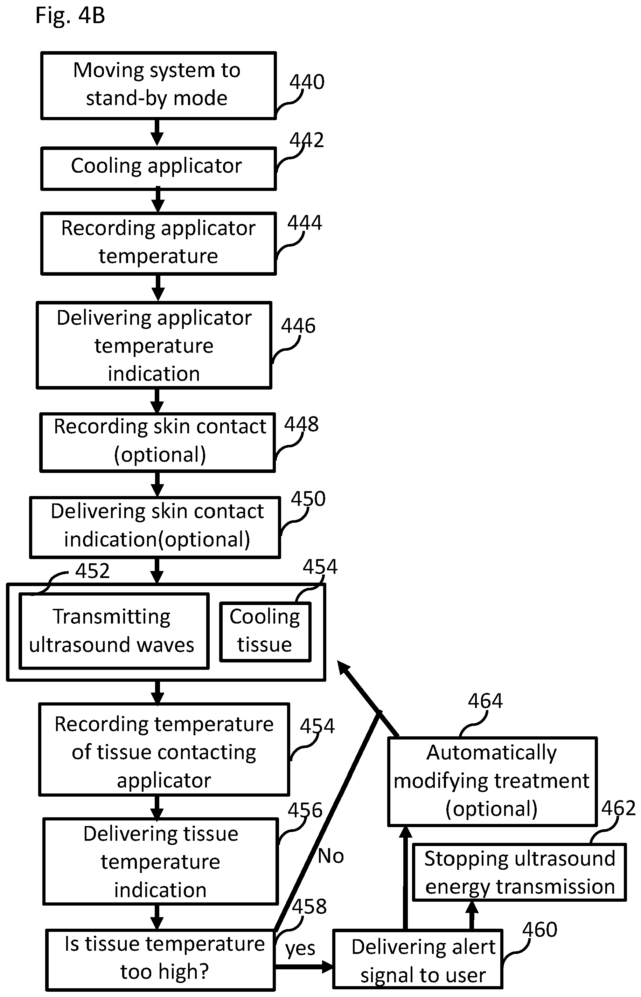

Reference is now made to fig. 4B, depicting actions of a system for delivery of ultrasound waves for skin treatments, according to some exemplary embodiments of the invention.

According to some exemplary embodiments, upon activation by an operator, the system moves to a stand-by state, at block 440. In some embodiments, in a stand-by state, ultrasound waves are not emitted.

According to some exemplary embodiments, the applicator is cooled to a predetermined temperature, at block 442. In some embodiments, the applicator is cooled when the system is in a stand-by state. In some embodiments, at least part of a surface of the applicator shaped configured to be placed in contact with the skin is cooled down. In some embodiments, the transducers and/or a holder of the transducers is cooled down.

According to some exemplary embodiments, the applicator temperature is recorded at block 444. In some embodiments, the temperature of one or more of the transducers, the skin contacting surface of the applicator, the transducers holder, and the coolant liquid, is recorded at block 444. In some embodiments, the temperature is recorded, for example sensed and transmitted, by temperature sensors, for example thermistors contacting the transducers and/or the holder of the transducers comprising a heat conducting material. In some embodiments, the thermistors are positioned between the transducers, and underneath a skin contacting surface of the applicator. In some embodiments, the thermistors are positioned underneath at least one isolating layer isolating the transducers and/or thermistors from liquid and/or air. In some embodiments, the at least one isolating layer prevents a direct contact between internal components of the applicator, for example thermistors and/or transducers with the skin.

According to some exemplary embodiments, an indication regarding the recorded temperature is delivered at block 446. In some embodiments, the indication, for example a human detectable indication, is delivered by a user interface of the console, for example a display of the console, and/or a user interface of the applicator, for example at least one LED.

According to some exemplary embodiments, contact of the applicator, and/or transducers with the skin is recorded at block 448. In some embodiments, the contact is recorded by at least one sensor, for example pressure sensor or a sensor measuring at least one electric property of the transducers. In some embodiments, the at least one electric sensor records electric impedance and/or conductivity of the transducers or changes in the impedance and/or conductivity. In some embodiments, the contact of the applicator and/or transducers with the skin is detected from changes in electric power delivery to the transducers.

According to some exemplary embodiments, an indication regarding to the contact between the applicator and/or transducers with the skin is generated and delivered at block 450. In some embodiments, the indication is a human detectable indication, for example a visual and/or an audio indication. In some embodiments, the indication is delivered to an operator of the system using the user interface of the console, for example a display of the console, and/or the user interface of the applicator, for example a LED indicator of the applicator.

According to some exemplary embodiments, ultrasound waves are transmitted at block 452. In some embodiments, the ultrasound waves are transmitted by ultrasound transducers, for example an array of ultrasound transducers, to the skin through the at least one isolating layer separating the transducers from the skin. In some embodiments, the ultrasound waves are transmitted in a continuous pulse or in a series of pulses, for example a train of pulses.

According to some exemplary embodiments, the ultrasound waves are generated and transmitted with parameter values adjusted according to one or more of a recorded temperature of the skin, transducers temperature, skin type, treatment area location, and/or skin condition. In some embodiments, values of the at least one parameter of the transmitted ultrasound waves changes during the delivery of the waves according to one or more of a recorded temperature of the skin, transducers temperature, skin type, treatment area location, and/or skin condition.

According to some exemplary embodiments, during the delivery of the ultrasound waves, the skin surface is being cooled by the applicator at block 454. In some embodiments, the skin surface contacting the applicator is cooled down by a cooling system of the applicator configured to cool the transducers and the skin surface contacting the transducers.

According to some exemplary embodiments, a temperature of a skin surface contacting the applicator is recorded during the delivery of ultrasound waves, at block 454. In some embodiments, the skin surface temperature is recorded by at least one thermistor placed in indirect contact with the skin surface, and records the temperature through at least one isolating layer.

According to some exemplary embodiments, an indication related to the temperature of the skin tissue during the delivery of the ultrasound waves is delivered to an operator at block 456. In some embodiments, the indication, for example a human detectable indication, is delivered during the delivery of the ultrasound waves. In some embodiments, the indication comprises a visual indication, for example changes in temperature values shown to an operator, for example on a display of the console. In some embodiments, the indication comprises a graphical indication, for example a graph, showing changes in temperature. Optionally, the graphical indication shows temperature levels or changes in temperature relative to a maximal temperature value.

According to some exemplary embodiments, the system determined if the recorded temperature, for example the recorded temperature of the skin and/or the transducers, is higher than a predetermined value, at block 458. In some embodiments, the predetermined value is personalized for a specific person, for example based on a predetermined threshold. Alternatively, the predetermined value is a value set by regulation authorities due to safety concerns.

According to some exemplary embodiments, if the recorded temperature is higher than the predetermined value, then an alert signal is generated and delivered to an operator, at block 460. In some embodiments, the alert signals comprises a visual and/or an audio signal.

According to some exemplary embodiments, if the recorded temperature is higher than the predetermined value, then the delivery of ultrasonic waves is stopped at block 462. In some embodiments, the delivery of the ultrasound energy is automatically stopped by the system. Alternatively, the delivery of the ultrasound energy is stopped manually by an operator, using the user interface of the console and/or a user interface of the applicator. In some embodiments, the ultrasound energy is manually stopped by activating an emergency cut off switch.

According to some exemplary embodiments, alternatively, if the recorded temperature is higher than the predetermined value, then values of at least one parameter of the treatment are modified at block 464, for example automatically by a control circuitry of the system. In some embodiments, values of at least one parameter of the transmitted ultrasound waves are modified at block 464, for example automatically by a control circuitry of the system. In some embodiments, the values of at least one parameter of the treatment and/or values of at least one parameter of the transmitted ultrasound waves are modified by a control circuitry, for example control circuitry 320 according to at least one indication stored in a memory 332 shown in fig. 3A.

According to some exemplary embodiments, changes in the treatment and/or parameter values of the ultrasound waves are performed using at least one algorithm and/or at least one look up table stored in the memory 332. In some embodiments, a correlation between input regarding temperature of the skin and/or transducers and entries in the look up table are calculated. In some embodiments, the treatment parameter and/or the at least one parameter of ultrasound waves is changed according to entries in the look up table.

According to some exemplary embodiments, if the recorded temperature is lower than a predetermined value or is within a range of allowed values, then the transmitting of the ultrasound waves continues.

### Exemplary delivery of ultrasound waves

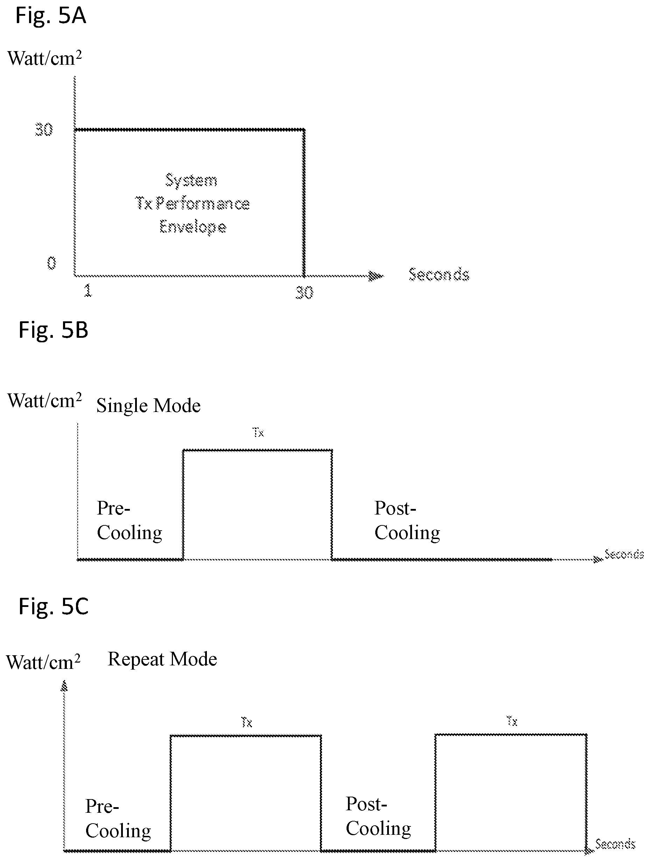

According to some exemplary embodiments, the ultrasound waves are generated and transmitted with amplitude and frequency values selected to allow heating of tissue volumes in deep layers of the skin. Reference is now made to figs. 5A-5C depicting different modes for delivery of the ultrasound waves, according to some exemplary embodiments of the invention.

According to some exemplary embodiments, for example as shown in fig. 5A, the system generates ultrasound waves with an amplitude, for example an intensity, of up to 30 Watt/cm², for example up to 15 Watt/cm², up to 20 Watt/cm², up to 25 Watt/cm² or any intermediate, smaller or larger range of values. In some embodiments, an intensity of the ultrasound waves is selected according to one or more of skin type, skin condition, sensitivity of a subject to pain, and wrinkles density in the skin.

According to some exemplary embodiments, the system generates ultrasound waves with a frequency in a range of 0.1 MHz - 30 MHz, for example 1 MHz - 20 MHz, 5 MHz - 15 MHz, 9 MHz - 13 MHz, 9 MHz - 22 MHz, 11 MHz - 13 MHz, 18 MHz - 22 MHz or any intermediate, smaller or larger range of values.

According to some exemplary embodiments, for example as shown in fig. 5B, the ultrasound waves are delivered to the skin tissue by a single pulse having an intensity of 5 W/cm² - 60 W/cm², for example 10 W/cm² - 40 W/cm², 15 W/cm² - 30 W/cm², or any intermediate, smaller or larger range of values. Additionally, in some embodiments, the ultrasound waves are delivered with a frequency in a range of 10 MHz - 13MHz, for example 10 MHz-12 MHz or any intermediate, smaller or larger range of values. In some embodiments, the surface of the skin, for example an area of the skin contacting the applicator and/or the transducers, is cooled before and after the delivery of the pulse. In some embodiments, the single pulse is delivered to the skin tissue in a time duration in a range of 0.5 sec - 15sec, for example 1 sec-10 sec, 2 sec-8 sec, 3 sec-6 sec, or any intermediate, smaller or larger range of values. In some embodiments, the pulse is delivered during a time period of 2 seconds, 3 seconds, 4 seconds, 5 seconds. In some embodiments, the skin surface is cooled prior to the delivery of the pulse for at least 2 seconds, for example at least 4 seconds, at least 8 seconds, or any intermediate, shorter or longer time duration. In some embodiments, the skin surface is cooled after the delivery of the pulse for at least 2 seconds, for example at least 4 seconds, at least 8 seconds, at least 10 seconds or any intermediate, shorter or longer time period.

According to some exemplary embodiments, for example as shown in fig. 5C, the ultrasound waves are delivered by a train of at least 2 pulses, for example at least 4 pulses, at least 7 pulses, at least 10 pulses, at least 20 pulses or any intermediate, smaller or larger number of pulses. In some embodiments, each pulse in a train of pulses has the same intensity, frequency and delivery time. Alternatively, at least some of the pulses in a train of pulses are delivered with intensity, frequency and delivery time which is different from other pulses in the train of pulses. In some embodiments, a time duration between two adjacent pulses in a train of pulses is in a range of 0.5 sec - 12 sec, for example 1 sec - 5 sec, 3 sec-8 sec, 5 sec-10 sec, or any intermediate, smaller or larger range of values. In some embodiments, the skin is cooled before and after each pulse.

### Exemplary monitoring during treatment

According to some exemplary embodiments, coupling of the transducers to the skin is monitored, for example to provide inherent safety and performance control on every pulse applied to the skin. In some embodiments data is sampled in a rate of at least 70 sampling points per second (p/sec), for example at least 80 p/sec, at least 100 p/sec, at least 120 p/sec or any intermediate, smaller or larger number of points per second, for example to provide accurate feedback to an operator of the system, for example a physician or any person certified to use the system. In some embodiments, the sampled data is stored in a memory of the control console, and/or in a cloud storage or a remote server. In some embodiments, the sampled data is used to generate a database, to improve treatment protocols, and or for the use of machine learning and artificial intelligence algorithms.

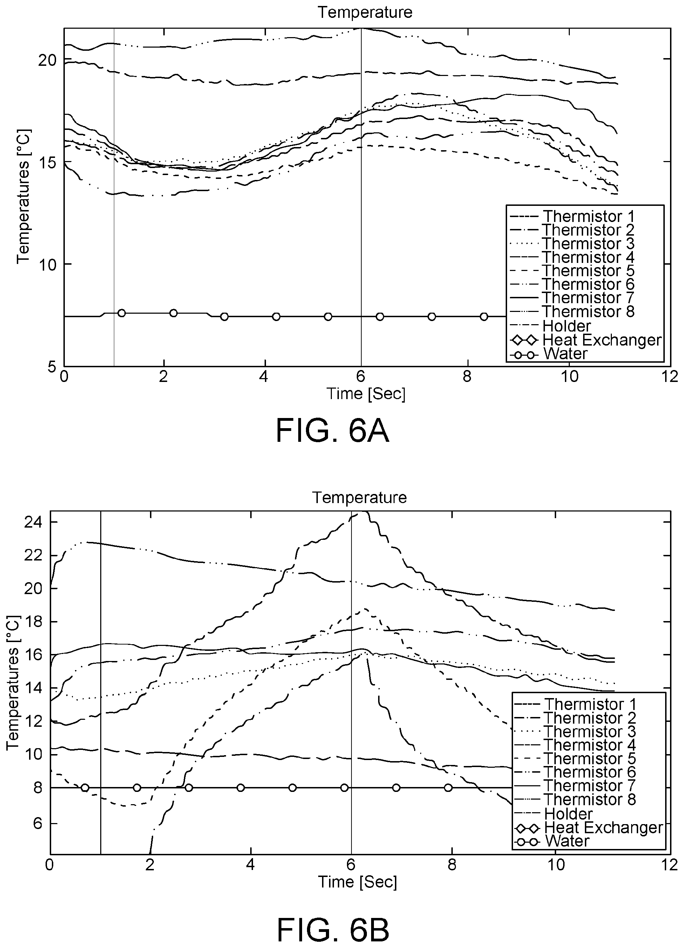

Reference is now made to figs. 6A and 6B depicting monitoring a temperature of the skin surface as an indicator for a proper contact of the transducers with the skin, according to some exemplary embodiments of the invention.

According to some exemplary embodiments, the system monitors the temperature of the skin surface, for example online with the delivery of ultrasound waves and/or prior to the delivery of ultrasound waves. In some embodiments, at least one algorithm stored in a memory of the control console measures the temperature level based on signals received from a plurality of thermistors located near the skin surface, for example up to 1mm, for example up to 0.1mm, up to 0.01mm or any intermediate, smaller or larger distance from the skin surface. In some embodiments, if a measured temperature exceeds a preset value, the system automatically stops the delivery of the ultrasound waves.

A potential advantage of measuring the skin surface temperature during the delivery of ultrasound waves may be to assure adequate contact with the skin, to ensure energy delivery to tissue.

According to some exemplary embodiments, for example as shown in fig. 6A changes in temperature levels over time in up to 7°C, for example up to 6°C, up to 5°C, or any intermediate, smaller or larger temperature, indicate a proper contact of the transducers with the skin.

According to some exemplary embodiments, for example as shown in fig. 6B, changes in temperature levels over time in a rate of more than 1°C/sec, for example 2°C/sec, 5°C/sec or any intermediate, smaller or larger value, indicate partial or no contact between at least some of the transducers and the skin.

According to some exemplary embodiments, electric impedance is monitored, for example to verify proper transducers performance, to assure efficient energy transmission to transducers, and to identify real-time performance reduction, which is optionally manifested in the efficiency of electric energy conversion to mechanical energy of PZT vibration which corresponds with the ultrasonic energy emission of the transducer. In some embodiments, if there is a change of the adhesion between the FLEX PCB and the PZT, there is a change in the electric impedance measured on the transducer.

According to some exemplary embodiments, forward/reflected electric power is monitored. In some embodiments, the RF power applied to the ultrasound transducers, and/or the emitted ultrasound energy is monitored.

Monitors RF power applied to the US transducers, and the ultrasound energy emitted.

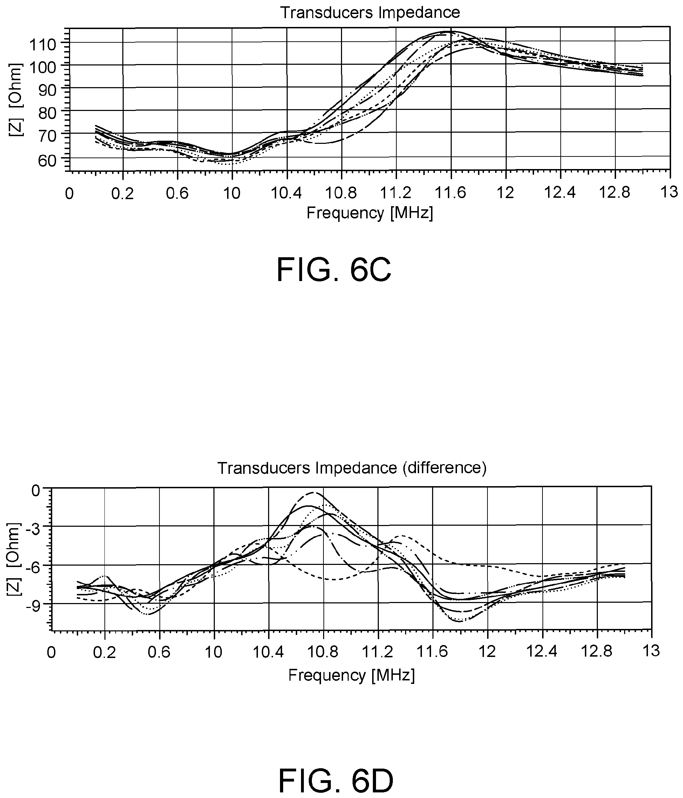

According to some exemplary embodiments, for example as shown in fig. 6C, the electric impedance of the transducers changes in a similar way, as frequency increases, indicating proper ultrasound transducers performance.

According to some exemplary embodiments, for example as shown in fig. 6D, difference between changes in impedance as frequency increases between some ultrasound transducers indicate inadequate operation of the transducers. In some embodiments, if changes in the activity of some transducers is detected, the delivery of the ultrasound waves is stopped. Additionally, an alert signal is delivered to an operator of the system.

### Exemplary process for identifying degradation in ultrasound energy delivery

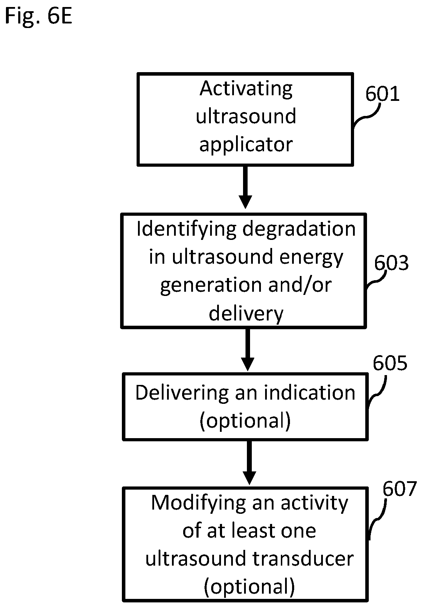

According to some exemplary embodiments, during the activation of at least one ultrasound transducer of an ultrasound applicator, ultrasound energy is generated. In some embodiments, during a skin treatment, for example a cosmetic treatment of the skin, the generated ultrasound energy is delivered into tissue layers of the skin. In some embodiments, the generation and delivery of the ultrasound energy from the at least one ultrasound transducer, for example the generation efficiency and/or the delivery efficiency is monitored. In some embodiments, the ultrasound energy generation and/or delivery is monitored to detect malfunction of one or more of the ultrasound applicator components, for example malfunction of at least one ultrasound transducer. Alternatively or additionally, the ultrasound energy generation and/or delivery is monitored to detect when the ultrasound applicator or system is not used properly, for example according to manufacturer user instructions. In some embodiments, monitoring of ultrasound energy generation comprises monitoring the conversion of electricity into ultrasound energy by the at least one ultrasound transducer. Reference is now made to fig. 6E depicting a process for identifying degradation in ultrasound energy delivery, according to some exemplary embodiments of the invention.

According to some exemplary embodiments, an ultrasound applicator is activated at block 601. In some embodiments, at least one ultrasound transducer of the ultrasound applicator, for example 2, 3, 4, 5, 6, 7, 8, 9 or any larger number of ultrasound applicators are activated. In some embodiments, the at least one ultrasound transducer is activated by a control console, for example at least one electrical circuitry configured to electrify the at least one ultrasound transducer.

According to some exemplary embodiments, the ultrasound applicator is activated for time periods of at least 30 days, for example for time periods of at least 40 days, 50 days, 60 days or any intermediate, shorter or longer time periods. In some embodiments, during the activation time period, the applicator generates at least 10,000 pulses of ultrasound energy, where each pulse of ultrasound energy is a time duration of 30 seconds in which ultrasound energy is generated. **In** some embodiments, during the activation time period, the ultrasound applicator is used to treat two or more subjects.

According to some exemplary embodiments, degradation, for example reduction in ultrasound energy delivery is identified at block 603. **In** some embodiments, degradation in ultrasound energy generation and/or delivery potential efficiency to skin tissues is identified at block 603. **In** some embodiments, the degradation in ultrasound energy generation and/or delivery potential efficiency is identified during the activation of at least one ultrasound transducer of the ultrasound applicator. **In** some embodiments, the identifying of ultrasound energy delivery is performed at one or more time points or continuously during the activation of the one or more ultrasound transducers.

According to some exemplary embodiments, the degradation in ultrasound energy generation and/or delivery, for example efficiency of ultrasound energy generation and/or delivery is identified at block 603 by monitoring impedance values of the at least one ultrasound transducer during the activation of the transducer. In some embodiments, impedance values of each ultrasound transducer or a group of ultrasound transducers are measured to identify changes in impedance values during the activation of the ultrasound applicator, for example changes relative to a reference value indication stored in a memory of the system. In some embodiments, changes in impedance values of an ultrasound transducer indicate lack of contact of the ultrasound transducer with a skin and/or failure in the activity of the ultrasound transducer. In some embodiments, impedance values are measured based on changes in electrical signals transmitted by an electrical circuitry electrifying the ultrasound transducer, and electrical signals received by the electrical circuitry from the ultrasound transducer.

Additionally or alternatively, the degradation in ultrasound energy delivery, for example efficiency of ultrasound energy delivery is optionally identified at block 603 based on signals received from one or more temperature sensors, during the activation of the ultrasound applicator at block 601, for example during the activation of at least one ultrasound transducer of the applicator. In some embodiments, the one or more temperature sensors sense temperature levels or changes thereof inside the ultrasound applicator and/or near the ultrasound transducers. In some embodiments, an increase in temperature levels, for example due to increase in heat, during the activation of the ultrasound applicator optionally indicate lack of contact of one or more of the ultrasound transducers with the skin. In some embodiments, the temperature levels or changes thereof are sensed by one or more temperature sensors, for example thermistors of the ultrasound applicator located near the ultrasound transducers and/or near an emitting surface of the ultrasound applicator contacting the skin.

According to some exemplary embodiments, an indication is generated and delivered at block 605. In some embodiments, the indication is generated and delivered if a degradation in ultrasound energy delivery, for example a degradation in ultrasound energy deliver efficiency is larger than 10% or a reference or a baseline value. In some embodiments, the indication is a human detectable indication, for example an audio and/or a visual indication delivered to a user of the ultrasound system. In some embodiments, an indication delivered to the user include instructions to proceed with the activation of the ultrasound or to replace the ultrasound applicator.

Alternatively or additionally, the indication comprises at least one signal transmitted to a remote device, for example a remote computer, a remote server, a remote cloud storage or any other device not located within the same room as the ultrasound system. In some embodiments, the at least one transmitted signal comprises at least one log file of the system activity.

According to some exemplary embodiments, an activity of the ultrasound applicator, for example an activity of at least one ultrasound transducer is modified at block 607. In some embodiments, if degradation in ultrasound energy delivery is identified, the activity of at least one ultrasound transducer, for example the at least one ultrasound transducer in which undesired values of impedance are measured, I stopped. Optionally, the activity of the at least one ultrasound transducer is stopped while activation of other ultrasound transducers of the ultrasound applicator continues. In some embodiments, when stopping the activation of at least one ultrasound transducer, activation duration and/or activation power, for example voltage and/or current, is increased in other ultrasound transducers that remain active.

According to some exemplary embodiments, if degradation in ultrasound energy delivery is identified, the activity of the ultrasound applicator, for example the activity of all ultrasound transducers of the ultrasound applicator is disabled. In some embodiments, the activity modification of at least one ultrasound transducer and/or the disabling of the activity of the ultrasound applicator is performed remotely, for example using signals from the remote device. In some embodiments, a user of the ultrasound system receives instructions to change a position and/or an orientation of the ultrasound applicator relative to the skin if degradation in ultrasound energy delivery is identified at block 603.

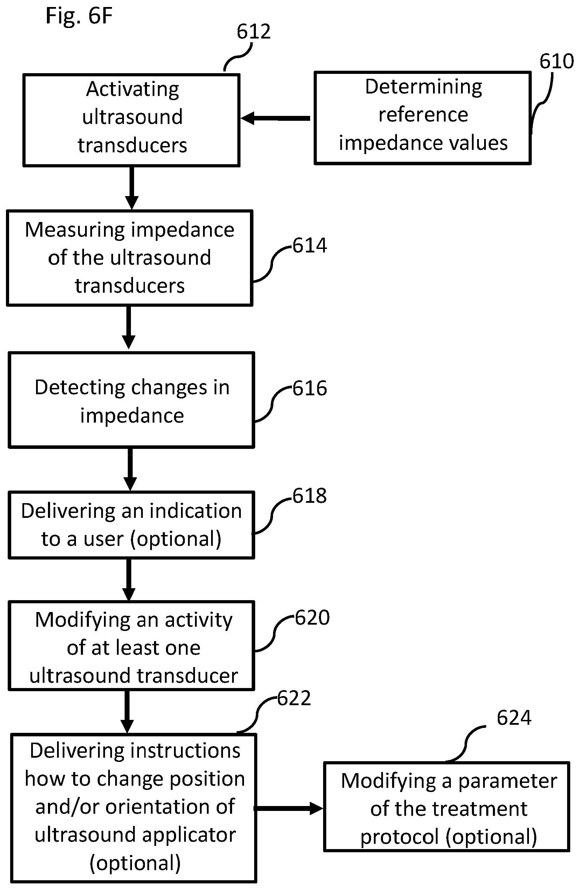

Reference is now made to fig. 6F depicting a process for measuring impedance of one or more ultrasound transducers and detection of changes in impedance values, according to some exemplary embodiments of the invention.

According to some exemplary embodiments, impedance reference values of two or more ultrasound transducers are determined at block 610. In some embodiments, the two or more ultrasound transducers are activated separately. In some embodiments, impedance values of each ultrasound transducer are measured over a selected range of frequencies, for example in a range of 5-20 MHz, 9-13MHz, 5-10MHz, 10-20MHz or any intermediate, smaller or larger range of frequencies. In some embodiments, the measured impedance values are stored as reference impedance values for each transducer of the two or more transducers of an ultrasound applicator.

In some embodiments, the impedance measurements performed at block 610 are performed as part of a calibration process of each ultrasound transducer or a calibration process of the ultrasound applicator. In some embodiments, the calibration process is performed prior to a treatment process of a patient. Optionally, the calibration process is performed as part of the manufacturing of the ultrasound applicator and/or system.

According to some exemplary embodiments, the two or more ultrasound transducers are activated at block 612 as part of a treatment process. In some embodiments, the two or more ultrasound transducers are activated with activation parameters values selected to generate one or more specific heated regions in deep layers of the skin. In some embodiments, the two or more ultrasound transducers are activated to generate ultrasound waves with similar parameter values, for example similar values of amplitude and/or frequency. Alternatively, the two or more ultrasound transducers are activated to generate ultrasound waves with different parameter values, for example with different values of amplitude and/or frequency. In some embodiments, the activation parameters of the ultrasound transducers comprise activation duration, frequency, acoustic intensity, pre-cooling, post-cooling, and drive voltage.

According to some exemplary embodiments, each of the ultrasound transducers is activated in a range of frequencies that is included in the range of frequencies used during a calibration process performed at block 610.

According to some exemplary embodiments, each or at least some of the ultrasound transducers are activated separately from the rest of the ultrasound transducers. In some embodiments, each of the ultrasound transducers is connected to a different electrical circuitry configured to deliver electricity to the ultrasound transducer. Alternatively, at least some of the ultrasound transducers are connected to the same electrical circuitry.

According to some exemplary embodiments, impedance values of the ultrasound transducers are measured at block 614. In some embodiments, the impedance values are measured before, during or after the delivery of an ultrasound energy pulse. In some embodiments, the impedance values are measured separately for each of the ultrasound transducers. Alternatively, an impedance value is measured for two or more of the transducers. In some embodiments, an average or mean impedance value is measured for two or more of the transducers, for example for all of the activated transducers. In some embodiments, the impedance values are measured for a range of frequencies included in the frequency range used for the calibration process at block 610.

According to some exemplary embodiments, the impedance values are measured by a control circuitry of the ultrasound system. In some embodiments, the impedance values of each transducer are measured by the electrical circuitry connected to the ultrasound transducer. In some embodiments, the electrical circuitry is connected to the control circuitry. In some embodiments, the electrical circuitry for each ultrasound transducers is electrically isolated from the rest of the electrical circuitries and/or from other ultrasound transducers. In some embodiments, the electrical circuitry is connected to or is part of an ultrasound card located, for example, in a control console of the system.

According to some exemplary embodiments, changes in impedance values are detected at block 616. In some embodiments, the changes are detected by the control circuitry of the ultrasound system. In some embodiments, changes in impedance values compared to the reference impedance values measured at block 610, are detected. In some embodiments, changes in impedance values along a range of frequency values compared to the impedance reference values measured at block 610 are detected. In some embodiments, changes in impedance values between different ultrasound transducers are detected. In some embodiments, changes between impedance values of one or more ultrasound transducers, and an average or mean impedance value are detected.

According to some exemplary embodiments, the changes in impedance values are detected by determining a relation between impedance values measured at block 616 and the stored reference impedance values, measured at block 610.

According to some exemplary embodiments, an indication is optionally delivered at block 618. In some embodiments, an indication, for example a human detectable indication is delivered to a user of the ultrasound system. In some embodiments, the indication is delivered if the detected changes in impedance values are higher than a predetermined value. In some embodiments, the indication is delivered if the detected changes are higher than 1%, for example higher than 5%, higher than 10%, higher than 20% or any intermediate, smaller or larger percentage of change between impedance values of an ultrasound transducers and impedance values of other transducers or an average or mean impedance value. Alternatively or additionally, the indication is delivered if the detected changes are higher than 1%, for example higher than 5%, higher than 10%, higher than 20% or any intermediate, smaller or larger percentage of change between measured impedance values of an ultrasound transducer and stored reference impedance values, for example reference values of the same ultrasound transducer.

According to some exemplary embodiments, activity of at least one ultrasound transducer, for example an ultrasound transducer where impedance changes are detected, is modified at block 620. In some embodiments, the activity is modified based on the changes in impedance detected at block 616. In some embodiments, modifying activity of an ultrasound transducer comprises modifying electrical current and/or voltage values delivered to the at least one ultrasound transducers. Alternatively or additionally, modifying activity of an ultrasound transducer comprises modifying at least one of vibration duration, vibration frequency, and vibration amplitude of a PZT element of the at least one ultrasound transducer.

According to some exemplary embodiments, the activity of the at least one ultrasound transducer is modified if the detected changes in impedance values are higher than a predetermined value. In some embodiments, the activity of the at least one ultrasound transducer is modified if the detected changes are higher than 1%, for example higher than 5%, higher than 10%, higher than 20% or any intermediate, smaller or larger percentage of change between impedance values of the at least one ultrasound transducer and impedance values of other transducers, an average or mean impedance value, or stored reference impedance values.

According to some exemplary embodiments, modifying an activity of at least one ultrasound transducer comprises stopping the activity of the at least one ultrasound transducer. Alternatively, modifying an activity comprises modifying an activity of one or more ultrasound transducer to compensate for the undesired activity of one or more ultrasound transducers as indicated by the detected changes in impedance values.

According to some exemplary embodiments, the activity of at least one ultrasound transducer is selectively modified in order, for example, to generate a predetermined heated volume, optionally having specific size and/or shape.
According to some exemplary embodiments, instructions on how to change position and/or orientation of the ultrasound applicator relative to the skin surface are optionally delivered at block 622. In some embodiments, the instructions are provided according to the changes in impedance values detected at block 616.

According to some exemplary embodiments, at least one parameter of a skin treatment protocol is optionally modified at block 624. In some embodiments, the at least one treatment protocol parameter comprises the duration needed to activate at least some of the ultrasound transducers per a specific treatment region in the skin. In some embodiments, at least one treatment protocol parameter comprises the number and/or treating order of skin regions, for example per a single treatment session.

### Exemplary ultrasound system for delivery of skin treatments

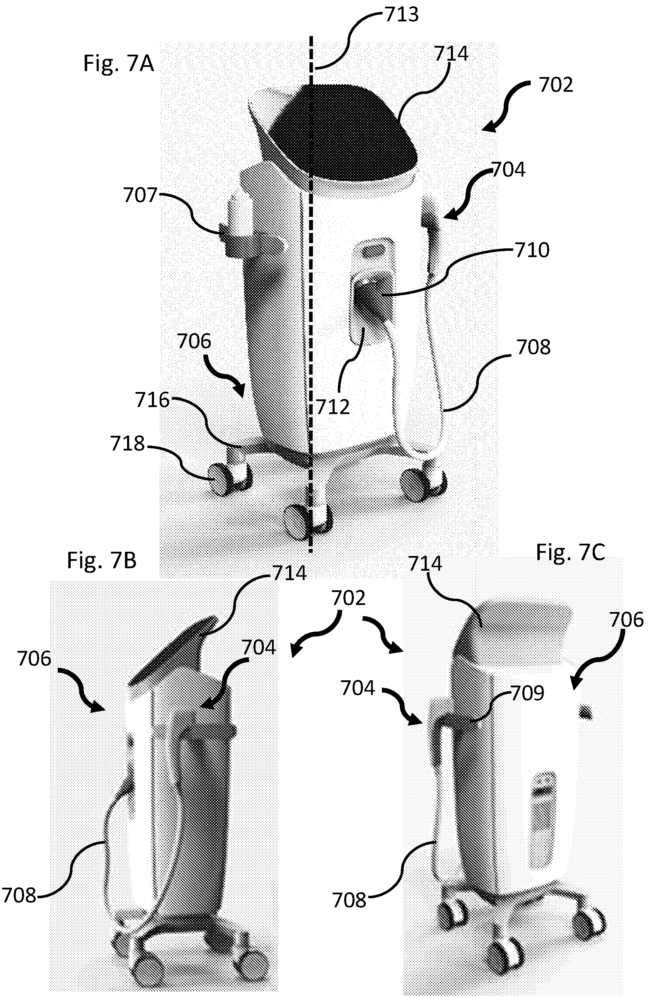

According to some exemplary embodiments, an ultrasound system for delivery of skin treatment is a movable system configured to move on a surface, for example a floor, and to be positioned next to a subject to be treated by the system. In some embodiments, the system is shaped as a tower having a small footprint on the floor. Reference is now made to figs. 7A-7C, depicting a movable ultrasound system for delivery of skin treatments, according to some exemplary embodiments of the invention.

According to some exemplary embodiments, a movable ultrasound system, for example system 702 comprises an ultrasound applicator 704 and a control console, for example console 706. In some embodiments, the applicator 704 is coupled to the console 706 via an umbilical connecting channel 708.

According to some exemplary embodiments, the connecting channel 708 is an elongated connecting channel having a length of at least 1 meter, for example at least 1.5 m, at least 2 m or any intermediate shorter or longer connecting channel 708. In some embodiments, the connecting channel is flexible, for example to allow movement of the applicator to different treatment locations in the subject. In some embodiments, the connecting channel comprises a proximal end close to the control console 706 and a distal end, connected to the applicator 704. In some embodiments, the proximal end of the connecting channel 708 comprises at least one connector 710 shaped and sized to connect to at least one main socket 712 in the console 706.

According to some exemplary embodiments, the console has a vertical axis 713, a lower end and an upper end. In some embodiments, a lower end of the console 706 comprises a wheel base 716 comprising at least two wheels facing a surface on which the console 706 is positioned in an upright orientation, for example when the vertical axis of the console 706 is perpendicular to the floor. In some embodiments, a projection of the console 706 on a surface on which the console is positioned in an upright orientation has a surface area size of less than 2m², for example less than 1.5m² or any intermediate, smaller or larger surface area.

According to some exemplary embodiments, an upper end of the console 706 comprises a display 714. In some embodiments, the display is tilted in a range between 30° degrees to 60° degrees, for example 30° degrees-45° degrees, 40° degrees- 50° degrees or any intermediate, smaller or larger range of values, for example to allow visualization of the display from different angles. In some embodiments, a back surface of the display is positioned in an angle relating to the console.

According to some exemplary embodiments, the console 706 comprises n applicator holder 709, for example to hold the applicator 704 when not in use. In some embodiments, the console comprises at least one bottle holder 707, shaped and sized to hold a bottle, for example a bottle of ultrasound gel.

### Exemplary applicator

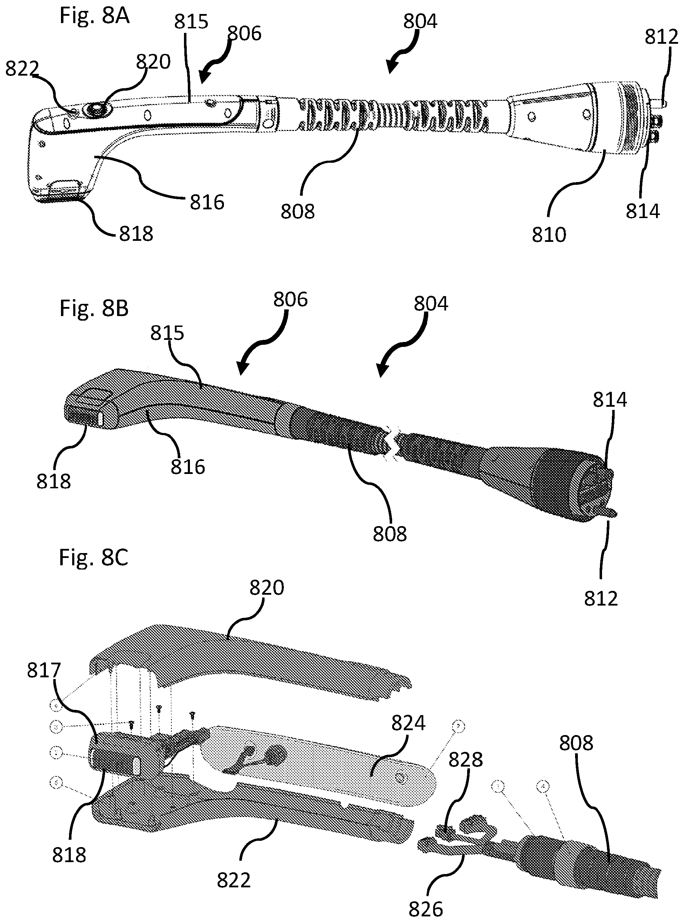

Reference is now made to Figs. 8A-8C depicting an ultrasound applicator, according to some exemplary embodiments of the invention.

According to some exemplary embodiments, an ultrasound applicator, for example applicator 804 comprises an applicator body 806 and an umbilical connecting channel 808. In some embodiments, the applicator body is coupled to a distal end of the channel 808. In some embodiments, a proximal end of the channel 808 comprises at least one console connector, configured to connect the applicator 804 to a control console of the ultrasound system. In some embodiments, the console connector comprises at least one electrical grounding connector, for example pin 812, configured to allow grounding of the applicator. In some embodiments, at least one console connector of the channel 808 comprises at least one coolant liquid connector, configured to connect a cooling system of the applicator 804 to a cooling system of the console, for example to allow circulation of coolant liquid between the applicator 804 and the console.

According to some exemplary embodiments, the applicator body 806 comprises a handle 815, for example an elongated handle, having at least one gripping member, shaped and sized to be held by a single hand of an operator. In some embodiments, the applicator body 806 comprises an emitting head portion 816 oriented in an angle between 70° degrees-180 ° degrees, for example 70° degrees-90° degrees, 80° degrees-100° degrees, 90° degrees-120° degrees or any intermediate, smaller or larger range of values, to the handle 815. A potential advantage of positioning an emitting head portion in an angle relating to a handle may be to allow easy directing of the emitting head portion to a treatment area in a subject, and/or to allow easier application of force by an operator holding the applicator body against the treatment area.

According to some exemplary embodiments, the emitting head portion 816 comprises a plurality of transducers 818, for example two or more transducers 818 near a surface of the applicator, which is shaped and sized to be placed in contact with the skin. In some embodiments, the transducers are linearly organized in the form of an array underneath and near the skin contacting surface of the emitting head portion 816.

According to some exemplary embodiments, the handle 815 of the applicator body 806 comprises a user interface comprising at least one activation switch, for example switch 820, and/or at least one indicator, for example a visual and/or a sound indicator configured to generate at least one human detectable indication to an operator holding the handle 815. In some embodiments, the indicator comprises a visual indicator, for example a LED visual indicator 822. In some embodiments, the user interface is oriented on the handle in distance from the at least one gripping member of the handle selected to allow holding and activating the user interface with the same hand of an operator. In some embodiments, the user interface is positioned on the handle 815 at a viewing angle of an operator holding the applicator body 806.

According to some exemplary embodiments, the handle 815 is elongated and optionally at least partly cylindrical, having a width or diameter small enough to be positioned within a palm of an operator's hand.

According to some exemplary embodiments, for example as shown in fig. 8C, a casing, for example a rigid casing, of the applicator body is formed from two complementary side panels 820 and 822, an energizer cover 817 and an upper panel 834 comprising a user interface. In some embodiments, electrical wiring 828 and/or coolant fluid tubes 826 pass within a lumen of the applicator body casing and into an inner lumen of the umbilical connecting channel 808.

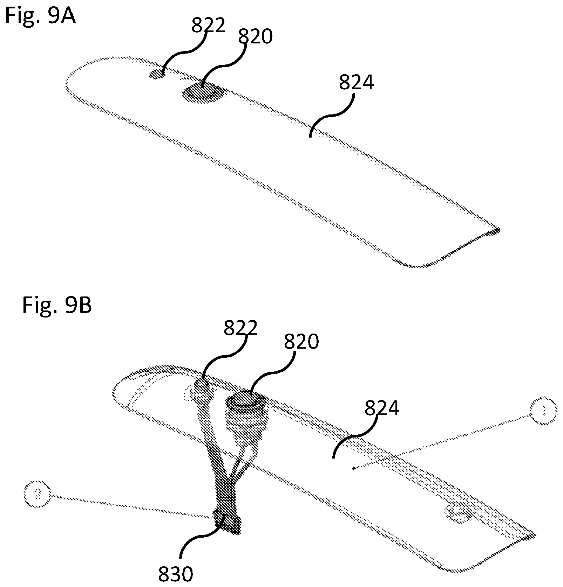

Reference is now made to figs. 9A and 9B depicting an upper panel of the applicator body casing, according to some exemplary embodiments of the invention.

According to some exemplary embodiments, the upper panel 824 of the casing comprises an applicator user interface, configured to deliver at least one indication to an operator of the system. Alternatively or additionally, the user interface of the applicator is configured to receive input from the operator, for example ultrasound delivery activation command for delivery of at least one pulse of ultrasound waves into the skin.

According to some exemplary embodiments, for example as shown in fig. 9A, the applicator user interface comprises at least one activation switch, for example activation button 820. In some embodiments, pressing the activation button 820, delivers ultrasound waves into the skin. In some embodiments, the activation button is water resistant, configured to prevent penetration of water or any other liquid into the applicator body.

According to some exemplary embodiments, the applicator user interface comprises at least one visual indicator, for example LED 822. In some embodiments, the visual indicator delivers a visual human detectable indication to an operator, for example according to a status of the system, or according to the status of the activation button 820. In some embodiments, an operator of the system monitors the delivery of the ultrasound waves based on signals received from the at least one visual indicator of the applicator, for example, when a user interface of the control console, for example the display of the console, is not within a line of sight of the operator.

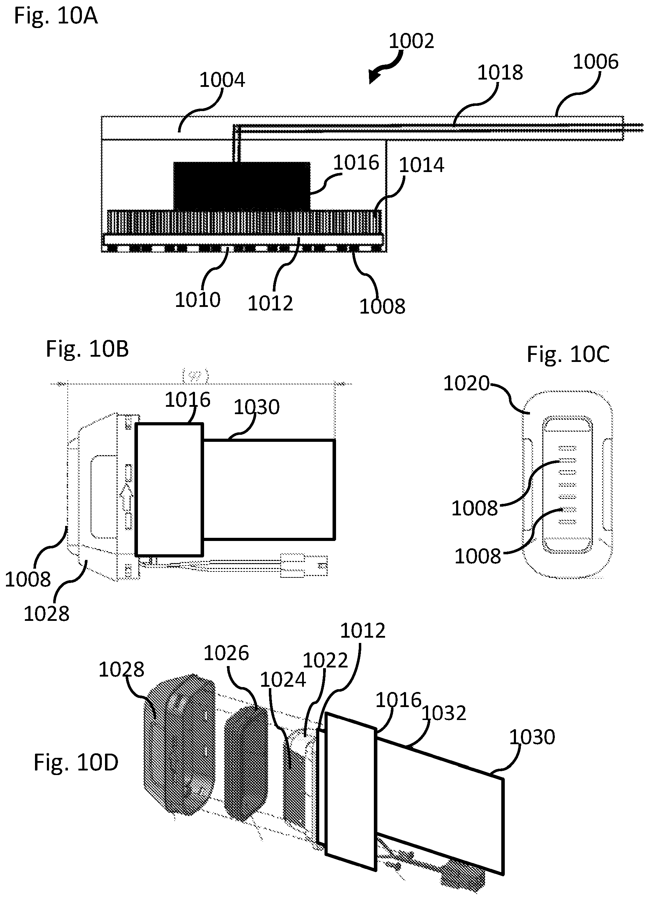

Reference is now made to figs. 10A-10D depicting arrangement of applicator components, according to some exemplary embodiments of the invention.

According to some exemplary embodiments, an applicator 1002 comprises an applicator body 1004 and an applicator handle 1006. In some embodiments, the handle 1006 is an elongated handle, shaped to be positioned within a palm of a hand. In some embodiments, the applicator body 1004 and handle 1006 comprise a single inner lumen, in which energizing components, for example wiring of the applicator are positioned.

According to some exemplary embodiments, the applicator 1002 comprises at least two piezoelectric elements (PZT), for example ceramic PZT, positioned near a surface of the applicator body shaped and sized to contact the skin. In some embodiments, the PZT are configured to vibrate and generate ultrasound waves in response to an electric current. In some embodiments, for example as shown in figs. 10A and 10C, the PZT elements are arranged in an array of elements, for example a linear array, where the PZT elements are arranged along a linear axis of the applicator body.

According to some exemplary embodiments, at least one heat sensor 1010, for example at least one thermistor is positioned near the PZT elements, for example between adjacent PZT elements of the array. In some embodiments, the at least one heat sensor 1010 is configured to record temperature levels of the skin in a timed relation with the delivery of ultrasound waves, for example before, during and/or following the ultrasound waves delivery.

According to some exemplary embodiments, for example as shown in fig.10A, the PZT elements, for example a surface of each PZT element is placed in contact with a thermoelectric cooler (TEC). In some embodiments, the surface of the PZT element is placed in contact with a cold surface of the TEC. Alternatively, for example as shown in fig. 10D, the PZT elements are arranged on at least one heat conducting transducers holder, for example holder 1022. In some embodiments, a surface of the holder 1022 is placed in contact with the TEC 1012, for example with a cold surface of the TEC 1012. In some embodiments, the at least one heat conducting holder 1022 is configured to transfer heat from the PZT elements to a cold surface of a TEC. In some embodiments, the heat-conducting holder comprises or, is at least partly made from a heat conducting material, for example aluminum.

According to some exemplary embodiments, for example as shown in figs. 10A and 10D, a hot surface of the TEC is in contact with a surface of a heat exchanger, for example a water-cooled heat exchanger 1016. In some embodiments, the heat exchanger 1016 is configured to cool down the hot surface of the TEC by a coolant fluid 1018 within the heat exchanger. In some embodiments, the coolant fluid 1018 circulates between a cooling system in a control console and the heat exchanger 1016 of the applicator via ports 1016 of the heat exchanger.

According to some exemplary embodiments, for example as shown in fig. 10A, a heat sink 1014 is positioned between a hot surface of the TEC and a surface of the heat exchanger 1016.

According to some exemplary embodiments, the applicator comprises at least one main printed circuit board (PCB), having a flex portion 1024 electrically connected to a rigid board 1030. In some embodiments, the PCB transmits an electric power signal to the transducers, and receives electric signals from the transducers and/or from the at least one temperature sensor, for example the at least one thermistor 1010. In some embodiments, the flex portion 1024 of the PCB is flexible and is shaped to bend at least partly around the holder 1022 to electrically connect the transducers and/or the at least one thermistor to the rigid board 1030.

According to some exemplary embodiments, an energizer assembly of the applicator 1002 comprises the PZT elements 1008 arranged on the holder 1022, the TEC 1012 placed in contact with the holder 1022 and the heat exchanger 1016. Additionally, the energizer assembly comprises the PCB for example the flex-PCB coupled to the transducers and/or thermistors.

According to some exemplary embodiments, the energizer assembly is sealed from liquid and/or air, or example by a gasket 1026 placed around the holder 1022, and a cover 1028 firmly coupled to the gasket 1026.

### Exemplary energizer assembly

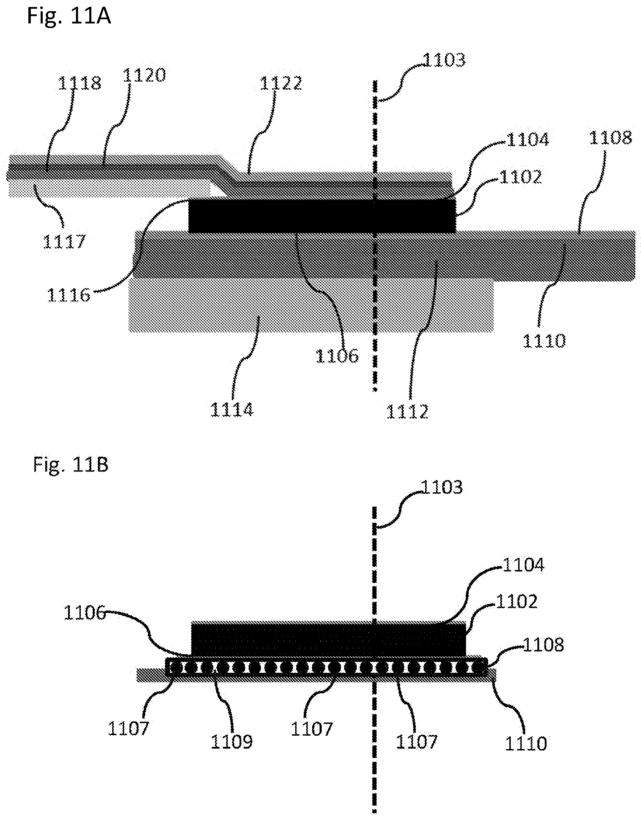

According to some exemplary embodiments, a PZT-holder assembly of an energizer is configured to resist pressure applied on the PZT elements, for example when during contact on the applicator with the skin. In some embodiments, in order to resist the applied pressure, the PZT element are glued to a holder, for example to limit movement of the PZT under the applied pressure. Additionally, the PZT elements are sealed by at least one sealing layer, for example to prevent liquid and/or air contact with electrical conducting elements of the energizer. Reference is now made to figs. 11A and 11B showing interactions between layers of an energizer unit, according to some exemplary embodiments of the invention.

According to some exemplary embodiments, a PZT element, for example PZT 1102 has a longitudinal axis 1103, and comprises a first electrode contact, for example upper electrode 1104, and a second electrode contact, for example a lower electrode 1106. In some embodiments, a thickness of a PZT element is in a range of 150 µm -220 µm, for example 150 µm- 180 µm, 170 µm-200 µm, 180 µm -220 µm or any intermediate, smaller or larger range of values.

According to some exemplary embodiments, the upper electrode 1104 and the lower electrode 1106 are electrically conductive and configured to deliver electric power to the PZT element 1102. In some embodiments, an electrically conductive layer 1108 comprising a non-electrically conductive filler 1109, for example a non-conductive adhesive, and conductive particles 1107, for example layer 1108 is positioned between the lower electrode 1106 and a conductive metal strip, for example a copper strip 1110. In some embodiments, electricity is transmitted between the copper strip 1110 and the lower electrode 1106 through the electrically conductive particles in the layer 1108.

According to some exemplary embodiments, for example during manufacturing, pressing on the PZT element towards the copper strip 1110 allows movement of the PZT element within the layer 1108 until a monolayer of conductive particles positioned between the lower electrode 1106 of the PZT and the copper strip underneath the layer 1108, is formed within the layer 1108. In some embodiments, the particles in the layer 1108 have a similar size and/or diameter.

According to some exemplary embodiments, optionally at least 10%, for example at least 20%, at least 30%, at least 40% or any intermediate, smaller or larger percentage volume of the filler 1109 contains the conductive particles 1107.

According to some exemplary embodiments, the lower copper strip 1110 has a thickness in a range of 30 µm- 80 µm, for example 30 µm-50 µm, 40 µm-70 µm, 60 µm-80 µm or any intermediate, smaller or larger range of values. In some embodiments, a layer of glue, for example a non-conducting glue layer 1112 is positioned between the lower copper strip 1110 and a surface of a heat-conducting holder, for example an aluminum holder 1114. In some embodiments, the non-conducting glue layer 1112 between the holder 1114 and the lower copper strip 1110 is a rigid glue layer. In some embodiments, pressing the PZT element against the holder 1114 through the adhesive layer 1108 allows, for example, to fixate the attachment of the PZT element to the holder, and optionally to eliminate future movement of the PZT, for example in response to pressure from the skin. Alternatively or additionally, pressing the PZT element against the holder 1114 through the adhesive layer 1108 forming a monolayer of conductive particles levels the PZT element on a horizontal plane perpendicular to the longitudinal axis 1103 of the PZT element 1102 due to the monolayer of conductive particles in the layer 1108 keeping, for example the PZT element 1102 in an equal distance from the holder along the entire surface of PZT element 1102.

According to some exemplary embodiments, an upper electrode 1104 of the PZT element 1102 is coupled to an electrical conducting strip, for example a silver strip 1117 by gap (spark) welding.

According to some exemplary embodiments, a layer of strong non-conductive glue, for example a non-conductive strong epoxy glue 1118 is applied on top the upper electrode 1104 of the PZT 1102, for example to ensure a strong adhesion between the PZT element 1102 and other components of the energizer. In some embodiments, the layer of the strong non conducting glue 1118 has a thickness in a range of 5 µm-20 µm, for example 5 µm-15 µm, 7 µm-12 µm, 20 µm-20 µm or any intermediate, smaller or larger range of values.

According to some exemplary embodiments, a polyimide, for example Kapton, layer 1120 coats the non-conductive strong glue 1118. In some embodiments, the Kapton layer 1120 has a thickness in a range of 10 µm -14 µm, for example 10 µm, 12 µm, 12.5 µm, 13 µm or any intermediate, smaller or larger value. In some embodiments, an additional layer of Parylene coating 1122 is formed on top the Kapton coating, for example to prevent direct contact between the PZT element 1102 and the skin, and/or to prevent exposure of the PZT element 1102 to liquid and/or air.

According to some exemplary embodiments, the Parylene coating 1122 is a Parylene-C coating having a thickness of at least 5 µm, for example at least 8 µm, at least 10 µm or any intermediate, smaller or larger value. In some embodiments, gaps, for example predesigned gaps between the frame and the holder allows for example the coating to penetrate into the internal cavity between the frame and the holder, and to cover, for example evenly cover all surfaces.

Potential advantages of the Parylene coating may be to allow one or more of the following, bio-compatibility of an applicator surface configured to be placed in contact with a skin of a subject, electric isolation of all the exposed electrically conductive surfaces and corrosion prevention inside the frame cavity, mechanical stiffening and stabilization of the thin silver strips and the PZT positioning, increased electric isolation of an applicator surface configured to be in contact with the skin /surrounding environment, and increased sealing against water and humidity penetration through the applicator surface.

According to some exemplary embodiments, the copper strip 1110 that is electrically connected to the lower PZT electrode 1106 is electrically isolated from the Aluminum holder 1114, for example by a non-conductive glue film, and optionally by passivation of the Aluminum surface. In some embodiments, electrical isolation of the copper strip allows, for example, measuring capacitance and/or impedance on each PZT separately, with minimal or no errors/noises from other PZT elements, and optionally no capacitive effects of water electrolytes on the flex external surface. In addition, it will also allow separate operation of different transducers in different phase and/or different frequencies.

Reference is now made to fig. 11B, depicting an electrically conducting layer comprises a non-electrically conducting filler and electrically conducting particles within the filler, where the particles are positioned between an electrode of the PZT and an electrical conductor, according to some exemplary embodiments of the invention.

According to some exemplary embodiments, at least one electrode of the PZT element 1102, for example a lower electrode 1106 is placed in contact with the electrically conductive layer 1108, comprising a non-electrically conductive filler 1109 and electrically conductive particles 1107. In some embodiments, at least 50% of the particles 1107, for example at least 60%, at least 80%, at least 90%, at least 95% or any intermediate, smaller or larger percentage of the particles 1107 have a similar size or diameter. Optionally, at least 60%, at least 80%, at least 90%, at least 95% or any intermediate, smaller or larger percentage of the particles 1107 have a spherical or a round shape.

According to some exemplary embodiments, a size or a diameter of the particles vary in less than 10%, for example less than 8%, less than 5%, less than 4% or any other intermediate, smaller or larger value from an average size or diameter of the electrical conducting particles in the non-conducting filler 1109.

According to the invention, the particles are arranged as a monolayer between at least one electrode of the PZT, for example electrode 1106 and an electrical conductor, for example copper strip 1110. In some embodiments, at least one electrode and the electrical conductor contact the particles 1107 at two different contact points, for example two different contact points on a curved external surface of the particles 1107. Optionally, the two contacting points are points on a circumference of a particle and are located on the same diameter of the particle.

According to some exemplary embodiments, the particles 1107 conduct electricity between at least one PZT electrode and the electric conductor, through the contacting points. In some embodiments, at least some or all of the particles 1107 comprise a non-electrical conductive core, for example a polymer core, surrounded by a layer of electrical conductive material, for example an electrical conductive metal. In some embodiments, the contacting points between the particles 1107 and the at least one electrode and the electrical conductor are positioned on the electrical conducting layer of each particle. In some embodiments, electricity is conducted through the circumference of each particle between the two contacting points, and not through the core of each particle.

According to some exemplary embodiments, the surrounding electrical conductive layer of each particle is made from a heat-conducting material. In some embodiments, the particles 1107 conduct heat away from the PZT element through the surrounding layer of each particle, for example towards a holder, a heat sink or a cold surface of a TEC.

According to some exemplary embodiments, the surrounding layer of each particle or particles 1107 is formed from a softer material, optionally elastic, compared to the material of the rigid core of each particle. In some embodiments, the soft surrounding layer of each particle contacting the at least one electrode of the PZT element allows, for example, less dampening of the PZT element vibrations compared to a surrounding layer made from a rigid material. In some embodiments, the surrounding layer of each particle contacting the PZT element or the at least one electrode of the PZT element is configured to elastically bend when the PZT element vibrates.

### Exemplary coupling an electrical conducting strip to PZT electrode

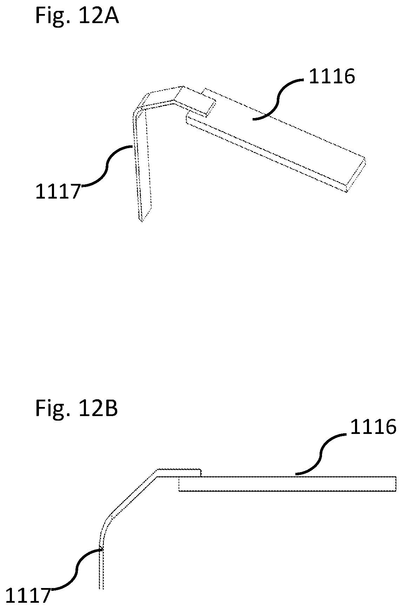

Reference is now made to figs. 12 A and 12B depicting coupling of an electrical conducting strip to an upper PZT electrode, according to some exemplary embodiments of the invention.

According to some exemplary embodiments, an upper PZT electrode 1116 is shaped and sized to be pressed by a skin surface contacting the applicator. In some embodiments, the upper PZT electrode 1116 is planar, and flat, for example does not contain bulges and/or protrusions extending towards the skin, in order to prevent injury to the skin when the applicator is pressed against the skin.

According to some exemplary embodiments, an electrical conducting thin strip 1117 is coupled to the upper PZT electrode 1116 by spark welding, and not soldering. In some embodiments, the strip 1117 is a flat and wide strip, having a width in a range of 0.8mm-2mm, for example 0.8mm-1.5mm, 1mm-1.2mm, 1.2mm-2mm or any intermediate, smaller or larger range of values.

Potential advantages of using spark welding and not soldering may include preventing soldering bulges, and/or stress concentration when pressing on the PZT element from above, for example when the applicator is pressed against the skin, and prevention of solder usage thus allowing repeatable transmission-block electrode area coverage.

Potential advantages of a wide electric conducting strip may be a larger electric current conductivity compared to a wire, and/or suitability for standard commercial PCB automated flat assembly technologies for mass production.

### Exemplary thermistors flex-PCB

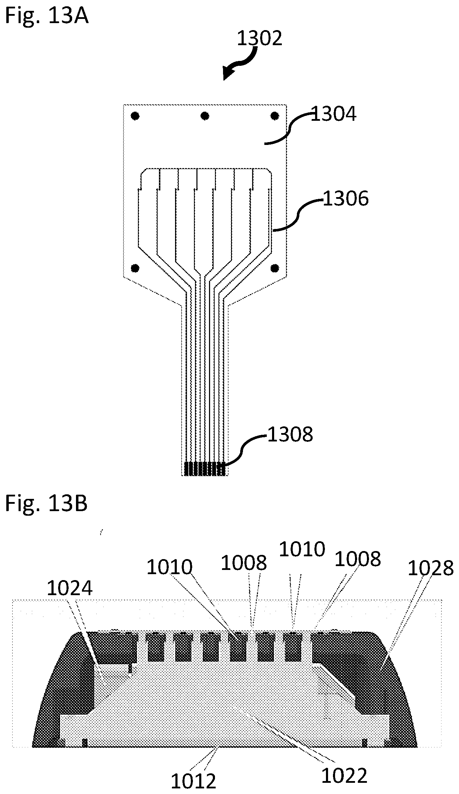

Reference is now made to figs. 13A and 13B depicting a flex PCB with thermistors circuitry, according to some exemplary embodiments of the invention.

According to some exemplary embodiments, for example as shown in fig. 13A, a flex-PCB, for example flex-PCB 1302 comprises thermistors circuitry. In some embodiments, the flex-PCB 1302 is a flex-Kapton (polyimide) PCB. In some embodiments, a thickness of the flex-PCB varies, for example a thickness of a region designed to be positioned on top PZT elements is in a range of 15µm-30µm, for example 15 µm-25 µm, 20 µm-30 µm or any intermediate, smaller or larger range of values. In some embodiments, a thickness of other regions of the flex-PCB is in a range of 80 µm-120 µm, for example 80 µm-100 µm, 90 µm-110 µm, 100 µm-120 µm or any intermediate, smaller or larger range of values.

According to some exemplary embodiments, the flex-PCB comprises a wide electrical wiring region 1308, and a wide distal region 1304 between the electrical wiring region 1306 and a distal end of the flex-PCB, with no electrical wiring. In some embodiments, the flex-PCB comprises a narrow electrical wiring region con, which is more rigid than the region 1308. In some embodiments, an arrangement of thermistors on the flex-PCB matches an arrangement of PZT elements, for example bending of the flex-PCB positions the thermistors of the flex-PCB between adjacent PZT elements, for example as shown in fig. 13B. In some embodiments, when installed, the flex-PCB is oriented, for example flipped, in a way that does not place the electrical wiring of the flex-PCB 1302 between the PZT elements and the skin.

According to some exemplary embodiments, the flex-Kapton PCB 1302 is configured to seal the energizer assembly face from one or more of water/humidity/Ultrasound gel etc. In some embodiments, the small thickness of the flex-Kapton PCB above the PZT elements allows, for example, the ultrasonic energy to pass through the flex PCB material with negligible energy loss. Additionally, the relative rigidity of Polyimide allows, for example, better resistance to physical impacts from the environment, for example, to maintain the sealing of the energizer.

Potential advantage of using a flex-Kapton PCB may be to allow manufacturing of the flex-PCB in standard mass production methods, for example to reduce manufacturing costs. In addition, the flex-Kapton PCB may allow electric isolation from the skin/surrounding, for example an electric break level of at least 1500 V, for example at least 1700V, at least 2000, at least 2500V or any intermediate, smaller or larger value.). In addition, the small thickness of the flex-Kapton PCB allows, for example, fast heat transfer from the skin to the thermistors, thus allowing continually updated temperature measurement of the skin, and the thermistors are used as aligners to correct a positioning of the flex portion of the flex-PCB, by optionally positioning the thermistors of the flex region in specific grooves of the frame.

In addition, the flex-PCB allows a simple electric connection of the flex circuitry to the applicator main PCB.

According to some exemplary embodiments, the flexible PCB 1302 comprises a wide electrical wiring region 1306, and a wide distal region 1304 between the electrical wiring region 1306 and a distal end of flexible PCB 1302, with no electrical wiring. In some embodiments, the flexible PCB 1302 also comprises a narrow electrical wiring region 1308, which is more rigid than region 1306. In some embodiments, rigid, narrow electrical wiring region 1308 of flexible PCB 1302 serves as a connector and allows, for example, a simple electric connection of the flexible circuitry to an applicator main PCB.

According to some exemplary embodiments, an arrangement of thermistors 1153 on the flexible PCB 1302 matches an arrangement of spaced-apart transducers 1150, for example, bending of flexible PCB 1302 positions the thermistors 1153 of flexible PCB 1302 between adjacent transducers 1150, as shown for example in FIG. 16C. In some embodiments, the location of spaced-apart thermistors 1153 in proximity to each ultrasound transducer 1150, which is optionally about 1.0mm thick, allows, for example, to monitor and communicate instantly to a control console the skin and treated skin region temperature.

Although Kapton is not an efficient heat conductor, the small thickness of the flexible PCB 1302 made from Kapton, allows, for example, fast heat transfer from the skin to thermistors 328, thus optionally allowing continually updated temperature measurement of the skin.

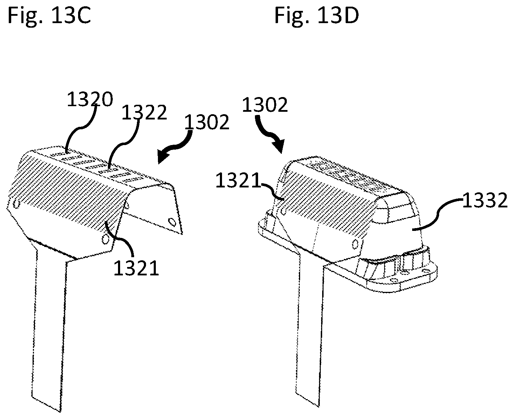

Reference is now made to figs. 13C-13D depicting assembly of the flex-PCB on top the PZT elements, according to some exemplary embodiments of the invention.

According to some exemplary embodiments, the flex-PCB, for example the flex-Kapton PCB comprises two or more openings, for example openings 1320 and 1322, which are shaped and sized to allow penetration of PZT elements arranged in an array through the flex-PCB 1302. In some embodiments, once the flex-PCB is positioned such that the PZT elements penetrate through the openings, for example openings 1320 and 1322, a frame 1330is positioned on top the flex-PCB 1302, for example to adhere the flex-PCB to the holder 1332.

According to some exemplary embodiments, a strong adhesive, for example EpoTEK ND353 glue is applied between the PZT and a frame 1330, and the Flex-PCB 1302. In some embodiments, following application of glue, pressure is applied on the assembly while curing in an oven. In some embodiments, the oven is heated in the curing process to a temperature of 120°C.

According to some exemplary embodiments, pressing the glue during the curing process allows, for example, gluing of the flex to the PZT upper silver electrode with a very thin, for example a thickness of 15µm-30µm (~20um), for example to prevent ultrasonic energy losses in the glue layer. Optionally, the EpoTEK ND353 glue mechanically fixes and supports the entire structure of silver strips and PZT elements, which are supported weekly on the film of soft conductive glue containing particles.

According to some exemplary embodiments, for example as shown in figs. 13C and 13D, the electrical wiring connected to the ultrasound transducers and/or comprising the thermistors, for example electrical wiring of the flex-PCB 1302 are optionally non-passivated, for example to keep a distance between an emitting surface of each ultrasound transducer and a skin surface, short as possible, for example to increase the efficiency of ultrasound energy delivery to the skin. In some embodiments, portions of the electrical wiring located between the ultrasound transducers and an emitting surface of the ultrasound applicator configured to contact the skin are not passivated, while other portions of the electrical circuitry, for example portion 1321 located away from the emitting surface of the ultrasound applicator are passivated. Optionally, all electrical conductors or wiring positioned between the ultrasound transducers and an emitting surface of an ultrasound transducers are not passivated. In some embodiments, all of the electrical circuitry or conductors electrifying the ultrasound transducers are not passivated.

### Exemplary positioning of PZT elements on holder

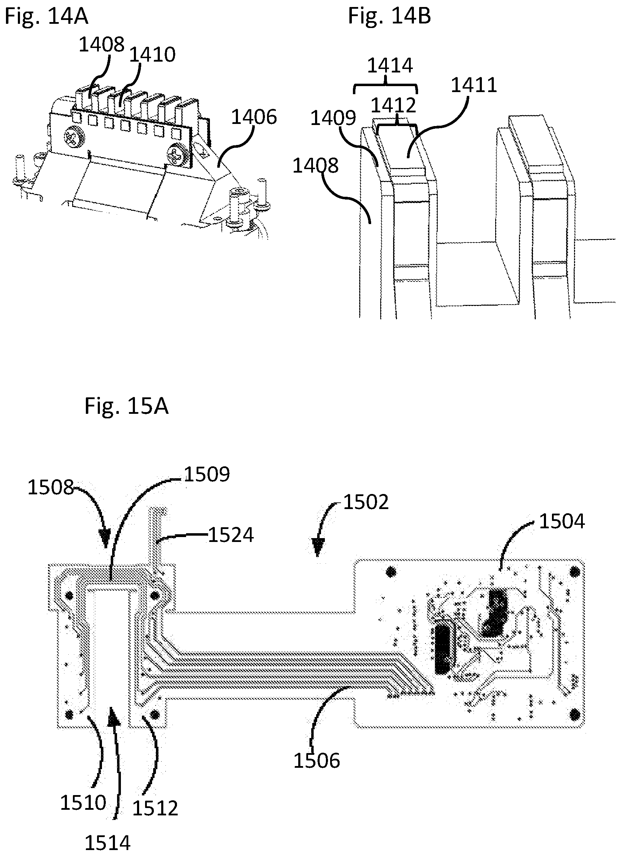

According to some exemplary embodiments, each PZT element is positioned on a surface of a heat conducting holder, for example to allow heat transfer from the PZT element to the holder. Reference is now made to figs. 14A and 14B depicting PZT holder assembly, according to some exemplary embodiments of the invention.

According to some exemplary embodiments, a heat-conducting holder 1406 comprises two or more upwardly extending protrusions, for example protrusions 1408 and 1410, optionally shaped as fingers. In some embodiments, an upper surface of each protrusion is planar and is shape and sized to be attached to a PZT element. In some embodiments, for example as show in fig. 14B, a size, for example a width 1414 of an upper surface 1409 of a protrusion 1408 is larger than a size, for example a width 1412 of a PZT element 1411. In some embodiments, a width 1414 is larger in at least 0.1mm from a width 1412 of the PZT element. For example, a PZT width is about 1mm, a width 1414 of a surface of the holder on which the PZT is positioned is about 1.3mm-1.7mm, for example 1.3mm-1.5mm, 1.4mm-1.7mm or any intermediate, smaller or larger range of values.

Potential advantages of wide holder surfaces for mounting PZT elements may be to allow glue, for example ND353 glue, leftovers to accumulate on the sides of the PZT elements, for example to provide mechanical support and adhesion of the PZT to the holder surface from the PZT sides, to allow sealing of the PZT from Parylene penetration between the PZT electrode and the glue, and to allow small errors in alignment of the PZT position on the holder surface.

### Exemplary transducers flex-PCB

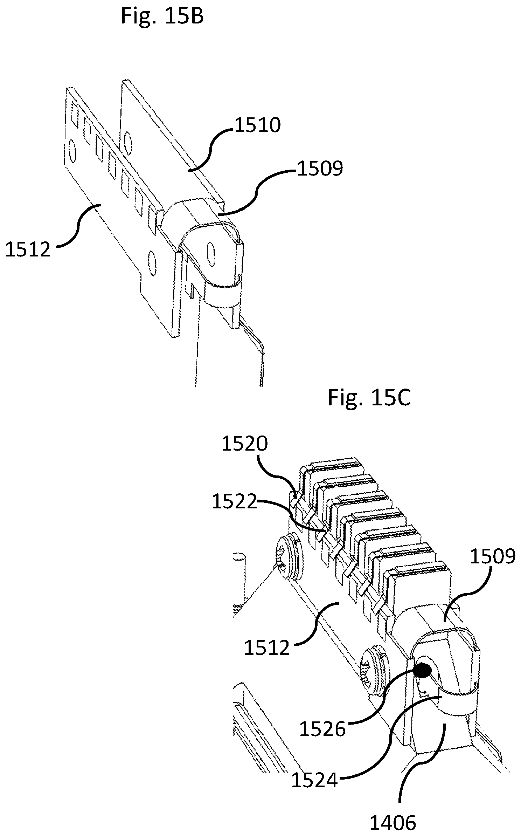

Reference is now made to figs. 15A and 15B, depicting a flex-PCB of an applicator, configured to deliver electric current to ultrasound transducers, according to some exemplary embodiments of the invention.

According to some exemplary embodiments, the ultrasound transducers are electrified by electrical wiring or electrical conductors attached to an electrode on a surface of each ultrasound transducer facing an emitting surface of the ultrasound applicator. Alternatively, the ultrasound transducers are electrified by electrical wiring or electrical conductors that penetrate through and/or contact a base on which the ultrasound transducers are positioned, for example holder 1406.

According to some exemplary embodiments, a transducers flex-PCB, for example flex-PCB 1502 comprising a rigid electrical board 1504 and a transducers interacting section 1508, electrically connected by an electric conducting flexible region, for example flex 1506. In some embodiments, the transducers interacting section 1508, comprises two electrically connected rigid boards 1510 and 1512, for example rigid PCBs. In some embodiments, the two rigid boards 1510 and 1512, for example rigid PCBs are spaced apart. In some embodiments, the two rigid boards 1510 and 1512 are separated at least partly by a void 1514. In some embodiments, the two rigid boards 1510 and 1512 are shaped and sized to be positioned on both sides, for example on the two most elongated sides of a transducers holder, while a holder portion carrying the transducers, for example protrusions 1408 and 1410 shown in fig. 14A, penetrate through the void 1514. In some embodiments, the two rigid boards 1510 and 1512 are shaped and sized to be positioned on both sides, for example on the two most elongated sides of a transducers array.

According to some exemplary embodiments, the flex-PCB is a rigid-flex-rigid-flex-rigid-flex PCB conformation, with optionally 50Ohm matching in all parts. In some embodiments, the flexibility of the transducers interacting section 1508, and the independent movement of each of the transducers boards 1510 and 1512 allows, for example bending of the PCB structure to fit on the holder structure, allows for example an easy assembly process, and optionally to transfer radiofrequency (RF) signals in a flex portion.

According to some exemplary embodiments, the flex 1506 comprises all the RF power channels, which are shielded to the ground on both sides, for example to allow low Electromagnetic compatibility (EMC), low disturbance or changes in channels capacitance and impedances, and no need for connectors and/or coax wires soldering.

According to some exemplary embodiments, for example as shown in fig. 15B, the two boards of the transducers interacting portion 1508, for example transducers boards 1510 and 1512 are electrically connected by a flex strip 1509. In some embodiments, the flex strip 1509 allows bending of the transducers board in at least 180° degrees relative to each other, for example to allow positioning of the transducers boards on both sides of a transducers holder.

According to some exemplary embodiments, for example as shown in fig. 15C, soldering pads of the applicator flex- PCB are positioned on bars, for example bars 1520 and 1522 in each side of a PZT. In some embodiments, placing the soldering footpad on bars may allow, for example, a shorter distance to a bottom copper strip and/or to an upper silver strip from the PZT to the pads that may lead to easier assembly, less EMC, and/or less parasitic capacitance and/or impedance variations.

According to some exemplary embodiments, a short flex extension 1524 extends from one of the transducers boards, for example transducers boards 1512 and 1510, and comprises a temperature sensor, for example a thermistor 1526 for recording the temperature of the holder 1406.

According to some exemplary embodiments, a RF phase bar of the applicator PCB includes a short extension flex, for example flex 1524 on which the holder temperature thermistor is positioned. In some embodiments, the flex 1524 tip and the thermistor 1526 are positioned in a hole in the holder side, and are optionally glued to the holder 1406 with a heat conducting glue. Using a separate thermistor for recording temperature of a holder and using a glue to attach the thermistor to the holder allows, for example simple assembly of the energizer for temperature measurement of the holder temperature, fast planar assembly of the applicator PCB and holder thermistor, with no need for manual soldering, transferring the thermistor reading directly to the electric board 1504 of the PCB through the flex 1506.

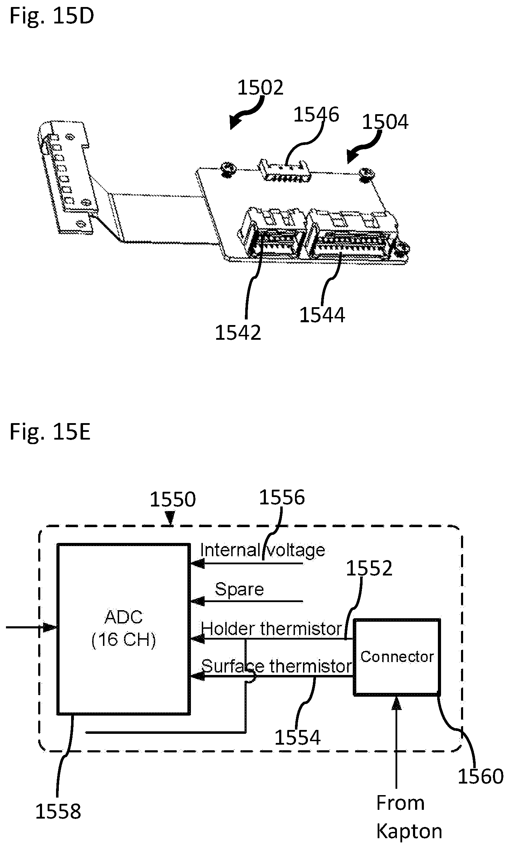

According to some exemplary embodiments, for example as shown in fig. 15D, an electric rigid board 1504 of the main flex-PCB 1502 of the applicator, comprises at least one connector, for example push and lock connectors 1542, 1544 and 1546. In some embodiments, the connectors comprise one or more RF connector, communication connector, a switch and/or a LED. In some embodiments, the connectors on the board may allow simple assembly of connection to the applicator umbilical connecting channel, electric connection of the user interface of the applicator, for example the switch 820 and/or LED 822 shown for example in fig. 9A, and simple connection of the flex electric circuitry for thermistor reading.

According to some exemplary embodiments, for example as shown in fig. 15E, an electric board of the applicator, for example the electric rigid board 1504 of the main flex-PCB 1502 comprises a measurement circuit, an A2D sampling and a SPI communication unit, for example to allow transmission of the temperature and readings from the user interface of the applicator to the control console. The measurement circuit, the A2D sampling and the SPI communication unit may allow minimal or no effects of EMC noises or capacitance errors on the transmitted readings accuracy to the console, eliminate a need for multiple wires for analog readings, thus decreasing EMC effects, and may allow integration of multiple additional signals from different sources.

According to some exemplary embodiments, for example as shown in fig. 15E, an electric board within the applicator comprises a measurements receiving circuitry, for example circuitry 1550. In some embodiments, the circuitry 1550 comprises an A2D converter 1558, configured to receive holder thermistor signals 1552, received from at least one heat sensor configured to record temperature levels of a heat conductor contacting the transducers, for example ultrasound transducers, of the applicator. In some embodiments, the holder thermistor signals indicate a temperature of the transducers. Additionally or alternatively, the converter 1558, is configured to receive surface thermistors signals 1554, received from at least one heat sensor configured to record temperature levels of a skin region contacting the applicator. In some embodiments, the holder thermistor signals 1552 and the surface thermistors signals 1554 are received from a connector 1560 on the electric board, that is optionally connected to at least one PCB comprising two or more thermistors, for example a flexible-PCB. In some embodiments, the A2D converter reading is transferred on the PCB to a communication circuitry, for example a SPI communication chip, from which the information of the temperature is transmitted to the system through the umbilical.

### Exemplary applicator assembly

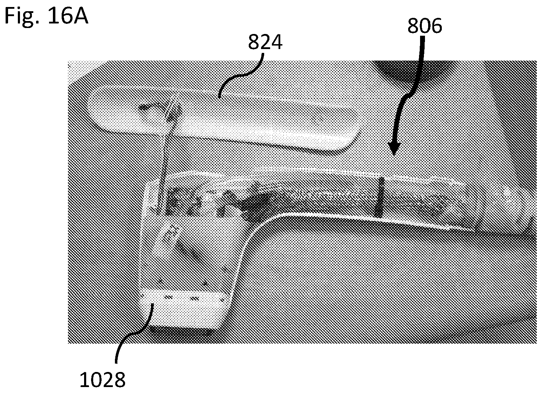

According to some exemplary embodiments, during the assembly of the applicator, some of the gaps within different components of the applicator are sealed, for example using a filling material, for example to prevent interaction between electrical components and components of the cooling system. In addition, at least some of the gaps within the applicator are filled with adhesive, for example to minimize movement of the components, and to isolate some of the components, for example tubes of the cooling system from liquids and/or humid air to prevent water condensation on the cooling system tubes. Reference is now made to fig. 16A depicting assembly of the applicator components, according to some exemplary embodiments of the invention.

According to some exemplary embodiments, an energizer assembly following Parylene-C coating is covered with an external applicator cover and a gasket. In some embodiments, a silicone filler, for example a non-acidic RTV silicone is positioned around and inside an air gap between an external cover and the heat exchanger, in order to seal it to water and air penetration. In some embodiments, sealing the gap between the cover and the heat exchanger may allow one or more of the following, prevention of water condensation and/or ice formation on the energizer holder caused by humid air or condensate water on the water tubes, preventing water or other liquids penetrating into the frame space and near the PZT elements which can generate changes in the PZT mechanical support or stress, electric impedance changes, and/or changes in sensitivity of thermistor measurements.

According to some exemplary embodiments, the electric card of the main PCB is secured with screws into a first applicator cover. In some embodiments, the energizer is positioned inside pre-designed pins of the cover that are shaped and sized to penetrate into complementary openings in an opposite and complementary applicator cover. In some embodiments, securing the electric card of the PCB may allow one or both of the following, to avoid damage due to stress on the flex parts of the main flex-PCB, for example the transducers flex-PCB, and to provide mechanical support against pulling forces applied on electrical wiring within the umbilical connecting channel.

According to some exemplary embodiments, for example as shown in fig. 16A, a filling material, for example soft silicone, is added to the inner lumen of the applicator to fill at least part of the inner lumen. In some embodiments, the electric connector of the top cover switch and LED is connected to the PCB rigid card. In some embodiments, an umbilical connecting channel insert is aligned relative to the applicator body correctly, and a first applicator casing is closed and glued, for example with a glue. In some embodiments, the applicator body is tilted in an angle smaller than 90⁰ degrees, for example an angle of 45° degrees and then soft silicon filling material is purred into the applicator body to fill the lumen of the applicator body up to an opening for the upper panel comprising the user interface of the applicator. Optionally, localized filling with filling material of the main flex-PCB of the applicator is performed, for example to reduce weight of the applicator body.

Filling the lumen of the applicator body at least partly with silicone or any other filling material that can be solidified may allow one or more of the following, prevention of water condensation on the water tubes due to penetration of humid air into the applicator body, prevention of water dripping and penetrating into the electric connectors of the rigid board of the flex-PCB and/or TEC connectors, electric isolation of the surrounding components, prevention of water dripping from the applicator head in case of condensation, and mechanical stabilization of the applicator body.

### Exemplary applicator assembly and parts

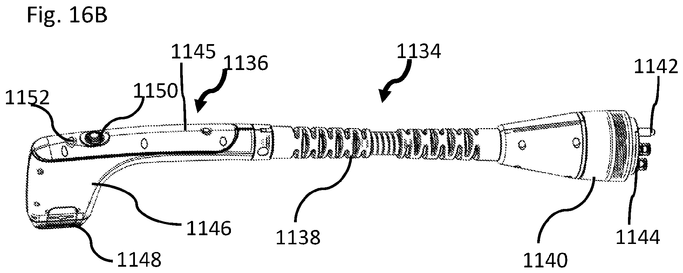

Reference is now made to fig. 16B depicting an applicator assembly, including an array of ultrasound transduces suitable for use in the described skin treatment method, according to some exemplary embodiments of the invention.

According to some exemplary embodiments, applicator assembly, for example applicator 1134 comprises an applicator body 1136 and an umbilical connecting channel 1138. In some embodiments, the umbilical connecting channel 1138 couples applicator body 1136 to a distal end of the umbilical connecting channel 1138. In some embodiments, the proximal end of umbilical connecting channel 1138 comprises at least one console connector 1140, configured to connect applicator 1134 to a control console (not shown) of an ultrasound system. In some embodiments, the console connector 1140 includes at least one electrical grounding pin 1142, configured to allow grounding of ultrasound applicator 1134. Additionally, the at least one console connector 1140 of the umbilical connecting channel 1138 includes at least one coolant liquid connector 1144, configured to connect a cooling system of ultrasound applicator 1136 to a cooling system of the console, for example to allow circulation of coolant liquid between applicator 1136 and the console.

According to some exemplary embodiments, applicator body 1136 comprises an elongated handle 1145, having at least one gripping member, shaped and sized to be held by a hand of an operator. In some embodiments, ultrasound applicator 1136 comprises an emitting head portion 1146 oriented in an angle between 70 degrees to 180 degrees, or any intermediate, smaller or larger range of angle values, relative to handle 1145. A potential advantage of positioning an emitting head portion in an angle relative to a handle of the applicator may be to allow easy directing of the emitting head portion to a treatment area in a subject, and/or to allow easier application of force by an operator holding applicator 1136 against the treatment area.

According to some exemplary embodiments, handle 1145 of applicator 1134 comprises a user interface that includes at least one activation switch 1150, and/or at least one visual indicator such as for example, a LED 1152. In some embodiments, the applicator 1134 comprises a sound indicator configured to generate at least one human detectable indication to an operator holding the handle 1145. In some embodiments, the user interface is oriented on handle 1145 in a distance from the at least one gripping member of the handle 1145 selected to allow holding and activating the user interface with the same hand of an operator. In some embodiments, the user interface is positioned on handle 1145 within a field of view of an operator holding the applicator 1136.

Reference is now made to fig. 16D, which is a cross section through head portion 1146 of applicator 1136 shown in fig. 16C, according to some exemplary embodiments of the invention.

According to some exemplary embodiments, head portion 1146 comprises a plurality of spaced-apart ultrasound transducers 1150, for example, two or more ultrasound transducers 1150. In some embodiments, the transducers 1150 are positioned near a surface of head portion 1146, which is shaped and sized to be placed in contact with the skin. In some embodiments, ultrasound transducers 1150 are configured to emit non-converging ultrasound waves. In some embodiments, the transducers 1150 are linearly organized in the form of an array underneath and near the skin contacting surface 1148 of the head portion 1146 shown in fig. 16B. In some embodiments, the ultrasound transducers 1150 deliver the generated ultrasound energy to the surface of the skin and deeper tissue layers.

According to some exemplary embodiments, the head portion 1146 comprise a frame or cover 1152, which covers, and optionally incorporates a heat-conducting holder 1154, ultrasound transducers 1150 and a thermoelectric cooler 1156.

According to some exemplary embodiments, each ultrasound transducer 1150, that includes for example a PZT element, is positioned on a surface of the heat-conducting holder 1154 to allow, for example, heat transfer from the transducer 1150 to holder 1154. In some embodiments, the heat-conducting holder 1154 comprises two or more protrusions, for example upwardly extending protrusions 1158, optionally shaped as fingers. In some embodiments, the upper surface of protrusions 1158 is planar and optionally is shaped and sized to be attached to a surface of a transducer 1150, for example to a planar surface of a PZT element of the transducer.

In some embodiments, an upper surface of each protrusion 1158 is planar and is configured to be in contact with a temperature sensor for example a thermistor.

According to some exemplary embodiments, a width 1160 of an upper surface of a protrusion 1158 is larger than a size, for example a width 1162 of a transducer 1158, for example a PZT element of the transducer 1158. In some embodiments, the width 1160 is larger in at least 0.1mm from the width 1162 of the transducer 1150. For example, a transducer 1150 width 1162 is about 1mm, a width 1160 of a surface of the holder on which the transducer 1150 is positioned is in a range of about 1.3mm-1.7mm, for example, 1.3mm-1.5mm, 1.4mm-1.7mm or any intermediate, smaller or larger range of values. In some embodiments, a cross-section of protrusions 1158 is selected to support the optimal heat exchange process between transducer 1150, for example a PZT element of transducer 1150, and thermoelectric cooler (TEC) 1156.

According to some exemplary embodiments, a surface of heat conducting holder 1154 is placed in contact with a cold surface of a thermoelectric cooler (TEC) 1156. In some embodiments, the heat conducting holder 1154 is configured to transfer heat from one or more of the transducers 1150 to a cold surface of TEC 1156. Optionally, the heat conducting holder 1154 is configured to cool the surface of a transducer 1150 that is in contact with the skin or is positioned close to the skin, for example when a layer of an isolating material is placed between the transducer and the skin. In some embodiments, the heat conducting holder 1154 comprises or, is at least partly made from a heat-conducting material, for example, aluminum.

Potential advantages of wide heat-conducting holder 1154 surfaces for mounting the transducers 1150 may be to allow glue, for example, ND353 glue, leftovers to accumulate on the sides of the transducers, for example on the sides of PZT elements and to provide mechanical support and adhesion of each of the transducers 1150 to heat-conducting holder 1154 surface from the transducer side. In some embodiments, the mounting of the transducers 1150 on top a wide surface of the holder protrusions allows, for example, sealing of the PZT element from possible Parylene penetration in a gap between the PZT element and the glue. Additionally or alternatively, mounting of the transducers on a wide surface of the holder protrusions allows, for example, small errors in alignment of a transducer 1150, for example the PZT element of the transducer position on a planar upper surface of each protrusion 1158.

### Exemplary umbilical connecting channel

According to some exemplary embodiments, an umbilical connecting channel is an elongated and flexible channel shaped and sized to connect an applicator body to a control console. In some embodiments, the connecting channel is irreversible coupled to an applicator body, for example to prevent disconnection of the applicator body from the connecting channel by an operator of the system. In some embodiments, electrical wiring and at least one tube of the cooling system pass from the applicator body through the connecting channel to console connectors located at a proximal end of the connecting channel.

According to some exemplary embodiments, both ends of the connecting channel, for example an end of the connecting channel connected to the applicator body, and an end of the connecting channel comprising the console connectors, are sealed in the spaces between the cables and the inserts, for example with a non-acidic RTV, to prevent penetration of humid air into the connecting channel. Air sealing of the connecting channel may allow one or more of minimizing or eliminating condensation of water on the water tubes from the air in the umbilical connecting channel, minimizing or eliminating water drip into the applicator space from the lumen of the umbilical connecting channel in case of condensation on the coolant fluid tubes, mechanical support of cables inside the connecting channel for prevention of twisting.

According to some exemplary embodiments, at least some or all of the electric cables within the umbilical connecting channel are electrically shielded separately. In some embodiments, an additional electrical shielding surrounds a bundle of all the cables, and is optionally electrically connected to the shielding of the cables. In some embodiments, the cables bundle shielding is electrically connected to the console chassis, and not to the RF or signal electric ground. The electrical shielding of the cables may allow an EMC shielding which is sufficient to pass regulatory requirements.

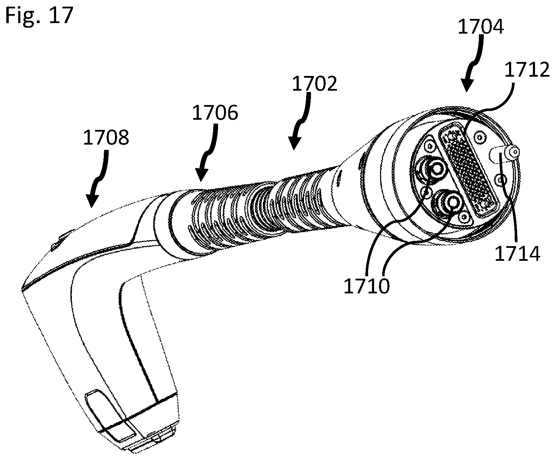

Reference is now made to fig. 17, depicting console connectors of an umbilical connecting channel, according to some exemplary embodiments of the invention.

According to some exemplary embodiments, an umbilical connecting channel 1702 is an elongated connecting channel having a distal end 1706 connected to an applicator body 1708, and a proximal end 1704 comprising two or more connecting channel connectors, for example electrical and/or coolant fluid connectors to a control console of an ultrasound system for delivery of skin treatments.

According to some exemplary embodiments, the connecting channel connectors comprise an electrical connector, for example pin 1714, configured to electrically connect the shielding of the electric cables in the umbilical connecting channel 1702 to the control console, for example to a chassis of the control console. In some embodiments, the pin 1714 is used for alignment of the console connectors to matching connectors in the control console.

According to some exemplary embodiments, the connecting channel connectors comprises at least one coolant fluid connector, for example connectors 1710, configured to connect the cooling system of the applicator to the cooling system of the console.

According to some exemplary embodiments, the electric connector of the umbilical connecting channel to the control console comprises a 48 pin connector 1712 that match a 48 socket connector in the control console for delivery of US power from the control console to the transducers in the applicator body 1708. In some embodiments, the use of a 48 pin connector may allow one or more of the following, low costs due to usage of standard parts, simple assembly with no need for soldering, and a simple applicator assembly by clicking the connectors into the sockets in the applicator main PCB.

According to some exemplary embodiments, the connecting channel connectors of the connecting channel are filled with a solidified filling material, for example silicon, to cover uninsulated portions and wires of the 48 pin connector. In some embodiments, adding a solidified filling material to the connectors may allow electric isolation between the pins, for example in case of water penetration or condensation on the coolant fluid tubes, and prevention of corrosion.

### Exemplary control console

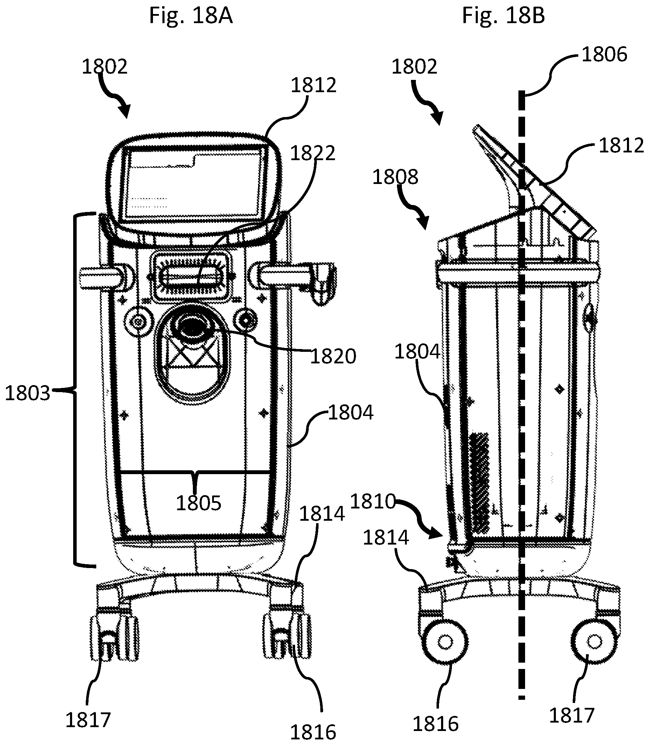

Reference is now made to figs. 18A and 18B depicting a control console of an ultrasound system for delivery of skin treatments, according to some exemplary embodiments of the invention.

According to some exemplary embodiments, a control console, for example control console 1802 comprises a tower shaped body, having a vertical axis 1806 an upper end 1818 and a lower end 1810. In some embodiments, a ratio between a height 1803 and a width 1805 of the body is at least 1.5:1, for example 2:1, 3:1 or any intermediate, smaller or larger ratio. In some embodiments, the console 1802 is a mobile, for example a movable console, configured to move in an upright orientation on a surface, for example a floor.

According to some exemplary embodiments, an upper end 1808 of the body 1804 comprises a user interface, for example a display 1812. In some embodiments, the display is tilted in an angle in a range of 30° degrees-90° degrees, for example 30⁰ degrees-45° degrees, 40° degrees-50° degrees, 45° degrees-65° degrees or any intermediate, smaller or larger range of angles, relative to the vertical axis 1806.

According to some exemplary embodiments, the lower end 1810 of the body 1804 is coupled to a wheel base 1814. In some embodiments, at least two wheels, for example wheels 1816 and 1817 are connected to a surface of the wheel base facing a floor. In some embodiments, the at least two wheels are configured to allow movement of the console 1802 on a surface, for example a floor.

According to some exemplary embodiments, the console 1802 comprises an umbilical connecting channel port, for example port 1820, configured to allow connection of the connecting channel to the console 1802. In some embodiments, the console 1802 comprises at least one handle, for example handle 1822, including at least one gripping member shaped and sized to fit inside a palm of an operator's hand, for example to allow manual movement of the console 1802 on the floor.

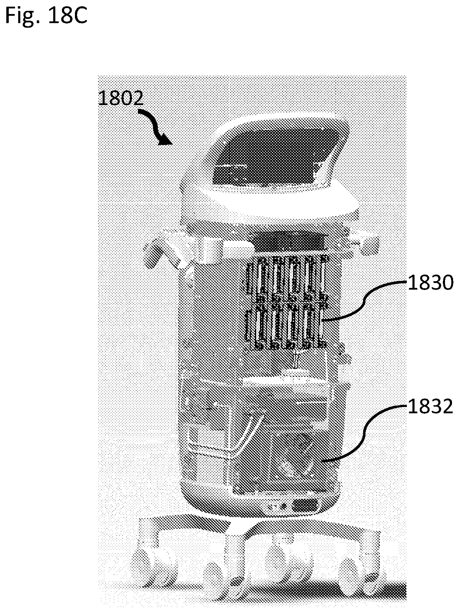

According to some exemplary embodiments, for example as shown in fig. 18C, the control console 1802 comprises two or more ultrasound (US) power amplification cards, for example power cards 1830 arranged within a supporting frame, for example a chassis of the console. Additionally. The console comprises a cooling system, for example a cooling system 1832 located at a lower position within the chassis, close to the lower end of the body, for example lower a center of mass of the console body.

### Exemplary ultrasound (US) power signal amplification and measurement

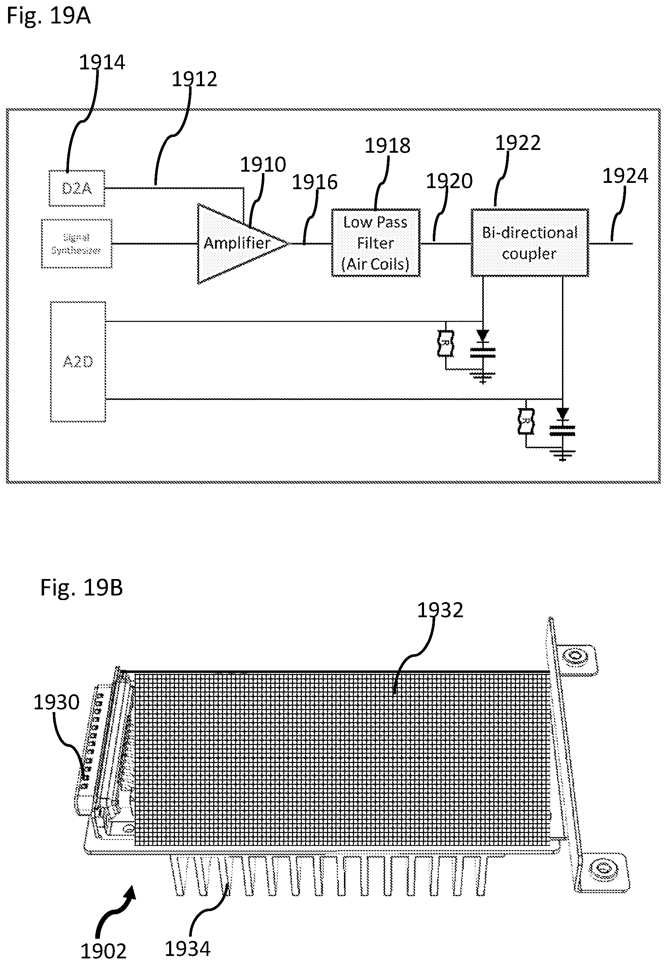

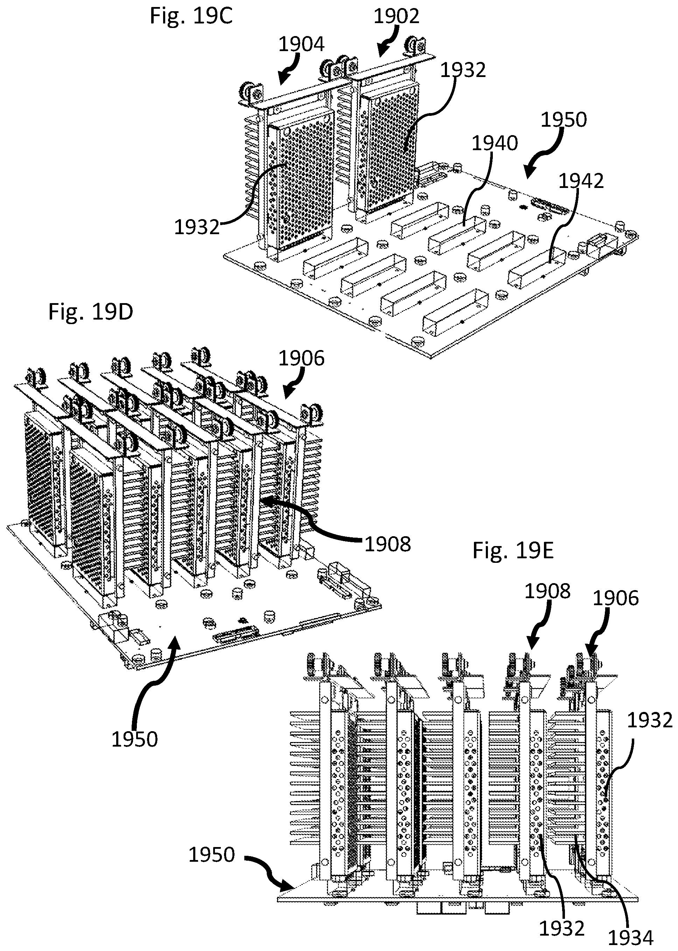

Reference is now made to fig. 19A, depicting US power signal amplification and measurement, according to some exemplary embodiments of the invention.

According to some exemplary embodiments, an US PCB power card is electrically connected to a backplane PCB 1904 in the console. In some embodiments, the backplane PCB is electrically connected to the main PCB 1906 of the console.

According to some exemplary embodiments, each US transducer receives an electric power signal from a separate power channel. In some embodiments, each power channel is generated by a separate US card, for example US card 1902 located in the control console. In some embodiments, the US card comprises an amplifier, for example amplifier 1910 that generates US electric excitation according to a determined electric power output.

According to some exemplary embodiments, each US card, for example US card 1902 receives a control signal 1912, generated by a specific D2A channel or a D2A converter 1914. In some embodiments, the control signal 1912 is set by a control software of the console.

According to some exemplary embodiments, the amplifier 1910, for example a non-linear amplifier, generates a distorted sinus signal 1916. In some embodiments, the distorted sinus signal 1916 is fed into a low-pass filter 1918, before the electrical power output is measured by the bi-directional coupler 1920. In some embodiments, the low-pass filter is used to convert the distorted sinus signal 1916 into a sinus signal that can be measured by the bi-direction coupler, for example to provide accurate measurements results.

According to some exemplary embodiments, the low-pass filter comprises at least one air coil, for example 2, 4, 6 or any intermediate, smaller or larger number of air coils. In some embodiments, each of the air coils is used in low RF frequency, for example in a RF frequency of up to 100MHz, for example up to 80MHz, up to 50MHz or any intermediate, smaller or larger value, and an amplitude, for example power of at least 1 Watts, for example, 1.5 Watts, 3 Watts, 4.5 Watts, 5 Watts or any intermediate, smaller or larger number of Watts.

According to some exemplary embodiments, each of the air coils is formed by rolling an electrical conducive wire, for example a copper wire, having a diameter of 1mm-2mm, on a cylinder having a diameter in a range of 5mm-15mm, for example about 10mm. Such air coils are currently not available in the market as a standard product, and therefore were custom made by rolling a copper wire with diameter of 1-2mm on a cylinder with diameter of 10mm. The number of rounds we adjusted in order to get the required inductance values.

According to some exemplary embodiments, the low pass filter 1918 is configured to removes all the frequencies, higher than a main frequency, to generate a sinus waveform output signal 1920 of the main frequency.
In some embodiments, the output signal passes through a bi-directional coupler 1922, and exits as a power output signal 1924.

According to some exemplary embodiments, table A below describes the different signals in the US power card:

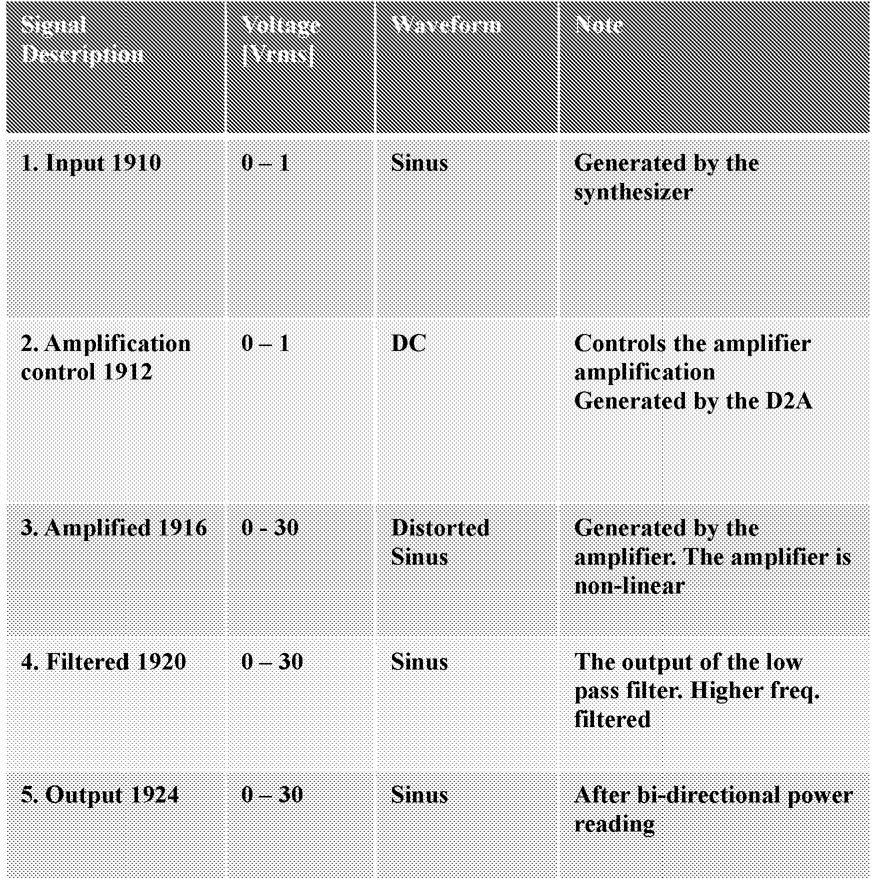

According to some exemplary embodiments, the measurement output signals of the bi-directional coupler are 1/100 of the forward and reverse electric power. **In** some embodiments, the measurement output signals comprise two or more signals with 1/100 of the original signals. **In** some embodiments, in order to calculate the forward and reverse (reflected) power, there is a need to sample the signals and calculate the electric power. **In** some embodiments, since the signal 1920 generated by the low pass filter 1918 is a sinus signal, the amplitude of the output signal is measured.

According to some exemplary embodiments, during the measurement of the output signal, a forward and reverse signals are connected each to a capacitor charging circuited, used as a pick-detector, where the voltage level on the capacitor is linearly dependent on the amplitude of signal measured by the bi-directional coupler. In some embodiments, the voltage on the capacitor is proportional to the amplitude of the sinus signal. In some embodiments, each signal is sampled at a low frequency in a range of 10Hz-100Hz, for example 10Hz-30Hz, 20Hz-50Hz, 40Hz-70Hz, 60Hz-90Hz, 50Hz-100Hz, or any intermediate, smaller or a larger range of values. In some embodiments, the voltage on the capacitor of each channel is measured by an A2D, and read by the control software of the console. Optionally, initialization calibration protocols may allow to calculate a measured power output from a measured voltage.

According to some exemplary embodiments, the amplifier 1910 is configured to generate a high frequency radiofrequency (RF) signal, for example a RF signal having a frequency in a range of 5-20 MHz, for example 5-10 MHz, 8-15 MHz, 10-20 MHz or any intermediate, smaller or larger range of frequencies. In some embodiments, a sinus signal is generated from the high frequency RF signal, for example using a low pass filter, for example low pass filter 1918 optionally comprising air coils.

According to some exemplary embodiments, a fraction of the sinus signal is sampled, for example a fraction of 1/5, 1/10, 1/100 or any intermediate, smaller or larger fraction of the signal is sampled. In some embodiments, the signal fraction is delivered to a capacitor, for example a chargeable capacitor. In some embodiments, an RMS voltage of the sampled sinus signal is calculated. In some embodiments, the calculated RMS voltage values are monitored. Alternatively or additionally, variability in the calculated RMS voltage values is monitored. In some embodiments, an amplification, for example values of at least one parameter of the amplification is modified according to the results of the monitoring, for example the monitoring of the calculated RMS voltage values and/or the monitoring of the variability of RMS voltage values.

According to some exemplary embodiments, an indication related to the amplitude of the sinus signal is measured, for example a square of an amplitude value or any other mathematical variation of the amplitude, is measured. In some embodiments, an acoustic output of ultrasound transducers receiving the high frequency RF signal, for example the high frequency RF sinus signal, is determined based on the measured amplitude indication, for example based on the measured square of the amplitude. In some embodiments, there is a linear relation between the acoustic output and the measured amplitude indication, for example the square of the amplitude.

A potential advantage of using the capacitor charging voltage measurement may be the ability to use a standard multi-channel A2D component, and sample the signal at 10-100 Hz, instead of sampling 14 signals, in case that 7 power signals exist, at 100MHz, or to use a multiplexer. The problem solved by the described configuration is that in order to use the bi-directional coupler to measure the forward and reverse power levels, there is a need to sample the measured signal of the bi-directional coupler and calculate the signal's RMS value Vrms, from which the power is calculated by V^2/R where R = 50 Ohm of the coupler. The measured signal from the coupler is in the power signal frequency of 10-12 MHz. In order to calculate the Vrms of such a signal, there is a need to sample it in at least 100 MHz, where for each power channel there are two such signals: forward and reverse. If there are 7 power channels, in this example, there is a need to sample 14 signals at 100MHz, or to use relevant multiplexer, which yields an expensive and complex system for both options. But, by using the capacitor charging voltage measurement, it is possible to use a very simple and cheap standard multi-channel A2D component, and sample the signal at 10-100 Hz.

### Exemplary US amplification cards

Reference is now made to figs. 19B-19D depicting ultrasound (US) amplifier cards and arrangement of the US amplifier cards side-by-side within the control console, according to some exemplary embodiments of the invention. In some embodiments, a US amplifier card comprises a radiofrequency (RF) signal amplifier card.

According to some exemplary embodiments, a US amplifier card, for example card 1902 comprises a filter, for example a low-pass filter is formed from at least one, for example 2,4,6 or any intermediate, smaller or larger number of air coils. In some embodiments, the air coils are used to generate an output sinus signal. In some embodiments, the output sinus signal may allow one or more of the following, correct power measurement, high efficiency of energy conversion to US power, and electric power measurement using a capacitor charging, which allows, for example, measurements in a low sampling rate.

According to some exemplary embodiments, forward and reverse power measurements are performed in the US amplifier card 1902. Additionally or alternatively, impedance measurement are performed in the US amplifier card 1902.

According to some exemplary embodiments, for example as shown in figs. 19B-19D, each US amplifier card, for example card 1902, comprises at least one integral connector, for example integral connector 1930. In some embodiments, for example as shown in fig. 19B, each US amplifier card, for example cards 1902 and 1904 are connected to an electric board 1950, for example a backplane PCB board, within the console, for example by inserting the integral connector of a US amplifier card into an integral socket connector, for example socket connectors 1940 and 1942 of the electric board 1950.

According to some exemplary embodiments, placing two or more US amplifier boards next to each other, for example US amplifier cards 1906 and 1908, on the electric board 1950 can cause electromagnetic interference to measurement circuitries on the boards. In some embodiments, in order to reduce or eliminate electromagnetic interference between closely packed US amplifier boards, each board is covered at least partly, by at least one electromagnetic shield, for example shield 1932, in the form of a grid or a net. In some embodiments, the at least one shield covers the amplifying circuitry and/or the measurement circuitry of the US amplifier card. Alternatively or additionally, the at least one shield covers at least 50%, for example at least 60%, at least 80%, at least 90% or any intermediate, smaller or larger percentage of the external surface of the US amplifier card.

According to some exemplary embodiments, the at least one electromagnetic shield comprises a heat sink connected to the US amplifier card, for example heat sink 1934.

According to some exemplary embodiments, for example as shown in fig. 19E, a minimal distance between electric circuitries, for example amplifying circuitries and/or measurement circuitries of two adjacent RF signal amplifier cards, for example cards 1906 and 1908 is at least 3 cm, for example at least 5cm, at least 10cm or any intermediate, smaller or larger distance. In some embodiments, a minimal distance between the electric circuitries is in a range of 1cm-12cm, for example 1-8cm, 5-10cm, 8-12cm or any intermediate, smaller or larger range of values.

### Exemplary console cooling system

Reference is now made to figs. 18C, depicting a cooling system of the control console, according to some exemplary embodiments of the invention.

According to some exemplary embodiments, for example as shown in fig. 18C, the control console comprises a cooling system 1832. In some embodiments, the cooling system 1832 comprises at least one pump, for example electrical pump and a coolant fluid reservoir. In some embodiments, the pump is configured to circulate the coolant fluid between the reservoir and the applicator, for example a heat exchanger of the applicator.

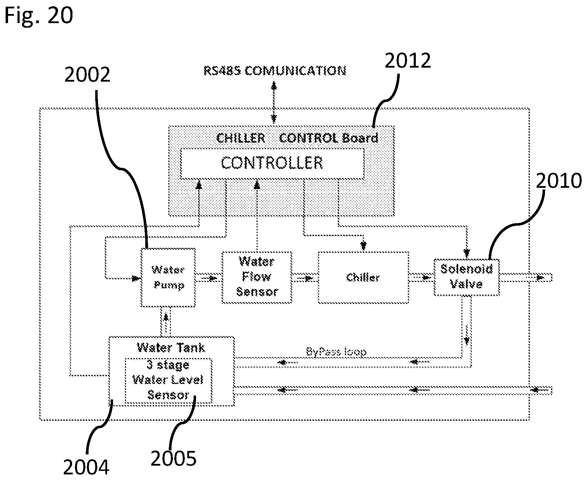

Reference is now made to fig. 20 depicting flow of coolant fluid, according to some exemplary embodiments of the invention.

According to some exemplary embodiments, a cooling system controller 2012 activates a pump, for example water pump 2002 to circulate coolant fluid, for example water between a reservoir, for example a water tank 2004 in the control console and an US applicator. In some embodiments, the water tank 2004 comprises at least one water level sensor 2005 electrically connected to the controller 2012, and configured to record water level in the water tank 2004.

According to some exemplary embodiments, controller 2012 signals the pump 2002 to move water to a chiller 2008, configured to cool the water. In some embodiments, at least one flow sensor, for example a water flow sensor 2006 controls the flow of water in the cooling system, for example into the chiller 2008. In some embodiments, the cooling system comprises at least one valve, for example a solenoid valve electrically connected to the controller 2012. In some embodiments, the controller signals the solenoid valve to open, for example to allow flow of water into the applicator.

### Exemplary umbilical connecting channel connector of the console

According to some exemplary embodiments, a console connector for the umbilical connecting channel of the applicator is configured to allow electrical connection between the console and the applicator. Additionally, the console connector is configured to allow coolant fluid connection between the cooling system of the console and the applicator. Optionally, the console connector is configured to allow electric connection for grounding at least one electrical signal from the applicator.

According to some exemplary embodiments, a console connector for an umbilical connecting channel comprises at least one electrical connector, for example a socket connector, matching a 48 pin electrical connector of the connecting channel, for example 48 pin connector 1712 shown in fig. 17.

According to some exemplary embodiments, the console connector comprises at least one coolant fluid connector that matches a coolant fluid connector of the connecting channel, for example connectors 1710 shown in fig. 17.

According to some exemplary embodiments, the console connector comprises a socket having an electrical conductive leaf spring, configured to ensure electrical conduction between the pin 1714 of the connecting channel connectors shown in fig. 17 and an electrical wiring of the console when the pin 1714 is introduced into a socket in the console connector.

### Exemplary visual interface to operator

According to some exemplary embodiments, an operator of the system, controls one or more parameters of the skin treatment, for example overall duration of the treatment, duration of a treatment session, type and number of treated regions and/or parameter values of the delivered ultrasound signals. In some embodiments, the operator adjusts one or more of the treatment parameters to a specific subject, for example based on physical and/or clinical characteristics of the subject, for example age, gender, skin type, wrinkle level, history of previous treatments, drug regime and/or sensitivity of the subject to pain. In some embodiments, the operator uses a user interface of the console, for example a display of the control console to insert information regarding the subject and/or the treatment. Alternatively or additionally, the operator receives indications regarding the status of the system and operation status of at least one system element, for example the applicator transducers.

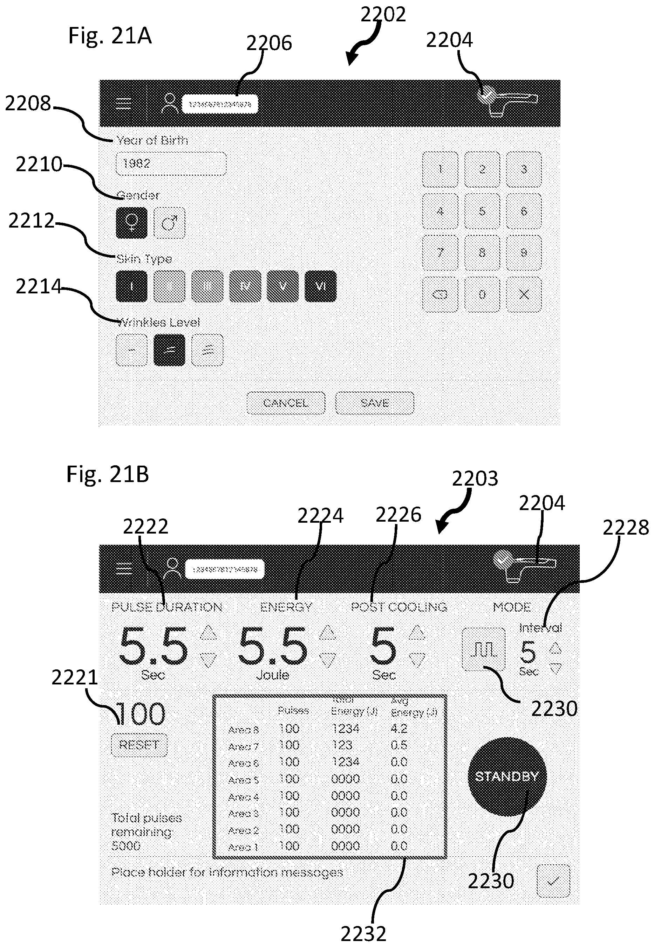

Reference is now made to figs. 21A and 21B depicting a visual interface presented to an operator of the system, according to some exemplary embodiments of the invention.

According to some exemplary embodiments, a subject detail interface 2202 is presented to an operator of the system. In some embodiments, the operator inserts details of a specific subject into a memory of the console, for example to generate a personal profile for the subject and/or to provide a personalized treatment. In some embodiments, subject-specific information inserted into a memory of the system is used to generate a database containing one or more of personal information on a subject, treatment history of the subject including history of treatment parameters, and results of the treatments. In some embodiments, the information in the database is used to develop new treatment protocols and/or to improve existing protocols. In some embodiments, the database is generated in a cloud storage and/or a remote computer.

According to some exemplary embodiments, in a subject detail interface, an operator inserts personal information of a subject, for example one or more of year of birth 2208, gender 2210, skin type 2212 and wrinkles level 2214. In some embodiments, a subject identifier 2206 for example a number is generated by the system for every subject, or is generated by an operator.

According to some exemplary embodiments, an operator adjusts one or more of the treatment parameter to a specific subject in a treatment parameter visual interface 2203. Alternatively, the interface presents parameter values of a stored treatment protocol selected automatically for a specific subject, for example based on the personal profile of the subject. Optionally, the interface presents parameter values of an existing stored protocol and the operator adjusts the values of the protocol parameters according to a personal profile of the subject.

According to some exemplary embodiments, a treatment parameter visual interface 2203 presents value of one or more parameters related to the ultrasound waves, for example number of pulses 2221 in a train of pulses of ultrasound waves, pulse duration 2222, energy level of each pulse, time duration for post cooling 2226. Additionally or alternatively the interface 2203 presents the type of treatment 2230, for example a single pulse or a train of pulses, and/or an interval 2228 between adjacent pulses.

According to some exemplary embodiments, the visual interface presents a treatment history 2232, including number or name of treated area, total energy delivered to a specific area, and/or energy level of each pulse delivered to the area.

According to some exemplary embodiments, the visual interface presents an indication, for example a graphical indication 2204 to describe a status or condition of the system and/or the applicator. Additionally or alternatively, the visual interface presents virtual buttons to the operator, for example a virtual button 2230, to allow changing of a system state between different programmed states, for example between On, Off, Stand-by, and Active states.

It is expected that during the life of a patent maturing from this application many relevant ultrasound transducers will be developed; the scope of the term ultrasound transducers is intended to include all such new technologies *a priori.*

As used herein with reference to quantity or value, the term "about" means "within ± 20 % of".

The terms "comprises", "comprising", "includes", "including", "has", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular forms "a", "an" and "the" include plural references unless the context clearly dictates otherwise. For example, the term "a compound" or "at least one compound" may include a plurality of compounds, including mixtures thereof.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. The invention is defined by the appended claims.

In addition, citation or identification of any reference in this application shall not be construed as an admission that such reference is available as prior art to the present invention. To the extent that section headings are used, they should not be construed as necessarily limiting.

## Claims

1. An applicator (304, 354, 804, 1002, 1134) of an ultrasound system (300), comprising:
an applicator body (806, 1004, 1136) having at least one ultrasound emitting surface shaped and sized to contact a skin,
at least one ultrasound transducer (244) comprising a surface opposite to said at least one ultrasound emitting surface and an electrode (1106) connected to said ultrasound transducer surface;
at least one electrical conductor (1110);
**characterized in that** said applicator further comprises a filler layer (1108) disposed between said electrode and said at least one electrical conductor, wherein said filler layer comprises a non-electrically conductive adhesive (1109) and a monolayer of electrically conducting rigid particles (1107) in said non-electrically conductive adhesive, wherein said electrically conducting rigid particles are disposed between and electrically connecting said electrode (1106) with said at least one electrical conductor (1110) and are configured to deliver electrical power between the at least one electrical conductor (1110) and the electrode (1106) of the at least one ultrasound transducer.

2. An applicator according to claim 1, wherein said rigid particles have a spherical or a round shape.

3. An applicator according to any one of claims 1 or 2, wherein a size or a diameter of said electrically conducting rigid particles varies in less than 5% from an average size or an average diameter of the electrically conducting rigid particles.

4. An applicator according to any one of the previous claims, wherein said electrically conducting rigid particles are shaped and sized to keep the at least one ultrasound transducer substantially parallel to said at least one electrical conductor.

5. An applicator according to any one of the previous claims, wherein said electrically conducting rigid particles are heat-conducting particles, configured to conduct heat away from said at least one ultrasound transducer through said filler layer.

6. An applicator according to any one of the previous claims, wherein said non-electrically conductive adhesive comprises a solidified adhesive material.

7. An applicator according to any one of the previous claims, wherein each of said electrically conducting rigid particles comprises a non-electrically conducting core surrounded by an electrically conducting layer.

8. An applicator according to claim 7, wherein said core comprises a non-electrically conducting polymer or mix of polymers.

9. An applicator according to any one of claims 7 or 8, wherein said electrically conducting layer surrounding said core comprises a metal material or a mix of metal materials comprising at least one of Copper, Silver, and Tungsten.

10. An applicator according to any one of claims 1 to 6, wherein at least 25% of said electrically conducting rigid particles comprises a rigid core surrounded at least partly with a soft layer which is softer relative to the rigid core.

11. An applicator according to claim 10, wherein said electrode is in contact with said soft layer.

12. An applicator according to any one of the previous claims, wherein at least 30% of a volume of said filler layer comprise said electrically conducting rigid particles.

13. An applicator according to any one of the previous claims, wherein said monolayer of electrically conducting rigid particles in said non-electrically conductive adhesive is formed by pressing the at least one ultrasound transducer into said non-electrically conductive adhesive towards the electrical conductor.

14. An applicator according to any one of the previous claims, wherein said at least one transducer comprises a piezoelectric element (1102) formed from a stiff material having a mechanical quality factor (Qm) larger than 600.

15. An applicator according to any one of claims 1 to 13, wherein said at least one ultrasound transducer comprises a piezoelectric element, and a polyimide printed circuit board (PCB) (1302) placed on top of said PZT element, and wherein a thickness of said polyimide is in a range between 10µm-35µm.

## Patentansprüche

1. Applikator (304, 354, 804, 1002, 1134) eines Ultraschallsystems (300), umfassend:
einen Applikatorkörper (806, 1004, 1136), der mindestens eine ultraschallemittierende Oberfläche aufweist, die für den Kontakt mit einer Haut geformt und bemessen ist,
mindestens einen Ultraschallwandler (244), der eine Oberfläche, die der mindestens einen Ultraschall emittierenden Oberfläche gegenüberliegt, und eine mit der Oberfläche des Ultraschallwandlers verbundene Elektrode (1106), umfasst;
mindestens einen elektrischen Leiter (1110);
**dadurch gekennzeichnet, dass** der Applikator ferner eine Füllschicht (1108) umfasst, die zwischen der Elektrode und dem mindestens einen elektrischen Leiter angeordnet ist, wobei die Füllschicht ein nicht elektrisch leitfähiges Haftmittel (1109) und eine Monoschicht aus elektrisch leitenden starren Partikeln (1107) in dem nicht elektrisch leitfähigen Haftmittel umfasst, wobei die elektrisch leitenden starren Partikel zwischen der Elektrode (1106) und dem mindestens einen elektrischen Leiter (1110) angeordnet sind und diese elektrisch miteinander verbinden und dazu konfiguriert sind, elektrische Energie zwischen dem mindestens einen elektrischen Leiter (1110) und der Elektrode (1106) des mindestens einen Ultraschallwandlers zu übertragen.

2. Applikator nach Anspruch 1, wobei die starren Partikel eine kugelförmige oder runde Form aufweisen.

3. Applikator nach einem der Ansprüche 1 oder 2, wobei eine Größe oder ein Durchmesser der elektrisch leitenden starren Partikel um weniger als 5 % von einer durchschnittlichen Größe oder einem durchschnittlichen Durchmesser der elektrisch leitenden starren Partikel abweicht.

4. Applikator nach einem der vorhergehenden Ansprüche, wobei die elektrisch leitenden starren Partikel so geformt und bemessen sind, dass der mindestens eine Ultraschallwandler im Wesentlichen parallel zu dem mindestens einen elektrischen Leiter gehalten wird.

5. Applikator nach einem der vorhergehenden Ansprüche, wobei die elektrisch leitenden starren Partikel wärmeleitende Partikel sind, die dazu konfiguriert sind, Wärme von dem mindestens einen Ultraschallwandler durch die Füllschicht abzuleiten.

6. Applikator nach einem der vorhergehenden Ansprüche, wobei das nicht elektrisch leitfähige Haftmittel ein verfestigtes Haftmittelmaterial umfasst.

7. Applikator nach einem der vorhergehenden Ansprüche, wobei jedes der elektrisch leitenden starren Partikel einen nicht elektrisch leitenden Kern umfasst, der von einer elektrisch leitenden Schicht umgeben ist.

8. Applikator nach Anspruch 7, wobei der Kern ein nicht elektrisch leitendes Polymer oder eine Mischung von Polymeren umfasst.

9. Applikator nach einem der Ansprüche 7 oder 8, wobei die den Kern umgebende elektrisch leitende Schicht ein Metallmaterial oder eine Mischung von Metallmaterialien umfasst, die mindestens eines von Kupfer, Silber und Wolfram umfasst.

10. Applikator nach einem der Ansprüche 1 bis 6, wobei mindestens 25 % der elektrisch leitenden starren Partikel einen starren Kern umfassen, der zumindest teilweise von einer weichen Schicht umgeben ist, die im Vergleich zum starren Kern weicher ist.

11. Applikator nach Anspruch 10, wobei die Elektrode in Kontakt mit der weichen Schicht steht.

12. Applikator nach einem der vorhergehenden Ansprüche, wobei mindestens 30% eines Volumens der Füllschicht die elektrisch leitenden starren Partikel umfassen.

13. Applikator nach einem der vorhergehenden Ansprüche, wobei die Monoschicht aus elektrisch leitenden starren Partikeln in dem nicht elektrisch leitfähigen Haftmittel gebildet wird, indem der mindestens eine Ultraschallwandler in das nicht elektrisch leitfähige Haftmittel in Richtung des elektrischen Leiters gedrückt wird.

14. Applikator nach einem der vorhergehenden Ansprüche, wobei der mindestens eine Wandler ein piezoelektrisches Element (1102) umfasst, das aus einem steifen Material mit einem mechanischen Qualitätsfaktor (Qm) größer als 600 gebildet ist.

15. Applikator nach einem der Ansprüche 1 bis 13, wobei der mindestens eine Ultraschallwandler ein piezoelektrisches Element und eine Polyimid-Leiterplatte (PCB) (1302) umfasst, die auf dem PZT-Element angeordnet ist, und wobei eine Dicke des Polyimids in einem Bereich zwischen 10 µm und 35 µm liegt.

## Revendications

1. Applicateur (304, 354, 804, 1002, 1134) d'un système à ultrasons (300), comprenant :
un corps d'applicateur (806, 1004, 1136) présentant au moins une surface émettrice d'ultrasons, dont la forme et la taille sont adaptées pour contacter avec la peau,
au moins un transducteur à ultrasons (244) comprenant une surface opposée à ladite au moins une surface émettrice d'ultrasons et une électrode (1106) connectée à ladite surface du transducteur à ultrasons ;
au moins un conducteur électrique (1110) ;
**caractérisé en ce que** ledit applicateur comprend en outre une couche de remplissage (1108) disposée entre ladite électrode et ledit au moins un conducteur électrique, dans lequel ladite couche de remplissage comprend un adhésif non conducteur d'électricité (1109) et une monocouche de particules rigides conductrices d'électricité (1107) dans ledit adhésif non conducteur d'électricité, dans lequel lesdites particules rigides conductrices d'électricité sont disposées entre ladite électrode (1106) et ledit au moins un conducteur électrique (1110) et les relient électriquement, et sont configurées pour fournir de l'énergie électrique entre ledit au moins un conducteur électrique (1110) et l'électrode (1106) dudit au moins un transducteur à ultrasons.

2. Applicateur selon la revendication 1, dans lequel lesdites particules rigides ont une forme sphérique ou ronde.

3. Applicateur selon l'une quelconque des revendications 1 ou 2, dans lequel la taille ou le diamètre desdites particules rigides électriquement conductrices varie de moins de 5 % par rapport à la taille moyenne ou au diamètre moyen des particules rigides électriquement conductrices.

4. Applicateur selon l'une quelconque des revendications précédentes, dans lequel lesdites particules rigides électriquement conductrices sont formées et dimensionnées de manière à maintenir le au moins un transducteur à ultrasons au moins sensiblement parallèle audit au moins un conducteur électrique.

5. Applicateur selon l'une quelconque des revendications précédentes, dans lequel lesdites particules rigides électriquement conductrices sont des particules thermoconductrices, destinées à évacuer la chaleur dudit au moins un transducteur à ultrasons à travers ladite couche de remplissage.

6. Applicateur selon l'une quelconque des revendications précédentes, dans lequel ledit adhésif non conducteur d'électricité comprend un matériau adhésif solidifié.

7. Applicateur selon l'une quelconque des revendications précédentes, dans lequel chacune desdites particules rigides électriquement conductrices comprend un noyau non électriquement conducteur entouré d'une couche électriquement conductrice.

8. Applicateur selon la revendication 7, dans lequel ledit noyau comprend un polymère ou un mélange de polymères non électriquement conducteurs.

9. Applicateur selon l'une quelconque des revendications 7 ou 8, dans lequel ladite couche électriquement conductrice entourant ledit noyau comprend un matériau métallique ou un mélange de matériaux métalliques comprenant au moins l'un parmi le cuivre, l'argent et le tungstène.

10. Applicateur selon l'une quelconque des revendications 1 à 6, dans lequel au moins 25 % desdites particules rigides électriquement conductrices comprennent un noyau rigide entouré au moins en partie d'une couche souple qui est plus souple que le noyau rigide.

11. Applicateur selon la revendication 10, dans lequel ladite électrode est en contact avec ladite couche souple.

12. Applicateur selon l'une quelconque des revendications précédentes, dans lequel au moins 30 % du volume de ladite couche de remplissage comprend lesdites particules rigides électriquement conductrices.

13. Applicateur selon l'une quelconque des revendications précédentes, dans lequel ladite monocouche de particules rigides électriquement conductrices dans ledit adhésif non conducteur d'électricité est formée en pressant ledit au moins un transducteur à ultrasons dans ledit adhésif non conducteur d'électricité vers le conducteur électrique.

14. Applicateur selon l'une quelconque des revendications précédentes, dans lequel ledit au moins un transducteur comprend un élément piézoélectrique (1102) formé à partir d'un matériau rigide ayant un facteur de qualité mécanique (Qm) supérieur à 600.

15. Applicateur selon l'une quelconque des revendications 1 à 13, dans lequel ledit au moins un transducteur à ultrasons comprend un élément piézoélectrique et une carte de circuit imprimé (PCB) en polyimide (1302) placée au-dessus dudit élément PZT, et dans lequel l'épaisseur dudit polyimide est comprise entre 10 µm et 35 µm.
